(19) Europäisches Patentamt
European Patent Office
Office européen des brevets

(11) EP 2 733 153 A1

(12) **EUROPEAN PATENT APPLICATION**

(43) Date of publication:
**21.05.2014 Bulletin 2014/21**

(51) Int Cl.:
***C07K 16/00*** *(2006.01)* ***C12N 9/48*** *(2006.01)*

(21) Application number: **12306424.8**

(22) Date of filing: **15.11.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **INSERM (Institut National de la Santé et de la Recherche Médicale)**
**75013 Paris (FR)**

(72) Inventor: **The designation of the inventor has not yet been filed**

(74) Representative: **Hirsch, Denise Marie**
**Inserm Transfert**
**IP Department**
**7 rue Watt**
**75013 Paris (FR)**

# (54) Methods for the preparation of immunoconjugates and uses thereof

(57) The present invention relates to a fusion protein consisting of a constant domain (CL) of a light chain of an immunoglobulin fused at its C-terminal end to a polypeptide (P) characterized by the sequence:

LPXT(G)n (P)

wherein n is an integer number and X represents an amino acid other than tryptophan and cysteine.

EP 2 733 153 A1

SEQUENCE LISTING

<110> INSERM

<120> METHODS FOR THE PREPARATION OF IMMUNOCONJUGATES AND USES THEREOF

<130> BIO12160 MALISSEN NC

<160> 131

<170> PatentIn version 3.3

<210> 1
<211> 5
<212> PRT
<213> Artificial

<220>
<223> Polypeptide P

<400> 1

Leu Pro Ala Thr Gly
1               5

<210> 2
<211> 6
<212> PRT
<213> Artificial

<220>
<223> Polypeptide P

<400> 2

Leu Pro Ala Thr Gly Gly
1               5

<210> 3
<211> 7
<212> PRT
<213> Artificial

<220>
<223> Polypeptide P

<400> 3

Leu Pro Ala Thr Gly Gly Gly
1               5

<210> 4
<211> 8
<212> PRT
<213> Artificial

<220>
<223> Polypeptide P

<400> 4

Leu Pro Ala Thr Gly Gly Gly Gly

1 5

<210> 5
<211> 9
<212> PRT
<213> Artificial

<220>
<223> Polypeptide P

<400> 5

Leu Pro Ala Thr Gly Gly Gly Gly Gly
1 5

<210> 6
<211> 10
<212> PRT
<213> Artificial

<220>
<223> Polypeptide P

<400> 6

Leu Pro Ala Thr Gly Gly Gly Gly Gly Gly
1 5 10

<210> 7
<211> 5
<212> PRT
<213> Artificial

<220>
<223> Polypeptide P

<400> 7

Leu Pro Asp Thr Gly
1 5

<210> 8
<211> 6
<212> PRT
<213> Artificial

<220>
<223> Polypeptide P

<400> 8

Leu Pro Asp Thr Gly Gly
1 5

<210> 9
<211> 7
<212> PRT
<213> Artificial

<220>

<223> Polypeptide P

<400> 9

Leu Pro Asp Thr Gly Gly Gly
1 5

<210> 10
<211> 8
<212> PRT
<213> Artificial

<220>
<223> Polypeptide P

<400> 10

Leu Pro Asp Thr Gly Gly Gly Gly
1 5

<210> 11
<211> 9
<212> PRT
<213> Artificial

<220>
<223> Polypeptide P

<400> 11

Leu Pro Asp Thr Gly Gly Gly Gly Gly
1 5

<210> 12
<211> 10
<212> PRT
<213> Artificial

<220>
<223> Polypeptide P

<400> 12

Leu Pro Asp Thr Gly Gly Gly Gly Gly Gly
1 5 10

<210> 13
<211> 5
<212> PRT
<213> Artificial

<220>
<223> Polypeptide P

<400> 13

Leu Pro Glu Thr Gly
1 5

<210> 14

<211> 6
<212> PRT
<213> Artificial

<220>
<223> Polypeptide P

<400> 14

Leu Pro Glu Thr Gly Gly
1 5

<210> 15
<211> 7
<212> PRT
<213> Artificial

<220>
<223> Polypeptide P

<400> 15

Leu Pro Glu Thr Gly Gly Gly
1 5

<210> 16
<211> 8
<212> PRT
<213> Artificial

<220>
<223> Polypeptide P

<400> 16

Leu Pro Glu Thr Gly Gly Gly Gly
1 5

<210> 17
<211> 9
<212> PRT
<213> Artificial

<220>
<223> Polypeptide P

<400> 17

Leu Pro Glu Thr Gly Gly Gly Gly Gly
1 5

<210> 18
<211> 10
<212> PRT
<213> Artificial

<220>
<223> Polypeptide P

<400> 18

Leu Pro Glu Thr Gly Gly Gly Gly Gly Gly
1               5                   10

<210>  19
<211>  5
<212>  PRT
<213>  Artificial

<220>
<223>  Polypeptide P

<400>  19

Leu Pro Phe Thr Gly
1               5

<210>  20
<211>  6
<212>  PRT
<213>  Artificial

<220>
<223>  Polypeptide P

<400>  20

Leu Pro Phe Thr Gly Gly
1               5

<210>  21
<211>  7
<212>  PRT
<213>  Artificial

<220>
<223>  Polypeptide P

<400>  21

Leu Pro Phe Thr Gly Gly Gly
1               5

<210>  22
<211>  8
<212>  PRT
<213>  Artificial

<220>
<223>  Polypeptide P

<400>  22

Leu Pro Phe Thr Gly Gly Gly Gly
1               5

<210>  23
<211>  9
<212>  PRT
<213>  Artificial

<220>
<223> Polypeptide P

<400> 23

Leu Pro Phe Thr Gly Gly Gly Gly Gly
1 5

<210> 24
<211> 10
<212> PRT
<213> Artificial

<220>
<223> Polypeptide P

<400> 24

Leu Pro Phe Thr Gly Gly Gly Gly Gly Gly
1 5 10

<210> 25
<211> 5
<212> PRT
<213> Artificial

<220>
<223> Polypeptide P

<400> 25

Leu Pro Gly Thr Gly
1 5

<210> 26
<211> 6
<212> PRT
<213> Artificial

<220>
<223> Polypeptide P

<400> 26

Leu Pro Gly Thr Gly Gly
1 5

<210> 27
<211> 7
<212> PRT
<213> Artificial

<220>
<223> Polypeptide P

<400> 27

Leu Pro Gly Thr Gly Gly Gly
1 5

<210> 28
<211> 8
<212> PRT
<213> Artificial

<220>
<223> Polypeptide P

<400> 28

Leu Pro Gly Thr Gly Gly Gly Gly
1 5

<210> 29
<211> 9
<212> PRT
<213> Artificial

<220>
<223> Polypeptide P

<400> 29

Leu Pro Gly Thr Gly Gly Gly Gly Gly
1 5

<210> 30
<211> 10
<212> PRT
<213> Artificial

<220>
<223> Polypeptide P

<400> 30

Leu Pro Gly Thr Gly Gly Gly Gly Gly Gly
1 5 10

<210> 31
<211> 5
<212> PRT
<213> Artificial

<220>
<223> Polypeptide P

<400> 31

Leu Pro His Thr Gly
1 5

<210> 32
<211> 6
<212> PRT
<213> Artificial

<220>
<223> Polypeptide P

<400> 32

Leu Pro His Thr Gly Gly
1               5

<210> 33
<211> 7
<212> PRT
<213> Artificial

<220>
<223> Polypeptide P

<400> 33

Leu Pro His Thr Gly Gly Gly
1               5

<210> 34
<211> 8
<212> PRT
<213> Artificial

<220>
<223> Polypeptide P

<400> 34

Leu Pro His Thr Gly Gly Gly Gly
1               5

<210> 35
<211> 9
<212> PRT
<213> Artificial

<220>
<223> Polypeptide P

<400> 35

Leu Pro His Thr Gly Gly Gly Gly Gly
1               5

<210> 36
<211> 10
<212> PRT
<213> Artificial

<220>
<223> Polypeptide P

<400> 36

Leu Pro His Thr Gly Gly Gly Gly Gly Gly
1               5                   10

<210> 37
<211> 5
<212> PRT
<213> Artificial

<220>
<223> Polypeptide P

<400> 37

Leu Pro Ile Thr Gly
1 5

<210> 38
<211> 6
<212> PRT
<213> Artificial

<220>
<223> Polypeptide P

<400> 38

Leu Pro Ile Thr Gly Gly
1 5

<210> 39
<211> 7
<212> PRT
<213> Artificial

<220>
<223> Polypeptide P

<400> 39

Leu Pro Ile Thr Gly Gly Gly
1 5

<210> 40
<211> 8
<212> PRT
<213> Artificial

<220>
<223> Polypeptide P

<400> 40

Leu Pro Ile Thr Gly Gly Gly Gly
1 5

<210> 41
<211> 9
<212> PRT
<213> Artificial

<220>
<223> Polypeptide P

<400> 41

Leu Pro Ile Thr Gly Gly Gly Gly Gly
1 5

<210> 42
<211> 10
<212> PRT
<213> Artificial

<220>
<223> Polypeptide P

<400> 42

Leu Pro Ile Thr Gly Gly Gly Gly Gly Gly
1 5 10

<210> 43
<211> 5
<212> PRT
<213> Artificial

<220>
<223> Polypeptide P

<400> 43

Leu Pro Lys Thr Gly
1 5

<210> 44
<211> 6
<212> PRT
<213> Artificial

<220>
<223> Polypeptide P

<400> 44

Leu Pro Lys Thr Gly Gly
1 5

<210> 45
<211> 7
<212> PRT
<213> Artificial

<220>
<223> Polypeptide P

<400> 45

Leu Pro Lys Thr Gly Gly Gly
1 5

<210> 46
<211> 8
<212> PRT
<213> Artificial

<220>
<223> Polypeptide P

<400> 46

Leu Pro Lys Thr Gly Gly Gly Gly
1 5

<210> 47
<211> 9
<212> PRT
<213> Artificial

<220>
<223> Polypeptide P

<400> 47

Leu Pro Lys Thr Gly Gly Gly Gly Gly
1 5

<210> 48
<211> 10
<212> PRT
<213> Artificial

<220>
<223> Polypeptide P

<400> 48

Leu Pro Lys Thr Gly Gly Gly Gly Gly Gly
1 5 10

<210> 49
<211> 5
<212> PRT
<213> Artificial

<220>
<223> Polypeptide P

<400> 49

Leu Pro Leu Thr Gly
1 5

<210> 50
<211> 6
<212> PRT
<213> Artificial

<220>
<223> Polypeptide P

<400> 50

Leu Pro Leu Thr Gly Gly
1 5

<210> 51
<211> 7
<212> PRT

<213> Artificial

<220>
<223> Polypeptide P

<400> 51

Leu Pro Leu Thr Gly Gly Gly
1 5

<210> 52
<211> 8
<212> PRT
<213> Artificial

<220>
<223> Polypeptide P

<400> 52

Leu Pro Leu Thr Gly Gly Gly Gly
1 5

<210> 53
<211> 9
<212> PRT
<213> Artificial

<220>
<223> Polypeptide P

<400> 53

Leu Pro Leu Thr Gly Gly Gly Gly Gly
1 5

<210> 54
<211> 10
<212> PRT
<213> Artificial

<220>
<223> Polypeptide P

<400> 54

Leu Pro Leu Thr Gly Gly Gly Gly Gly Gly
1 5 10

<210> 55
<211> 5
<212> PRT
<213> Artificial

<220>
<223> Polypeptide P

<400> 55

Leu Pro Met Thr Gly
1 5

<210> 56
<211> 6
<212> PRT
<213> Artificial

<220>
<223> Polypeptide P

<400> 56

Leu Pro Met Thr Gly Gly
1 5

<210> 57
<211> 7
<212> PRT
<213> Artificial

<220>
<223> Polypeptide P

<400> 57

Leu Pro Met Thr Gly Gly Gly
1 5

<210> 58
<211> 8
<212> PRT
<213> Artificial

<220>
<223> Polypeptide P

<400> 58

Leu Pro Met Thr Gly Gly Gly Gly
1 5

<210> 59
<211> 9
<212> PRT
<213> Artificial

<220>
<223> Polypeptide P

<400> 59

Leu Pro Met Thr Gly Gly Gly Gly Gly
1 5

<210> 60
<211> 10
<212> PRT
<213> Artificial

<220>
<223> Polypeptide P

<400> 60

Leu Pro Met Thr Gly Gly Gly Gly Gly Gly
1 5 10

<210> 61
<211> 5
<212> PRT
<213> Artificial

<220>
<223> Polypeptide P

<400> 61

Leu Pro Asn Thr Gly
1 5

<210> 62
<211> 6
<212> PRT
<213> Artificial

<220>
<223> Polypeptide P

<400> 62

Leu Pro Asn Thr Gly Gly
1 5

<210> 63
<211> 7
<212> PRT
<213> Artificial

<220>
<223> Polypeptide P

<400> 63

Leu Pro Asn Thr Gly Gly Gly
1 5

<210> 64
<211> 8
<212> PRT
<213> Artificial

<220>
<223> Polypeptide P

<400> 64

Leu Pro Asn Thr Gly Gly Gly Gly
1 5

<210> 65
<211> 9

<212> PRT
<213> Artificial

<220>
<223> Polypeptide P

<400> 65

Leu Pro Asn Thr Gly Gly Gly Gly Gly
1 5

<210> 66
<211> 10
<212> PRT
<213> Artificial

<220>
<223> Polypeptide P

<400> 66

Leu Pro Asn Thr Gly Gly Gly Gly Gly Gly
1 5 10

<210> 67
<211> 5
<212> PRT
<213> Artificial

<220>
<223> Polypeptide P

<400> 67

Leu Pro Pro Thr Gly
1 5

<210> 68
<211> 6
<212> PRT
<213> Artificial

<220>
<223> Polypeptide P

<400> 68

Leu Pro Pro Thr Gly Gly
1 5

<210> 69
<211> 7
<212> PRT
<213> Artificial

<220>
<223> Polypeptide P

<400> 69

Leu Pro Pro Thr Gly Gly Gly

1 5

<210> 70
<211> 8
<212> PRT
<213> Artificial

<220>
<223> Polypeptide P

<400> 70

Leu Pro Pro Thr Gly Gly Gly Gly
1 5

<210> 71
<211> 9
<212> PRT
<213> Artificial

<220>
<223> Polypeptide P

<400> 71

Leu Pro Pro Thr Gly Gly Gly Gly Gly
1 5

<210> 72
<211> 10
<212> PRT
<213> Artificial

<220>
<223> Polypeptide

<400> 72

Leu Pro Pro Thr Gly Gly Gly Gly Gly Gly
1 5 10

<210> 73
<211> 5
<212> PRT
<213> Artificial

<220>
<223> Polypeptide P

<400> 73

Leu Pro Gln Thr Gly
1 5

<210> 74
<211> 6
<212> PRT
<213> Artificial

<220>

<223> Polypeptide P

<400> 74

Leu Pro Gln Thr Gly Gly
1 5

<210> 75
<211> 7
<212> PRT
<213> Artificial

<220>
<223> Polypeptide P

<400> 75

Leu Pro Gln Thr Gly Gly Gly
1 5

<210> 76
<211> 8
<212> PRT
<213> Artificial

<220>
<223> Polypeptide P

<400> 76

Leu Pro Gln Thr Gly Gly Gly Gly
1 5

<210> 77
<211> 9
<212> PRT
<213> Artificial

<220>
<223> Polypeptide P

<400> 77

Leu Pro Gln Thr Gly Gly Gly Gly Gly
1 5

<210> 78
<211> 10
<212> PRT
<213> Artificial

<220>
<223> Polypeptide P

<400> 78

Leu Pro Gln Thr Gly Gly Gly Gly Gly Gly
1 5 10

<210> 79

<211> 5
<212> PRT
<213> Artificial

<220>
<223> Polypeptide P

<400> 79

Leu Pro Arg Thr Gly
1 5

<210> 80
<211> 6
<212> PRT
<213> Artificial

<220>
<223> Polypeptide P

<400> 80

Leu Pro Arg Thr Gly Gly
1 5

<210> 81
<211> 7
<212> PRT
<213> Artificial

<220>
<223> Polypeptide P

<400> 81

Leu Pro Arg Thr Gly Gly Gly
1 5

<210> 82
<211> 8
<212> PRT
<213> Artificial

<220>
<223> Polypeptide P

<400> 82

Leu Pro Arg Thr Gly Gly Gly Gly
1 5

<210> 83
<211> 9
<212> PRT
<213> Artificial

<220>
<223> Polypeptide P

<400> 83

Leu Pro Arg Thr Gly Gly Gly Gly Gly
1               5

<210> 84
<211> 10
<212> PRT
<213> Artificial

<220>
<223> Polypeptide P

<400> 84

Leu Pro Arg Thr Gly Gly Gly Gly Gly Gly
1               5                   10

<210> 85
<211> 5
<212> PRT
<213> Artificial

<220>
<223> Polypeptide P

<400> 85

Leu Pro Ser Thr Gly
1               5

<210> 86
<211> 6
<212> PRT
<213> Artificial

<220>
<223> Polypeptide P

<400> 86

Leu Pro Ser Thr Gly Gly
1               5

<210> 87
<211> 7
<212> PRT
<213> Artificial

<220>
<223> Polypeptide P

<400> 87

Leu Pro Ser Thr Gly Gly Gly
1               5

<210> 88
<211> 8
<212> PRT
<213> Artificial

<220>
<223>  Polypeptide P

<400>  88

Leu Pro Ser Thr Gly Gly Gly Gly
1               5

<210>  89
<211>  9
<212>  PRT
<213>  Artificial

<220>
<223>  Polypeptide P

<400>  89

Leu Pro Ser Thr Gly Gly Gly Gly Gly
1               5

<210>  90
<211>  10
<212>  PRT
<213>  Artificial

<220>
<223>  Polypeptide P

<400>  90

Leu Pro Ser Thr Gly Gly Gly Gly Gly Gly
1               5                   10

<210>  91
<211>  5
<212>  PRT
<213>  Artificial

<220>
<223>  Polypeptide P

<400>  91

Leu Pro Thr Thr Gly
1               5

<210>  92
<211>  6
<212>  PRT
<213>  Artificial

<220>
<223>  Polypeptide P

<400>  92

Leu Pro Thr Thr Gly Gly
1               5

<210> 93
<211> 8
<212> PRT
<213> Artificial

<220>
<223> Polypeptide P

<400> 93

Leu Pro Ala Thr Thr Gly Gly Gly
1 5

<210> 94
<211> 8
<212> PRT
<213> Artificial

<220>
<223> Polypeptide P

<400> 94

Leu Pro Thr Thr Gly Gly Gly Gly
1 5

<210> 95
<211> 9
<212> PRT
<213> Artificial

<220>
<223> Polypeptide P

<400> 95

Leu Pro Thr Thr Gly Gly Gly Gly Gly
1 5

<210> 96
<211> 10
<212> PRT
<213> Artificial

<220>
<223> Polypeptide P

<400> 96

Leu Pro Thr Thr Gly Gly Gly Gly Gly Gly
1 5 10

<210> 97
<211> 5
<212> PRT
<213> Artificial

<220>
<223> Polypeptide P

<400> 97

Leu Pro Val Thr Gly
1               5

<210>  98
<211>  6
<212>  PRT
<213>  Artificial

<220>
<223>  Polypeptide P

<400>  98

Leu Pro Val Thr Gly Gly
1               5

<210>  99
<211>  7
<212>  PRT
<213>  Artificial

<220>
<223>  Polypeptide P

<400>  99

Leu Pro Val Thr Gly Gly Gly
1               5

<210>  100
<211>  8
<212>  PRT
<213>  Artificial

<220>
<223>  Polypeptide P

<400>  100

Leu Pro Val Thr Gly Gly Gly Gly
1               5

<210>  101
<211>  9
<212>  PRT
<213>  Artificial

<220>
<223>  Polypeptide P

<400>  101

Leu Pro Val Thr Gly Gly Gly Gly Gly
1               5

<210>  102
<211>  10
<212>  PRT
<213>  Artificial

<220>
<223>  Polypeptide P

<400>  102

Leu Pro Val Thr Gly Gly Gly Gly Gly Gly
1               5                   10

<210>  103
<211>  5
<212>  PRT
<213>  Artificial

<220>
<223>  Polypeptide P

<400>  103

Leu Pro Tyr Thr Gly
1               5

<210>  104
<211>  6
<212>  PRT
<213>  Artificial

<220>
<223>  Polypeptide P

<400>  104

Leu Pro Tyr Thr Gly Gly
1               5

<210>  105
<211>  7
<212>  PRT
<213>  Artificial

<220>
<223>  Polypeptide P

<400>  105

Leu Pro Tyr Thr Gly Gly Gly
1               5

<210>  106
<211>  8
<212>  PRT
<213>  Artificial

<220>
<223>  Polypeptide P

<400>  106

Leu Pro Tyr Thr Gly Gly Gly Gly
1               5

<210> 107
<211> 9
<212> PRT
<213> Artificial

<220>
<223> Polypeptide P

<400> 107

Leu Pro Tyr Thr Gly Gly Gly Gly Gly
1 5

<210> 108
<211> 10
<212> PRT
<213> Artificial

<220>
<223> Polypeptide P

<400> 108

Leu Pro Tyr Thr Gly Gly Gly Gly Gly Gly
1 5 10

<210> 109
<211> 2
<212> PRT
<213> Artificial

<220>
<223> Linker L

<400> 109

Gly Ser
1

<210> 110
<211> 4
<212> PRT
<213> Artificial

<220>
<223> Linker L

<400> 110

Gly Ser Gly Ser
1

<210> 111
<211> 6
<212> PRT
<213> Artificial

<220>
<223> Linker L

<400> 111

Gly Ser Gly Ser Gly Ser
1 5

<210> 112
<211> 8
<212> PRT
<213> Artificial

<220>
<223> Linker L

<400> 112

Gly Ser Gly Ser Gly Ser Gly Ser
1 5

<210> 113
<211> 10
<212> PRT
<213> Artificial

<220>
<223> Linker L

<400> 113

Gly Ser Gly Ser Gly Ser Gly Ser Gly Ser
1 5 10

<210> 114
<211> 1
<212> PRT
<213> Artificial

<220>
<223> Tag

<400> 114

His
1

<210> 115
<211> 2
<212> PRT
<213> Artificial

<220>
<223> Tag

<400> 115

His His
1

<210> 116
<211> 3
<212> PRT

<213> Artificial

<220>
<223> Tag

<400> 116

His His His
1

<210> 117
<211> 4
<212> PRT
<213> Artificial

<220>
<223> Tag

<400> 117

His His His His
1

<210> 118
<211> 5
<212> PRT
<213> Artificial

<220>
<223> Tag

<400> 118

His His His His His
1               5

<210> 119
<211> 6
<212> PRT
<213> Artificial

<220>
<223> Tag

<400> 119

His His His His His His
1               5

<210> 120
<211> 7
<212> PRT
<213> Artificial

<220>
<223> Tag

<400> 120

His His His His His His His
1               5

<210> 121
<211> 8
<212> PRT
<213> Artificial

<220>
<223> Tag

<400> 121

His His His His His His His His
1 5

<210> 122
<211> 9
<212> PRT
<213> Artificial

<220>
<223> Tag

<400> 122

His His His His His His His His His
1 5

<210> 123
<211> 10
<212> PRT
<213> Artificial

<220>
<223> Tag

<400> 123

His His His His His His His His His His
1 5 10

<210> 124
<211> 20
<212> PRT
<213> Artificial

<220>
<223> Copoc cassette

<400> 124

Gly Ser Gly Ser Gly Ser Gly Ser Leu Pro Glu Thr Gly Gly His His
1 5 10 15

His His His His
20

<210> 125
<211> 321
<212> DNA

<213> Mus musculus

<400> 125
gctgatgctg caccaactgt atccatcttc ccaccatcca gtgagcagtt aacatctgga 60

ggtgcctcag tcgtgtgctt cttgaacaac ttctacccca aagacatcaa tgtcaagtgg 120

aagattgatg gcagtgaacg acaaaatggc gtcctgaaca gttggactga tcaggacagc 180

aaagacagca cctacagcat gagcagcacc ctcacgttga ccaaggacga gtatgaacga 240

cataacagct atacctgtga ggccactcac aagacatcaa cttcacccat tgtcaagagc 300

ttcaacagga atgagtgtta g 321

<210> 126
<211> 318
<212> DNA
<213> Homo sapiens

<400> 126
actgtggctg caccatctgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgt 318

<210> 127
<211> 378
<212> DNA
<213> Homo sapiens

<400> 127
actgtggctg caccatctgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgtgg ttccggttcc ggttccggtt ccctgcccga gacaggagga 360

catcatcatc atcatcat 378

<210> 128
<211> 16746
<212> DNA
<213> Artificial

<220>
<223> Sequence of the targeting vector

<400> 128

```
cgcgccgcgg ccgcgggaat tcgattatcg aattctaccg ggtaggggag gcgcttttcc      60
caaggcagtc tggagcatgc gctttagcag ccccgctggg cacttggcgc tacacaagtg     120
gcctctggcc tcgcacacat tccacatcca ccggtaggcg ccaaccggct ccgttctttg     180
gtggcccctt cgcgccacct tctactcctc ccctagtcag gaagttcccc cccgccccgc     240
agctcgcgtc gtgcagacgt gacaaatgga agtagcacgt ctcactagtc tcgtgcagat     300
ggacagcacc gctgagcaat ggaagcgggt aggcctttgg ggcagcggcc aatagcagct     360
ttgctccttc gctttctggg ctcagaggct gggaaggggt gggtccgggg gcgggctcag     420
gggcgggctc aggggcgggg cgggcgcccg aaggtcctcc ggaggcccgg cattctgcac     480
gcttcaaaag cgcacgtctg ccgcgctgtt ctcctcttcc tcatctccgg gcctttcgac     540
ctgcaggtcc tcgccatgga tcctgatgat gttgttgatt cttctaaatc ttttgtgatg     600
gaaaactttt cttcgtacca cgggactaaa cctggttatg tagattccat tcaaaaaggt     660
atacaaaagc caaaatctgg tacacaagga aattatgacg atgattggaa agggttttat     720
agtaccgaca ataaatacga cgctgcggga tactctgtag ataatgaaaa cccgctctct     780
ggaaaagctg gaggcgtggt caaagtgacg tatccaggac tgacgaaggt tctcgcacta     840
aaagtggata atgccgaaac tattaagaaa gagttaggtt taagtctcac tgaaccgttg     900
atggagcaag tcggaacgga agagtttatc aaaaggttcg gtgatggtgc ttcgcgtgta     960
gtgctcagcc ttcccttcgc tgaggggagt tctagcgttg aatatattaa taactgggaa    1020
caggcgaaag cgttaagcgt agaacttgag attaattttg aaacccgtgg aaaacgtggc    1080
caagatgcga tgtatgagta tatggctcaa gcctgtgcag gaaatcgtgt caggcgatct    1140
ctttgtgaag gaaccttact tctgtggtgt gacataattg gacaaactac ctacagagat    1200
ttaaagctct aaggtaaata taaaattttt aagtgtataa tgtgttaaac tactgattct    1260
aattgtttgt gtattttaga ttccaaccta tggaactgat gaatgggagc agtggtggaa    1320
tgcagatcct agagctcgct gatcagcctc gactgtgcct tctagttgcc agccatctgt    1380
tgtttgcccc tcccccgtgc cttccttgac cctggaaggt gccactccca ctgtcctttc    1440
ctaataaaat gaggaaattg catcgcattg tctgagtagg tgtcattcta ttctgggggg    1500
tggggtgggg caggacagca agggggagga ttgggaagac aatagcaggc atgctgggga    1560
tgcggtgggc tctatggctt ctgaggcgga aagaaccagc tggggctcga cgagaacgaa    1620
gcgctaaccg tttttatcag gctctgggag gcagaataaa tgatcatatc gtcaattatt    1680
acctccacgg ggagagcctg agcaaactgg cctcaggcat ttgagaagca cacggtcaca    1740
ctgcttccgg tagtcaataa accggtaaac cagcaataga cataagcggc tatttaacga    1800
ccctgccctg aaccgacgac cgggtcgaat ttgctttcga atttctgcca ttcatccgct    1860
tattatcact tattcaggcg tagcaaccag gcgtttaagg gcaccaataa ctgccttaaa    1920
aaaattacgc cccgccctgc cactcatcgc agtactgttg taattcatta agcattctgc    1980
```

cgacatggaa gccatcacaa acggcatgat gaacctgaat cgccagcggc atcagcacct 2040
tgtcgccttg cgtataatat ttgcccatgg tgaaaacggg ggcgaagaag ttgtccatat 2100
tggccacgtt taaatcaaaa ctggtgaaac tcacccaggg attggctgag acgaaaaaca 2160
tattctcaat aaacccttta gggaaatagg ccaggttttc accgtaacac gccacatctt 2220
gcgaatatat gtgtagaaac tgccggaaat cgtcgtggta ttcactccag agcgatgaaa 2280
acgtttcagt ttgctcatgg aaaacggtgt aacaagggtg aacactatcc catatcacca 2340
gctcaccgtc tttcattgcc atacggaatt ccggatgagc attcatcagg cgggcaagaa 2400
tgtgaataaa ggccggataa aacttgtgct tatttttctt tacggtcttt aaaaaggccg 2460
taatatccag ctgaacggtc tggttatagg tacattgagc aactgactga aatgcctcaa 2520
aatgttcttt acgatgccat tgggatatat caacggtggt atatccagtg atttttttct 2580
ccattttagc ttccttagct cctgaaaatc tcgataactc aaaaaatacg cccggtagtg 2640
atcttatttc attatggtga aagttggaac ctcttacgtg ccgatcaacg tctcattttc 2700
gccaaaagtt ggcccagggc ttcccggtat caacagggac accaggattt atttattctg 2760
cgaagtgatc ttccgtcaca ggtatttatt cggcgcaaag tgcgtcgggt gatgctgcca 2820
acttactgat ttagtgtatg atggtgtttt tgaggtgctc cagtggcttc tgtttctatc 2880
agctgtccct cctgttcagc tactgacggg gtggtgcgta acggcaaaag caccgccgga 2940
catcagcgct gataatcact agtgaattcg cggccgcatc tttttttatg tgtaagacac 3000
aggttttcat gttaggagtt aaagtcagtt cagaaaatct tgagaaaatg gagagggctc 3060
attatcagtt gacgtggcat acagtgtcag attttctgtt tatcaagcta gtgagattag 3120
gggcaaaaag aggctttagt tgagaggaaa gtaattaata ctatggtcac catccaagag 3180
attggatcgg agaataagca tgagtagtta ttgagatctg ggtctgactg caggtagcgt 3240
ggtcttctag acgtttaagt gggagatttg gaggggatga ggaatgaagg aacttcagga 3300
tagaaaaggg ctgaagtcaa gttcagctcc taaaatggat gtgggagcaa actttgaaga 3360
taaactgaat gacccagagg atgaaacagc gcagatcaaa gaggggcctg gagctctgag 3420
aagagaagga gactcatccg tgttgagttt ccacaagtac tgtcttgagt tttgcaataa 3480
aagtgggata gcagagttga gtgagccgta ggctgagttc tctcttttgt ctcctaagtt 3540
tttatgacta caaaaatcag tagtatgtcc tgaaataatc attaagctgt ttgaaagtat 3600
gactgcttgc catgtagata ccatggcttg ctgaataatc agaagaggtg tgactcttat 3660
tctaaaattt gtcacaaaat gtcaaaatga gagactctgt aggaacgagt ccttgacaga 3720
cagctcaagg ggtttttttc ctttgtctca tttctacatg aaagtaaatt tgaaatgatc 3780
tttttttatta taagagtaga aatacagttg ggtttgaact atatgtttta atggccacgg 3840
ttttgtaaga catttggtcc tttgttttcc cagttattac tcgattgtaa ttttatatcg 3900

ccagcaatgg actgaaacgg tccgcaacct cttctttaca actgggtgac ctcgcggctg 3960
tgccagccat ttggcgttca ccctgccgct aagggccatg tgaacccccg cggtagcatc 4020
ccttgctccg cgtggaccac tttcctgagg cacagtgata ggaacagagc cactaatctg 4080
aagagaacag agatgtgaca gactacacta atgtgagaaa aacaaggaaa gggtgactta 4140
ttggagattt cagaaataaa atgcatttat tattatattc ccttatttta attttctatt 4200
agggaattag aaagggcata aactgcttta tccagtgtta tattaaaagc ttaatgtata 4260
taatctttta gaggtaaaat ctacagccag caaaagtcat ggtaaatatt ctttgactga 4320
actctcacta aactcctcta aattatatgt catattaact ggttaaatta atataaattt 4380
gtgacatgac cttaactggt taggtaggat atttttcttc atgcaaaaat atgactaata 4440
ataatttagc acaaaaatat ttcccaatac tttaattctg tgatagaaaa atgtttaact 4500
cagctactat aatcccataa ttttgaaaac tatttattag cttttgtgtt tgacccttcc 4560
ctagccaaag gcaactattt aaggaccctt taaaactctt gaaactactt tagagtcatt 4620
aagttattta accactttta attactttaa aatgatgtca attccctttt aactattaat 4680
ttattttaag gggggaaagg ctgctcataa ttctattgtt tttcttggta aagaactctc 4740
agttttcgtt tttactacct ctgtcaccca agagttggca tctcaacaga ggggactttc 4800
cgagaggcca tctggcagtt gcttaagatc agaagtgaag tctgccagtt cctcccaggc 4860
aggtggccca gattacagtt gacctgttct ggtgtggcta aaaattgtcc catgtggtta 4920
caaaccatta gaccagggtc tgatgaattg ctcagaatat ttctggacac ccaaatacag 4980
accctggctt aaggccctgt ccatacagta ggtttagctt ggctacacca aaggaagcca 5040
tacagaggct aatatcagag tattcttgga agagacagga gaaaatgaaa gccagtttct 5100
gctcttacct tatgtgcttg tgttcagact cccaaacatc aggagtgtca gataaactgg 5160
tctgaatctc tgtctgaagc atggaactga aaagaatgta gtttcaggga agaaaggcaa 5220
tagaaggaag cctgagaata tcttcaaagg gtcagactca atttactttc taaagaagta 5280
gctaggaact agggaataac ttagaaacaa caagattgta tatatgtgca tcctggcccc 5340
attgttcctt atctgtaggg ataagcgtgc ttttttgtgt gtctgtatat aacataactg 5400
tttacacata atacactgaa atggagccct tccttgttac ttcataccat cctctgtgct 5460
tccttcctca ggaactgtgg ctgcaccatc tgtcttcatc ttcccgccat ctgatgagca 5520
gttgaaatct ggaactgcct ctgttgtgtg cctgctgaat aacttctatc ccagagaggc 5580
caaagtacag tggaaggtgg ataacgccct ccaatcgggt aactcccagg agagtgtcac 5640
agagcaggac agcaaggaca gcacctacag cctcagcagc accctgacgc tgagcaaagc 5700
agactacgag aaacacaaag tctacgcctg cgaagtcacc catcagggcc tgagctcgcc 5760
cgtcacaaag agcttcaaca ggggagagtg tggttccggt tccggttccg gttccctgcc 5820
cgagacagga ggacatcatc atcatcatca ttgaagacaa acccgatctc gaggtcgact 5880

cgcgaaagct agcttataac ttcgtatagc atacattata cgaagttatg gatctccatg 5940
ggccaggcaa atatccctta ccagcctcac agagacctcc cccacccccc gcaaccctag 6000
agttctttta ctagtgaggg acaagtggac aatggtgctg ttgtgggccc caccctgtgt 6060
cccctgtgcc cacagtggtc actctgcttg gcaggcaggt gttgcaggct ggctgctcca 6120
ggccctggca ggaggtactg aaggacctgg taggctcaga tgccctggat gccaaggcac 6180
tgctggagta cttccaaccg gtcagccagt ggctggaaga gcagaatcag cggaatggcg 6240
aagtcctagg ctggccagag aatcagtggc gtccaccgtt acccgacaac tatccagagg 6300
gcattggtaa agctctgagt gagggtggac tgggaccaag agaagtcctg gcctctggcc 6360
tctggcttct gggtcaaagc ctcagcatcc tggtcacttt gctgccagct gagccccagt 6420
gtcctttgct tcagtgccaa gccacccctg ggctcatcct cagggcccta agcagaaatg 6480
ggtatgtctt tctctcaggg tcctagagac agtgtgccca agcctgaggg cccttggggt 6540
caggctggct ggcacattgc tctatgaggt cacactgcag gcttggctct tattggccgg 6600
tgatgggagc ttcagggctc tgctttcctg cggccatgcc caagaagaag aggaaggtgt 6660
ccaatttact gaccgtacac caaaatttgc ctgcattacc ggtcgatgca acgagtgatg 6720
aggttcgcaa gaacctgatg gacatgttca gggatcgcca ggcgttttct gagcatacct 6780
ggaaaatgct tctgtccgtt tgccggtcgt gggcggcatg gtgcaagttg aataaccgga 6840
aatggtttcc cgcagaacct gaagatgttc gcgattatct tctatatctt caggcgcgcg 6900
gtctggcagt aaaaactatc cagcaacatt tgggccagct aaacatgctt catcgtcggt 6960
ccgggctgcc acgaccaagt gacagcaatg ctgtttcact ggttatgcgg cggatccgaa 7020
aagaaaacgt tgatgccggt gaacgtgcaa aacaggctct agcgttcgaa cgcactgatt 7080
tcgaccaggt tcgttcactc atggaaaata gcgatcgctg ccaggatata cgtaatctgg 7140
catttctggg gattgcttat aacaccctgt tacgtatagc cgaaattgcc aggatcaggg 7200
ttaaagatat ctcacgtact gacggtggga gaatgttaat ccatattggc agaacgaaaa 7260
cgctggttag caccgcaggt gtagagaagg cacttagcct gggggtaact aaactggtcg 7320
agcgatggat ttccgtctct ggtgtagctg atgatccgaa taactacctg ttttgccggg 7380
tcagaaaaaa tggtgttgcc gcgccatctg ccaccagcca gctatcaact cgcgccctgg 7440
aagggatttt tgaagcaact catcgattga tttacggcgc taaggtaaat ataaaatttt 7500
taagtgtata atgtgttaaa ctactgattc taattgtttg tgtattttag gatgactctg 7560
gtcagagata cctggcctgg tctggacaca gtgcccgtgt cggagccgcg cgagatatgg 7620
cccgcgctgg agtttcaata ccggagatca tgcaagctgg tggctggacc aatgtaaata 7680
ttgtcatgaa ctatatccgt aacctggata gtgaaacagg ggcaatggtg cgcctgctgg 7740
aagatggcga ttagccatta acgcgtaaat gattgctata attatttgat atttatggtg 7800

acatatgaga aaggatttca acatcgacgg aaaatatgta gtgctgtctg taagcactaa 7860

tattcagtcg ccagccgtca ttgtcactgt aaagctgagc ggcaataaaa agacagaata 7920

aaacgcacgg gtgttgggtc gtttgttcgg tcgagctcgc gaaagcttct accgggtagg 7980

ggaggcgctt ttcccaaggc agtctggagc atgcgcttta gcagccccgc tgggcacttg 8040

gcgctacaca agtggcctct ggcctcgcac acattccaca tccaccggta ggcgccaacc 8100

ggctccgttc tttggtggcc ccgtcgcgcc accttctact cctcccctag tcaggaagtt 8160

cccccccgcc ccgcagctcg cgtcgtgcag gacgtgacaa atggaagtag cacgtctcac 8220

tagtctcgtg cagatggaca gcaccgctga gcaatggaag cgggtaggcc tttggggcag 8280

cggccaatag cagctttgct ccttcgcttt ctgggctcag aggctgggaa ggggtgggtc 8340

cgggggcggg ctcaggggcg ggctcagggg cggggcgggc gcccgaaggt cctccggagg 8400

cccggcattc tgcacgcttc aaaagcgcac gtctgccgcg ctgttctcct cttcctcatc 8460

tccgggcctt tcgacctgca gcagcacgtg ttgacaatta atcatcggca tagtatatcg 8520

gcatagtata atacgacaag gtgaggaact aaaccatggg atcggccatt gaacaagatg 8580

gattgcacgc aggttctccg gccgcttggg tggagaggct attcggctat gactgggcac 8640

aacagacgat cggctgctct gatgccgccg tgttccggct gtcagcgcag gggcgcccgg 8700

ttctttttgt caagaccgac ctgtccggtg ccctgaatga actgcaggac gaggcagcgc 8760

ggctatcgtg gctggccacg acgggcgttc cttgcgcagc tgtgctcgac gttgtcactg 8820

aagcgggaag ggactggctg ctattgggcg aagtgccggg gcaggatctc ctgtcatctc 8880

accttgctcc tgccgagaaa gtatccatca tggctgatgc aatgcggcgg ctgcatacgc 8940

ttgatccggc tacctgccca ttcgaccacc aagcgaaaca tcgcatcgag cgagcacgta 9000

ctcggatgga agccggtctt gtcgatcagg atgatctgga cgaagagcat caggggctcg 9060

cgccagccga actgttcgcc aggctcaagg cgcgcatgcc cgacggcgag gatctcgtcg 9120

tgacccatgg cgatgcctgc ttgccgaata tcatggtgga aaatggccgc ttttctggat 9180

tcatcgactg tggccggctg ggtgtggcgg accgctatca ggacatagcg ttggctaccc 9240

gtgatattgc tgaagagctt ggcggcgaat gggctgaccg cttcctcgtg ctttacggta 9300

tcgccgcccc cgattcgcag cgcatcgcct tctatcgcct tcttgacgag ttcttctgag 9360

cgggactctg gggttcgaat aaagaccgac caagcgacgt ctgagagctc cctggcgaat 9420

tcggtaccaa taaaagagct ttattttcat gatctgtgtg ttggtttttg tgtgcggcgc 9480

gataacttcg tatagcatac attatacgaa gttatcccaa ttcatcgata tctagatcgg 9540

ggtcctgaga cgccaccacc agctccccag ctccatccta tcttcccttc taaggtcttg 9600

gaggcttccc cacaagcgac ctaccactgt tgcggtgctc caaacctcct ccccacctcc 9660

ttctcctcct cctccctttc cttggctttt atcatgctaa tatttgcaga aaatattcaa 9720

taaagtgagt ctttgcactt gagatctctg tctttcttac taaatggtag taatcagttg 9780

tttttccagt tacctgggtt tctcttctaa agaagttaaa tgtttagttg ccctgaaatc 9840
caccacactt aaaggataaa taaaaccctc cacttgccct ggttggctgt ccactacatg 9900
gcagtccttt ctaaggttca cgagtactat tcatggctta tttctctggg ccatggtagg 9960
tttgaggagg catacttcct agttttcttc ccctaagtcg tcaaagtcct gaagggggac 10020
agtctttaca agcacatgtt ctgtaatctg attcaaccta cccagtaaac ttggcgaagc 10080
aaagtagaat cattatcaca ggaagcaaag gcaacctaaa tgtgcaagca ataggaaaat 10140
gtggaagccc atcatagtac ttggacttca tctgcttttg tgccttcact aagtttttaa 10200
acatgagctg gctcctatct gccattggca aggctgggca ctacccacaa cctacttcaa 10260
ggacctctat accgtgagat tacacacata catcaaaatt tgggaaaagt tctaccaagc 10320
tgagagctga tcacccccact cttaggtgct tatctctgta caccagaaac cttaagaagc 10380
aaccagtatt gagagactca tttatgaaag tctaaaactg gatacaacca aaatgtccac 10440
caacagttaa attatgacat gttcacaatt gagctattac ttaataagga gaattaataa 10500
aataaaactt aagagcatag tttaatctca taaacaagat aataagcaaa acaaaacatt 10560
ttttcatcca tgtaagttta aaagcaggta aaatttaaaa ttaagagaga cataagtttt 10620
gaggtagcaa gatggaaact ctggggcttg gggaatgttc tgtctctctg tatgggatgt 10680
gaaagttact attgtggaat tgggatctat gttcttcctg tatatattgt atacttcata 10740
ataacttcac ctaaagaaat atctaatacc cagtgcatac ataaaagagg atacaaggaa 10800
tgaatcatac gtcaaggcca gaaagacaat aaagtagggg atccaggatc aaatctccca 10860
caaccttgag ccttctacta ttctgccttc cagagctcaa agtacaaaac acataattca 10920
aacacatgat ccctccttgg ggtctcttcc ttcatgcatc gaattagaaa tagccatgta 10980
taaaatgaga tagaagagac cttcatcaac aggtcaaaga atataggtaa ttttgtctgg 11040
gtatgaagag cccacgtatc aaaggttaca ttagggaagg aagaggacac taacagtgac 11100
tttcattctc cccctcttcc tggaggcccc tgcatttagt ccctcgtggg ctcatccact 11160
cagcacacat ttactaagca tcttctcagc ctacactctg aaggcagtgc agaataatgt 11220
tagtgtccct tcccccagtt aatatgcagt ccagtttccc tgctccttcc ctttctcagt 11280
ccacataagg atgatgggaa aggacagtca ccaaatagga gagggcaacc ctttgccttc 11340
ctacctcttg agaatgtaca ttattatcca ctttttgaaa cttcttttaa ttgctttttt 11400
ttaatttgtc ttttcaaata gcataacctt gttcatccat ttctgggaac caaatttatc 11460
aatcaacagt gcctctaatc tggctattaa tacaaaaatg cctcctcaaa atatatatgt 11520
tcgagtctta tctaaaacag aacccacaat aaaaaagaag aaagaataca tataagcatt 11580
tatataattc tgagcaacct tgtgctttgt gaaaaaaata taatctaatg tcacatgctg 11640
tattcttttt atttaacact ggtgaaatta taccattaga gagaaagagg acagatcact 11700

gatcctagga tctagggatg ttacagataa gaaaacaaat gtgacaaaga gctgtcacaa 11760
ggaggatctt caaggtcaca gaatcactgt cttgatttca gtggtggtta catacattta 11820
aatatgtgat aaaatgttgt tgaactatat tcatatattg taccaatgtc aaatgcttaa 11880
ttttggctct atagtataat tatgcactaa ataactattt ggacaaagaa aatgatgttt 11940
acatcaaagg tgaggccata tttgttagga acataactta aaaaccattt tggataacta 12000
atgaaaagcc attttgtgtg ccttggcata tcatgcctaa gctgtcacca gatagatcta 12060
ataagaccta agcctcagaa gcaagcccct gcccagcaag caggcagcac agataagagc 12120
taaacccagg acaggccatg atatgctaat gaactacctt caaggtggtg ttgctgacct 12180
agtgaaccag ccccaagctg tgagccccaa tagcacaaag ctactgccca aagaaattat 12240
acaaaaattg gaactttggg aatggtgtgc aggatcgctc tgctgtatgc ctggaacaca 12300
gcttctctat gttttgtatt gataccagtc tagaagcttc caaaactttc tcactgaaga 12360
agattcccca tgtgggaccc ctacagactc ttttgcccaa acaactgctt ccctcctggt 12420
gtgatatctg ttttgctttt atgttagcat aatattataa ggaatgtttg tgtgaataaa 12480
ccaaacatat tttaaaagca aatattgtat gcacatccta attgctaaaa agtttacagc 12540
taatagtccc atgctctcca caatactgga tccaaataag tcctaatttc aatgttgggc 12600
atctttacag agagaaagac attaaaaatg aagagacatg cagagagtgc accatgccat 12660
cgtggagaca gactgaagtg acacaactgt tagtcaaaga ggattaagga cttccagaag 12720
ccaccaaagg aaggaggtat gaagtggttt ctccctcaga gtatccagag gagactaaac 12780
caaccaacac ctttttgctt aagacttctt gccttcagga ctgtgagaag gtagcttcct 12840
attgttctaa gccccagtat gtggcatttt gttaaggtag agtcaagaaa ccaataaaat 12900
gcagacagac aaaaggatag ctgagttttc caggcccttc cttcttattt ttggttttgt 12960
tggtggtggt ggtggtggtg gtgatggtgg tggttttgtt tatgttttgt ttggggagtt 13020
ttttggggtt tttttgggtt ttgtttttgt tgttgttttg ggggtttttg ttgttgttgt 13080
tgtttgcttt tttgtttttt gtttttgtt tttttgagac agtgtttctc tgtatagccc 13140
tggctgtcct ggagttcctt ctatctctaa tgtctacatc tcagagggga tcctctaatt 13200
tcaaatgagc agtagctctc catttttagc tcttatttat tcatttattt acttacttac 13260
ttattgtctg tagatgaaag aattttggag tgggaaaggg ttcatgagcc cccagcaact 13320
aatgaggagc tacagacaat tgatgtttct ggggaaagga gactcagttt ctttgagagt 13380
atagcttctg atgggtcaac catgttcctg tggctgatgt cacacccagg agtatgcaga 13440
caacagaaac tggagttaat gagttgtttt aaaaataaaa aagggcatga agcttgggat 13500
agaaattaag gataaataca attaaataca ggaaattctg aaagaattaa taaaaacatt 13560
tcttttttta aaaaaaaatc cagaattagc tatgcttctt caaaattgct tctggagaac 13620
tttacaagtt aaataagtta tattgtagaa aaggtagaga ggagaatagt ggaagagaga 13680

gataaggaga cttcaaaagg agtggaggga gatagaggag gagaaagcag aagcaatggc 13740
tgatagacac aggataagag ggaacagaaa ggagaaagag gaagccagga tgggtatttc 13800
tttgcctatc tgtgacttgc acatggtctt ggcaattatt gatgcctcga ggggggggccc 13860
ggtacccagc ttttgttccc tttagtgagg gttaattgcg cgcttggcgt aatcatggtc 13920
atagctgttt cctgtgtgaa attgttatcc gctcacaatt ccacacaaca tacgagccgg 13980
gagcataaag tgtaaagcct ggggtgccta atgagtgagc taactcacat taattgcgtt 14040
gcgctcactg cccgctttcc agtcgggaaa cctgtcgtgc cagctgcatt aatgaatcgg 14100
ccaacgcgcg gggagaggcg gtttgcgtat tgggcgctct tccgcttcct cgctcactga 14160
ctcgctgcgc tcggtcgttc ggctgcggcg agcggtatca gctcactcaa aggcggtaat 14220
acggttatcc acagaatcag gggataacgc aggaaagaac atgtgagcaa aaggccagca 14280
aaaggccagg aaccgtaaaa aggccgcgtt gctggcgttt ttccataggc tccgcccccc 14340
tgacgagcat cacaaaaatc gacgctcaag tcagaggtgg cgaaacccga caggactata 14400
aagataccag gcgtttcccc ctggaagctc cctcgtgcgc tctcctgttc cgaccctgcc 14460
gcttaccgga tacctgtccg cctttctccc ttcgggaagc gtggcgcttt ctcatagctc 14520
acgctgtagg tatctcagtt cggtgtaggt cgttcgctcc aagctgggct gtgtgcacga 14580
accccccgtt cagcccgacc gctgcgcctt atccggtaac tatcgtcttg agtccaaccc 14640
ggtaagacac gacttatcgc cactggcagc agccactggt aacaggatta gcagagcgag 14700
gtatgtaggc ggtgctacag agttcttgaa gtggtggcct aactacggct acactagaag 14760
gacagtattt ggtatctgcg ctctgctgaa gccagttacc ttcggaaaaa gagttggtag 14820
ctcttgatcc ggcaaacaaa ccaccgctgg tagcggtggt ttttttgttt gcaagcagca 14880
gattacgcgc agaaaaaaag gatctcaaga agatcctttg atcttttcta cggggtctga 14940
cgctcagtgg aacgaaaact cacgttaagg gattttggtc atgagattat caaaaaggat 15000
cttcacctag atccttttaa attaaaaatg aagttttaaa tcaatctaaa gtatatatga 15060
gtaaacttgg tctgacagtt accaatgctt aatcagtgag gcacctatct cagcgatctg 15120
tctatttcgt tcatccatag ttgcctgact ccccgtcgtg tagataacta cgatacggga 15180
gggcttacca tctggcccca gtgctgcaat gataccgcga gacccacgct caccggctcc 15240
agatttatca gcaataaacc agccagccgg aagggccgag cgcagaagtg gtcctgcaac 15300
tttatccgcc tccatccagt ctattaattg ttgccgggaa gctagagtaa gtagttcgcc 15360
agttaatagt ttgcgcaacg ttgttgccat tgctacaggc atcgtggtgt cacgctcgtc 15420
gtttggtatg gcttcattca gctccggttc ccaacgatca aggcgagtta catgatcccc 15480
catgttgtgc aaaaaagcgg ttagctcctt cggtcctccg atcgttgtca gaagtaagtt 15540
ggccgcagtg ttatcactca tggttatggc agcactgcat aattctctta ctgtcatgcc 15600

atccgtaaga tgcttttctg tgactggtga gtactcaacc aagtcattct gagaatagtg 15660

tatgcggcga ccgagttgct cttgcccggc gtcaatacgg gataataccg cgccacatag 15720

cagaacttta aaagtgctca tcattggaaa acgttcttcg gggcgaaaac tctcaaggat 15780

cttaccgctg ttgagatcca gttcgatgta acccactcgt gcacccaact gatcttcagc 15840

atcttttact ttcaccagcg tttctgggtg agcaaaaaca ggaaggcaaa atgccgcaaa 15900

aaagggaata agggcgacac ggaaatgttg aatactcata ctcttccttt ttcaatatta 15960

ttgaagcatt tatcagggtt attgtctcat gagcggatac atatttgaat gtatttagaa 16020

aaataaacaa ataggggttc cgcgcacatt tccccgaaaa gtgccaccta aattgtaagc 16080

gttaatattt tgttaaaatt cgcgttaaat ttttgttaaa tcagctcatt ttttaaccaa 16140

taggccgaaa tcggcaaaat cccttataaa tcaaaagaat agaccgagat agggttgagt 16200

gttgttccag tttggaacaa gagtccacta ttaaagaacg tggactccaa cgtcaaaggg 16260

cgaaaaaccg tctatcaggg cgatggccca ctacgtgaac catcacccta atcaagtttt 16320

ttggggtcga ggtgccgtaa agcactaaat cggaacccta aagggagccc ccgatttaga 16380

gcttgacggg gaaagccggc gaacgtggcg agaaaggaag ggaagaaagc gaaaggagcg 16440

ggcgctaggg cgctggcaag tgtagcggtc acgctgcgcg taaccaccac acccgccgcg 16500

cttaatgcgc cgctacaggg cgcgtcccat tcgccattca ggctgcgcaa ctgttgggaa 16560

gggcgatcgg tgcgggcctc ttcgctatta cgccagctgg cgaaaggggg atgtgctgca 16620

aggcgattaa gttgggtaac gccagggttt tcccagtcac gacgttgtaa aacgacggcc 16680

agtgagcgcg cgtaatacga ctcactatag ggcgaattgg agctccaccg cggtggcagc 16740

cgctgg 16746

<210> 129
<211> 7128
<212> DNA
<213> Mus musculus

<400> 129
atcttttttt atgtgtaaga cacaggtttt catgttagga gttaaagtca gttcagaaaa 60

tcttgagaaa atggagaggg ctcattatca gttgacgtgg catacagtgt cagattttct 120

gtttatcaag ctagtgagat taggggcaaa aagaggcttt agttgagagg aaagtaatta 180

atactatggt caccatccaa gagattggat cggagaataa gcatgagtag ttattgagat 240

ctgggtctga ctgcaggtag cgtggtcttc tagacgttta agtgggagat ttggagggga 300

tgaggaatga aggaacttca ggatagaaaa gggctgaagt caagttcagc tcctaaaatg 360

gatgtgggag caaactttga agataaactg aatgacccag aggatgaaac agcgcagatc 420

aaagaggggc ctggagctct gagaagagaa ggagactcat ccgtgttgag tttccacaag 480

tactgtcttg agttttgcaa taaaagtggg atagcagagt tgagtgagcc gtaggctgag 540

```
ttctctcttt tgtctcctaa gtttttatga ctacaaaaat cagtagtatg tcctgaaata    600
atcattaagc tgtttgaaag tatgactgct tgccatgtag ataccatggc ttgctgaata    660
atcagaagag gtgtgactct tattctaaaa tttgtcacaa aatgtcaaaa tgagagactc    720
tgtaggaacg agtccttgac agacagctca aggggttttt ttcctttgtc tcatttctac    780
atgaaagtaa atttgaaatg atcttttta ttataagagt agaaatacag ttgggtttga    840
actatatgtt ttaatggcca cggttttgta agacatttgg tcctttgttt tcccagttat    900
tactcgattg taattttata tcgccagcaa tggactgaaa cggtccgcaa cctcttcttt    960
acaactgggt gacctcgcgg ctgtgccagc catttggcgt tcaccctgcc gctaagggcc   1020
atgtgaaccc ccgcggtagc atcccttgct ccgcgtggac cactttcctg aggcacagtg   1080
ataggaacag agccactaat ctgaagagaa cagagatgtg acagactaca ctaatgtgag   1140
aaaaacaagg aaagggtgac ttattggaga tttcagaaat aaaatgcatt tattattata   1200
ttcccttatt ttaattttct attagggaat tagaaagggc ataaactgct ttatccagtg   1260
ttatattaaa agcttaatgt atataatctt ttagaggtaa aatctacagc cagcaaaagt   1320
catggtaaat attctttgac tgaactctca ctaaactcct ctaaattata tgtcatatta   1380
actggttaaa ttaatataaa tttgtgacat gaccttaact ggttaggtag gatatttttc   1440
ttcatgcaaa aatatgacta ataataattt agcacaaaaa tatttcccaa tactttaatt   1500
ctgtgataga aaaatgttta actcagctac tataatccca taattttgaa aactatttat   1560
tagcttttgt gtttgaccct tccctagcca aaggcaacta tttaaggacc ctttaaaact   1620
cttgaaacta ctttagagtc attaagttat ttaaccactt ttaattactt taaaatgatg   1680
tcaattccct tttaactatt aatttatttt aagggggaa aggctgctca taattctatt   1740
gtttttcttg gtaaagaact ctcagttttc gtttttacta cctctgtcac ccaagagttg   1800
gcatctcaac agaggggact ttccgagagg ccatctggca gttgcttaag atcagaagtg   1860
aagtctgcca gttcctccca ggcaggtggc ccagattaca gttgacctgt tctggtgtgg   1920
ctaaaaattg tcccatgtgg ttacaaacca ttagaccagg gtctgatgaa ttgctcagaa   1980
tatttctgga cacccaaata cagaccctgg cttaaggccc tgtccataca gtaggtttag   2040
cttggctaca ccaaaggaag ccatacagag gctaatatca gagtattctt ggaagagaca   2100
ggagaaaatg aaagccagtt tctgctctta ccttatgtgc ttgtgttcag actcccaaac   2160
atcaggagtg tcagataaac tggtctgaat ctctgtctga agcatggaac tgaaaagaat   2220
gtagtttcag ggaagaaagg caatagaagg aagcctgaga atatcttcaa agggtcagac   2280
tcaatttact ttctaaagaa gtagctagga actagggaat aacttagaaa caacaagatt   2340
gtatatatgt gcatcctggc cccattgttc cttatctgta gggataagcg tgcttttttg   2400
tgtgtctgta tataacataa ctgtttacac ataatacact gaaatggagc ccttccttgt   2460
tacttcatac catcctctgt gcttccttcc tcaggggctg atgctgcacc aactgtatcc   2520
```

atcttcccac catccagtga gcagttaaca tctggaggtg cctcagtcgt gtgcttcttg 2580
aacaacttct accccaaaga catcaatgtc aagtggaaga ttgatggcag tgaacgacaa 2640
aatggcgtcc tgaacagttg gactgatcag gacagcaaag acagcaccta cagcatgagc 2700
agcaccctca cgttgaccaa ggacgagtat gaacgacata acagctatac ctgtgaggcc 2760
actcacaaga catcaacttc acccattgtc aagagcttca acaggaatga gtgttagaga 2820
caaaggtcct gagacgccac caccagctcc ccagctccat cctatcttcc cttctaaggt 2880
cttggaggct tccccacaag cgacctacca ctgttgcggt gctccaaacc tcctccccac 2940
ctccttctcc tcctcctccc tttccttggc ttttatcatg ctaatatttg cagaaaatat 3000
tcaataaagt gagtctttgc acttgagatc tctgtctttc ttactaaatg gtagtaatca 3060
gttgtttttc cagttacctg ggtttctctt ctaaagaagt taaatgttta gttgccctga 3120
aatccaccac acttaaagga taaataaaac cctccacttg ccctggttgg ctgtccacta 3180
catggcagtc ctttctaagg ttcacgagta ctattcatgg cttatttctc tgggccatgg 3240
taggtttgag gaggcatact tcctagtttt cttcccctaa gtcgtcaaag tcctgaaggg 3300
ggacagtctt tacaagcaca tgttctgtaa tctgattcaa cctacccagt aaacttggcg 3360
aagcaaagta gaatcattat cacaggaagc aaaggcaacc taaatgtgca agcaatagga 3420
aaatgtggaa gcccatcata gtacttggac ttcatctgct tttgtgcctt cactaagttt 3480
ttaaacatga gctggctcct atctgccatt ggcaaggctg ggcactaccc acaacctact 3540
tcaaggacct ctataccgtg agattacaca catacatcaa aatttgggaa aagttctacc 3600
aagctgagag ctgatcaccc cactcttagg tgcttatctc tgtacaccag aaaccttaag 3660
aagcaaccag tattgagaga ctcatttatg aaagtctaaa actggataca accaaaatgt 3720
ccaccaacag ttaaattatg acatgttcac aattgagcta ttacttaata aggagaatta 3780
ataaaataaa acttaagagc atagtttaat ctcataaaca agataataag caaaacaaaa 3840
catttttttca tccatgtaag tttaaaagca ggtaaaattt aaaattaaga gagacataag 3900
ttttgaggta gcaagatgga aactctgggg cttggggaat gttctgtctc tctgtatggg 3960
atgtgaaagt tactattgtg gaattgggat ctatgttctt cctgtatata ttgtatactt 4020
cataataact tcacctaaag aaatatctaa tacccagtgc atacataaaa gaggatacaa 4080
ggaatgaatc atacgtcaag gccagaaaga caataaagta ggggatccag gatcaaatct 4140
cccacaacct tgagccttct actattctgc cttccagagc tcaaagtaca aaacacataa 4200
ttcaaacaca tgatccctcc ttggggtctc ttccttcatg catcgaatta gaaatagcca 4260
tgtataaaat gagatagaag agaccttcat caacaggtca aagaatatag gtaattttgt 4320
ctgggtatga agagcccacg tatcaaaggt tacattaggg aaggaagagg acactaacag 4380
tgactttcat tctccccctc ttcctggagg cccctgcatt tagtccctcg tgggctcatc 4440

cactcagcac acatttacta agcatcttct cagcctacac tctgaaggca gtgcagaata 4500
atgttagtgt cccttccccc agttaatatg cagtccagtt tccctgctcc ttccctttct 4560
cagtccacat aaggatgatg ggaaaggaca gtcaccaaat aggagagggc aaccctttgc 4620
cttcctacct cttgagaatg tacattatta tccacttttt gaaacttctt ttaattgctt 4680
ttttttaatt tgtcttttca aatagcataa ccttgttcat ccatttctgg gaaccaaatt 4740
tatcaatcaa cagtgcctct aatctggcta ttaatacaaa aatgcctcct caaaatatat 4800
atgttcgagt cttatctaaa acagaaccca caataaaaaa gaagaaagaa tacatataag 4860
catttatata attctgagca accttgtgct ttgtgaaaaa aatataatct aatgtcacat 4920
gctgtattct ttttatttaa cactggtgaa attataccat tagagagaaa gaggacagat 4980
cactgatcct aggatctagg gatgttacag ataagaaaac aaatgtgaca aagagctgtc 5040
acaaggagga tcttcaaggt cacagaatca ctgtcttgat ttcagtggtg gttacataca 5100
tttaaatatg tgataaaatg ttgttgaact atattcatat attgtaccaa tgtcaaatgc 5160
ttaattttgg ctctatagta taattatgca ctaaataact atttggacaa agaaaatgat 5220
gtttacatca aaggtgaggc catatttgtt aggaacataa cttaaaaacc attttggata 5280
actaatgaaa agccattttg tgtgccttgg catatcatgc ctaagctgtc accagataga 5340
tctaataaga cctaagcctc agaagcaagc ccctgcccag caagcaggca gcacagataa 5400
gagctaaacc caggacaggc catgatatgc taatgaacta ccttcaaggt ggtgttgctg 5460
acctagtgaa ccagccccaa gctgtgagcc ccaatagcac aaagctactg cccaaagaaa 5520
ttatacaaaa attggaactt tgggaatggt gtgcaggatc gctctgctgt atgcctggaa 5580
cacagcttct ctatgttttg tattgatacc agtctagaag cttccaaaac tttctcactg 5640
aagaagattc cccatgtggg acccctacag actcttttgc ccaaacaact gcttccctcc 5700
tggtgtgata tctgttttgc ttttatgtta gcataatatt ataaggaatg tttgtgtgaa 5760
taaaccaaac atattttaaa agcaaatatt gtatgcacat cctaattgct aaaaagttta 5820
cagctaatag tcccatgctc tccacaatac tggatccaaa taagtcctaa tttcaatgtt 5880
gggcatcttt acagagagaa agacattaaa aatgaagaga catgcagaga gtgcaccatg 5940
ccatcgtgga gacagactga agtgacacaa ctgttagtca aagaggatta aggacttcca 6000
gaagccacca aaggaaggag gtatgaagtg gtttctccct cagagtatcc agaggagact 6060
aaaccaacca acaccttttt gcttaagact tcttgccttc aggactgtga gaaggtagct 6120
tcctattgtt ctaagcccca gtatgtggca ttttgttaag gtagagtcaa gaaaccaata 6180
aaatgcagac agacaaaagg atagctgagt tttccaggcc cttccttctt atttttggtt 6240
ttgttggtgg tggtggtggt ggtggtgatg gtggtggttt tgtttatgtt ttgtttgggg 6300
agttttttgg ggtttttttg ggttttgttt ttgttgttgt tttgggggtt tttgttgttg 6360
ttgttgtttg ctttttgtt ttttgtttt tgttttttg agacagtgtt tctctgtata 6420

gccctggctg tcctggagtt ccttctatct ctaatgtcta catctcagag gggatcctct 6480

aatttcaaat gagcagtagc tctccatttt tagctcttat ttattcattt atttacttac 6540

ttacttattg tctgtagatg aaagaatttt ggagtgggaa agggttcatg agcccccagc 6600

aactaatgag gagctacaga caattgatgt ttctggggaa aggagactca gtttctttga 6660

gagtatagct tctgatgggt caaccatgtt cctgtggctg atgtcacacc caggagtatg 6720

cagacaacag aaactggagt taatgagttg ttttaaaaat aaaaaagggc atgaagcttg 6780

ggatagaaat taaggataaa tacaattaaa tacaggaaat tctgaaagaa ttaataaaaa 6840

catttctttt tttaaaaaaa aatccagaat tagctatgct tcttcaaaat tgcttctgga 6900

gaactttaca agttaaataa gttatattgt agaaaaggta gagaggagaa tagtggaaga 6960

gagagataag gagacttcaa aaggagtgga gggagataga ggaggagaaa gcagaagcaa 7020

tggctgatag acacaggata agagggaaca gaaaggagaa agaggaagcc aggatgggta 7080

tttctttgcc tatctgtgac ttgcacatgg tcttggcaat tattgatg 7128

<210> 130
<211> 10867
<212> DNA
<213> Artificial

<220>
<223> Sequence of the targeted allele in ES cells genome

<400> 130
atcttttttt atgtgtaaga cacaggtttt catgttagga gttaaagtca gttcagaaaa 60

tcttgagaaa atggagaggg ctcattatca gttgacgtgg catacagtgt cagattttct 120

gtttatcaag ctagtgagat taggggcaaa aagaggcttt agttgagagg aaagtaatta 180

atactatggt caccatccaa gagattggat cggagaataa gcatgagtag ttattgagat 240

ctgggtctga ctgcaggtag cgtggtcttc tagacgttta agtgggagat ttggagggga 300

tgaggaatga aggaacttca ggatagaaaa gggctgaagt caagttcagc tcctaaaatg 360

gatgtgggag caaactttga agataaactg aatgacccag aggatgaaac agcgcagatc 420

aaagaggggc ctggagctct gagaagagaa ggagactcat ccgtgttgag tttccacaag 480

tactgtcttg agttttgcaa taaaagtggg atagcagagt tgagtgagcc gtaggctgag 540

ttctctcttt tgtctcctaa gtttttatga ctacaaaaat cagtagtatg tcctgaaata 600

atcattaagc tgtttgaaag tatgactgct tgccatgtag ataccatggc ttgctgaata 660

atcagaagag gtgtgactct tattctaaaa tttgtcacaa aatgtcaaaa tgagagactc 720

tgtaggaacg agtccttgac agacagctca aggggttttt ttcctttgtc tcatttctac 780

atgaaagtaa atttgaaatg atcttttta ttataagagt agaaatacag ttgggtttga 840

actatatgtt ttaatggcca cggttttgta agacatttgg tcctttgttt tcccagttat 900

```
tactcgattg taattttata tcgccagcaa tggactgaaa cggtccgcaa cctcttcttt    960
acaactgggt gacctcgcgg ctgtgccagc catttggcgt tcaccctgcc gctaagggcc   1020
atgtgaaccc ccgcggtagc atcccttgct ccgcgtggac cactttcctg aggcacagtg   1080
ataggaacag agccactaat ctgaagagaa cagagatgtg acagactaca ctaatgtgag   1140
aaaaacaagg aaagggtgac ttattggaga tttcagaaat aaaatgcatt tattattata   1200
ttcccttatt ttaattttct attagggaat tagaaagggc ataaactgct ttatccagtg   1260
ttatattaaa agcttaatgt atataatctt ttagaggtaa aatctacagc cagcaaaagt   1320
catggtaaat attctttgac tgaactctca ctaaactcct ctaaattata tgtcatatta   1380
actggttaaa ttaatataaa tttgtgacat gaccttaact ggttaggtag gatatttttc   1440
ttcatgcaaa aatatgacta ataataattt agcacaaaaa tatttcccaa tactttaatt   1500
ctgtgataga aaaatgttta actcagctac tataatccca taattttgaa aactatttat   1560
tagcttttgt gtttgaccct tccctagcca aaggcaacta tttaaggacc ctttaaaact   1620
cttgaaacta ctttagagtc attaagttat ttaaccactt ttaattactt taaaatgatg   1680
tcaattccct tttaactatt aatttatttt aagggggggaa aggctgctca taattctatt   1740
gtttttcttg gtaaagaact ctcagttttc gtttttacta cctctgtcac ccaagagttg   1800
gcatctcaac agaggggact ttccgagagg ccatctggca gttgcttaag atcagaagtg   1860
aagtctgcca gttcctccca ggcaggtggc ccagattaca gttgacctgt tctggtgtgg   1920
ctaaaaattg tcccatgtgg ttacaaacca ttagaccagg gtctgatgaa ttgctcagaa   1980
tatttctgga cacccaaata cagaccctgg cttaaggccc tgtccataca gtaggtttag   2040
cttggctaca ccaaaggaag ccatacagag gctaatatca gagtattctt ggaagagaca   2100
ggagaaaatg aaagccagtt tctgctctta ccttatgtgc ttgtgttcag actcccaaac   2160
atcaggagtg tcagataaac tggtctgaat ctctgtctga agcatggaac tgaaaagaat   2220
gtagtttcag ggaagaaagg caatagaagg aagcctgaga atatcttcaa agggtcagac   2280
tcaatttact ttctaaagaa gtagctagga actagggaat aacttagaaa caacaagatt   2340
gtatatatgt gcatcctggc cccattgttc cttatctgta gggataagcg tgcttttttg   2400
tgtgtctgta tataacataa ctgtttacac ataatacact gaaatggagc ccttccttgt   2460
tacttcatac catcctctgt gcttccttcc tcaggaactg tggctgcacc atctgtcttc   2520
atcttcccgc catctgatga gcagttgaaa tctggaactg cctctgttgt gtgcctgctg   2580
aataacttct atcccagaga ggccaaagta cagtggaagg tggataacgc cctccaatcg   2640
ggtaactccc aggagagtgt cacagagcag gacagcaagg acagcaccta cagcctcagc   2700
agcaccctga cgctgagcaa agcagactac gagaaacaca aagtctacgc ctgcgaagtc   2760
acccatcagg gcctgagctc gcccgtcaca aagagcttca acaggggaga gtgtggttcc   2820
ggttccggtt ccggttccct gcccgagaca ggaggacatc atcatcatca tcattgaaga   2880
```

caaacccgat ctcgaggtcg actcgcgaaa gctagcttat aacttcgtat agcatacatt 2940
atacgaagtt atggatctcc atgggccagg caaatatccc ttaccagcct cacagagacc 3000
tcccccaccc cccgcaaccc tagagttctt ttactagtga gggacaagtg gacaatggtg 3060
ctgttgtggg ccccaccctg tgtcccctgt gcccacagtg gtcactctgc ttggcaggca 3120
ggtgttgcag gctggctgct ccaggccctg gcaggaggta ctgaaggacc tggtaggctc 3180
agatgccctg gatgccaagg cactgctgga gtacttccaa ccggtcagcc agtggctgga 3240
agagcagaat cagcggaatg gcgaagtcct aggctggcca gagaatcagt ggcgtccacc 3300
gttacccgac aactatccag agggcattgg taaagctctg agtgagggtg gactgggacc 3360
aagagaagtc ctggcctctg gcctctggct tctgggtcaa agcctcagca tcctggtcac 3420
tttgctgcca gctgagcccc agtgtccttt gcttcagtgc caagccaccc ctgggctcat 3480
cctcagggcc ctaagcagaa atgggtatgt ctttctctca gggtcctaga gacagtgtgc 3540
ccaagcctga gggcccttgg ggtcaggctg gctggcacat tgctctatga ggtcacactg 3600
caggcttggc tcttattggc cggtgatggg agcttcaggg ctctgctttc ctgcggccat 3660
gcccaagaag aagaggaagg tgtccaattt actgaccgta caccaaaatt tgcctgcatt 3720
accggtcgat gcaacgagtg atgaggttcg caagaacctg atggacatgt tcagggatcg 3780
ccaggcgttt tctgagcata cctggaaaat gcttctgtcc gtttgccggt cgtgggcggc 3840
atggtgcaag ttgaataacc ggaaatggtt tcccgcagaa cctgaagatg ttcgcgatta 3900
tcttctatat cttcaggcgc gcggtctggc agtaaaaact atccagcaac atttgggcca 3960
gctaaacatg cttcatcgtc ggtccgggct gccacgacca agtgacagca atgctgtttc 4020
actggttatg cggcggatcc gaaaagaaaa cgttgatgcc ggtgaacgtg caaaacaggc 4080
tctagcgttc gaacgcactg atttcgacca ggttcgttca ctcatggaaa atagcgatcg 4140
ctgccaggat atacgtaatc tggcatttct ggggattgct tataacaccc tgttacgtat 4200
agccgaaatt gccaggatca gggttaaaga tatctcacgt actgacggtg ggagaatgtt 4260
aatccatatt ggcagaacga aaacgctggt tagcaccgca ggtgtagaga aggcacttag 4320
cctgggggta actaaactgg tcgagcgatg gatttccgtc tctggtgtag ctgatgatcc 4380
gaataactac ctgttttgcc gggtcagaaa aaatggtgtt gccgcgccat ctgccaccag 4440
ccagctatca actcgcgccc tggaagggat ttttgaagca actcatcgat tgatttacgg 4500
cgctaaggta aatataaaat ttttaagtgt ataatgtgtt aaactactga ttctaattgt 4560
ttgtgtattt taggatgact ctggtcagag atacctggcc tggtctggac acagtgcccg 4620
tgtcggagcc gcgcgagata tggcccgcgc tggagtttca ataccggaga tcatgcaagc 4680
tggtggctgg accaatgtaa atattgtcat gaactatatc cgtaacctgg atagtgaaac 4740
aggggcaatg gtgcgcctgc tggaagatgg cgattagcca ttaacgcgta aatgattgct 4800

ataattattt gatatttatg gtgacatatg agaaaggatt tcaacatcga cggaaaatat 4860
gtagtgctgt ctgtaagcac taatattcag tcgccagccg tcattgtcac tgtaaagctg 4920
agcggcaata aaaagacaga ataaaacgca cgggtgttgg gtcgtttgtt cggtcgagct 4980
cgcgaaagct tctaccgggt aggggaggcg cttttcccaa ggcagtctgg agcatgcgct 5040
ttagcagccc cgctgggcac ttggcgctac acaagtggcc tctggcctcg cacacattcc 5100
acatccaccg gtaggcgcca accggctccg ttctttggtg gccccgtcgc gccaccttct 5160
actcctcccc tagtcaggaa gttccccccc gccccgcagc tcgcgtcgtg caggacgtga 5220
caaatggaag tagcacgtct cactagtctc gtgcagatgg acagcaccgc tgagcaatgg 5280
aagcgggtag gcctttgggg cagcggccaa tagcagcttt gctccttcgc tttctgggct 5340
cagaggctgg gaaggggtgg gtccgggggc gggctcaggg gcgggctcag gggcggggcg 5400
ggcgcccgaa ggtcctccgg aggcccggca ttctgcacgc ttcaaaagcg cacgtctgcc 5460
gcgctgttct cctcttcctc atctccgggc ctttcgacct gcagcagcac gtgttgacaa 5520
ttaatcatcg gcatagtata tcggcatagt ataatacgac aaggtgagga actaaaccat 5580
gggatcggcc attgaacaag atggattgca cgcaggttct ccggccgctt gggtggagag 5640
gctattcggc tatgactggg cacaacagac gatcggctgc tctgatgccg ccgtgttccg 5700
gctgtcagcg caggggcgcc cggttctttt tgtcaagacc gacctgtccg gtgccctgaa 5760
tgaactgcag gacgaggcag cgcggctatc gtggctggcc acgacgggcg ttccttgcgc 5820
agctgtgctc gacgttgtca ctgaagcggg aagggactgg ctgctattgg gcgaagtgcc 5880
ggggcaggat ctcctgtcat ctcaccttgc tcctgccgag aaagtatcca tcatggctga 5940
tgcaatgcgg cggctgcata cgcttgatcc ggctacctgc ccattcgacc accaagcgaa 6000
acatcgcatc gagcgagcac gtactcggat ggaagccggt cttgtcgatc aggatgatct 6060
ggacgaagag catcaggggc tcgcgccagc cgaactgttc gccaggctca aggcgcgcat 6120
gcccgacggc gaggatctcg tcgtgaccca tggcgatgcc tgcttgccga atatcatggt 6180
ggaaaatggc cgcttttctg gattcatcga ctgtggccgg ctgggtgtgg cggaccgcta 6240
tcaggacata gcgttggcta cccgtgatat tgctgaagag cttggcggcg aatgggctga 6300
ccgcttcctc gtgctttacg gtatcgccgc ccccgattcg cagcgcatcg ccttctatcg 6360
ccttcttgac gagttcttct gagcgggact ctggggttcg aataaagacc gaccaagcga 6420
cgtctgagag ctccctggcg aattcggtac caataaaaga gctttatttt catgatctgt 6480
gtgttggttt ttgtgtgcgg cgcgataact tcgtatagca tacattatac gaagttatcc 6540
caattcatcg atatctagat cggggtcctg agacgccacc accagctccc cagctccatc 6600
ctatcttccc ttctaaggtc ttggaggctt ccccacaagc gacctaccac tgttgcggtg 6660
ctccaaacct cctccccacc tccttctcct cctcctccct ttccttggct tttatcatgc 6720
taatatttgc agaaaatatt caataaagtg agtctttgca cttgagatct ctgtctttct 6780

tactaaatgg tagtaatcag ttgtttttcc agttacctgg gtttctcttc taaagaagtt 6840
aaatgtttag ttgccctgaa atccaccaca cttaaaggat aaataaaacc ctccacttgc 6900
cctggttggc tgtccactac atggcagtcc tttctaaggt tcacgagtac tattcatggc 6960
ttatttctct gggccatggt aggtttgagg aggcatactt cctagttttc ttcccctaag 7020
tcgtcaaagt cctgaagggg gacagtcttt acaagcacat gttctgtaat ctgattcaac 7080
ctacccagta aacttggcga agcaaagtag aatcattatc acaggaagca aaggcaacct 7140
aaatgtgcaa gcaataggaa aatgtggaag cccatcatag tacttggact tcatctgctt 7200
ttgtgccttc actaagtttt taaacatgag ctggctccta tctgccattg gcaaggctgg 7260
gcactaccca caacctactt caaggacctc tataccgtga gattacacac atacatcaaa 7320
atttgggaaa agttctacca agctgagagc tgatcacccc actcttaggt gcttatctct 7380
gtacaccaga aaccttaaga agcaaccagt attgagagac tcatttatga aagtctaaaa 7440
ctggatacaa ccaaaatgtc caccaacagt taaattatga catgttcaca attgagctat 7500
tacttaataa ggagaattaa taaaataaaa cttaagagca tagtttaatc tcataaacaa 7560
gataataagc aaaacaaaac attttttcat ccatgtaagt ttaaaagcag gtaaaattta 7620
aaattaagag agacataagt tttgaggtag caagatggaa actctggggc ttggggaatg 7680
ttctgtctct ctgtatggga tgtgaaagtt actattgtgg aattgggatc tatgttcttc 7740
ctgtatatat tgtatacttc ataataactt cacctaaaga aatatctaat acccagtgca 7800
tacataaaag aggatacaag gaatgaatca tacgtcaagg ccagaaagac aataaagtag 7860
gggatccagg atcaaatctc ccacaacctt gagccttcta ctattctgcc ttccagagct 7920
caaagtacaa aacacataat tcaaacacat gatccctcct tggggtctct tccttcatgc 7980
atcgaattag aaatagccat gtataaaatg agatagaaga gaccttcatc aacaggtcaa 8040
agaatatagg taattttgtc tgggtatgaa gagcccacgt atcaaaggtt acattaggga 8100
aggaagagga cactaacagt gactttcatt ctccccctct tcctggaggc ccctgcattt 8160
agtccctcgt gggctcatcc actcagcaca catttactaa gcatcttctc agcctacact 8220
ctgaaggcag tgcagaataa tgttagtgtc ccttccccca gttaatatgc agtccagttt 8280
ccctgctcct tccctttctc agtccacata aggatgatgg gaaaggacag tcaccaaata 8340
ggagagggca accctttgcc ttcctacctc ttgagaatgt acattattat ccactttttg 8400
aaacttcttt taattgcttt tttttaattt gtcttttcaa atagcataac cttgttcatc 8460
catttctggg aaccaaattt atcaatcaac agtgcctcta atctggctat taatacaaaa 8520
atgcctcctc aaaatatata tgttcgagtc ttatctaaaa cagaacccac aataaaaaag 8580
aagaaagaat acatataagc atttatataa ttctgagcaa ccttgtgctt tgtgaaaaaa 8640
atataatcta atgtcacatg ctgtattctt tttatttaac actggtgaaa ttataccatt 8700

```
agagagaaag aggacagatc actgatccta ggatctaggg atgttacaga taagaaaaca    8760
aatgtgacaa agagctgtca caaggaggat cttcaaggtc acagaatcac tgtcttgatt    8820
tcagtggtgg ttacatacat ttaaatatgt gataaaatgt tgttgaacta tattcatata    8880
ttgtaccaat gtcaaatgct taattttggc tctatagtat aattatgcac taaataacta    8940
tttggacaaa gaaaatgatg tttacatcaa aggtgaggcc atatttgtta ggaacataac    9000
ttaaaaacca ttttggataa ctaatgaaaa gccattttgt gtgccttggc atatcatgcc    9060
taagctgtca ccagatagat ctaataagac ctaagcctca gaagcaagcc cctgcccagc    9120
aagcaggcag cacagataag agctaaaccc aggacaggcc atgatatgct aatgaactac    9180
cttcaaggtg gtgttgctga cctagtgaac cagccccaag ctgtgagccc caatagcaca    9240
aagctactgc ccaaagaaat tatacaaaaa ttggaacttt gggaatggtg tgcaggatcg    9300
ctctgctgta tgcctggaac acagcttctc tatgttttgt attgatacca gtctagaagc    9360
ttccaaaact ttctcactga agaagattcc ccatgtggga cccctacaga ctcttttgcc    9420
caaacaactg cttccctcct ggtgtgatat ctgttttgct tttatgttag cataatatta    9480
taaggaatgt ttgtgtgaat aaaccaaaca tattttaaaa gcaaatattg tatgcacatc    9540
ctaattgcta aaaagtttac agctaatagt cccatgctct ccacaatact ggatccaaat    9600
aagtcctaat ttcaatgttg ggcatcttta cagagagaaa gacattaaaa atgaagagac    9660
atgcagagag tgcaccatgc catcgtggag acagactgaa gtgacacaac tgttagtcaa    9720
agaggattaa ggacttccag aagccaccaa aggaaggagg tatgaagtgg tttctccctc    9780
agagtatcca gaggagacta aaccaaccaa cacctttttg cttaagactt cttgccttca    9840
ggactgtgag aaggtagctt cctattgttc taagccccag tatgtggcat tttgttaagg    9900
tagagtcaag aaaccaataa aatgcagaca gacaaaagga tagctgagtt ttccaggccc    9960
ttccttctta tttttggttt tgttggtggt ggtggtggtg gtggtgatgg tggtggtttt   10020
gtttatgttt tgtttgggga gtttttttggg gtttttttgg gttttgtttt tgttgttgtt   10080
ttgggggttt ttgttgttgt tgttgtttgc tttttttgttt tttgtttttt gtttttttga   10140
gacagtgttt ctctgtatag ccctggctgt cctggagttc cttctatctc taatgtctac   10200
atctcagagg ggatcctcta atttcaaatg agcagtagct ctccattttt agctcttatt   10260
tattcattta tttacttact tacttattgt ctgtagatga aagaattttg gagtgggaaa   10320
gggttcatga gcccccagca actaatgagg agctacagac aattgatgtt tctggggaaa   10380
ggagactcag tttctttgag agtatagctt ctgatgggtc aaccatgttc ctgtggctga   10440
tgtcacaccc aggagtatgc agacaacaga aactggagtt aatgagttgt tttaaaaata   10500
aaaaagggca tgaagcttgg gatagaaatt aaggataaat acaattaaat acaggaaatt   10560
ctgaaagaat taataaaaac atttcttttt ttaaaaaaaa atccagaatt agctatgctt   10620
cttcaaaatt gcttctggag aactttacaa gttaaataag ttatattgta gaaaaggtag   10680
```

agaggagaat agtggaagag agagataagg agacttcaaa aggagtggag ggagatagag 10740

gaggagaaag cagaagcaat ggctgataga cacaggataa gagggaacag aaaggagaaa 10800

gaggaagcca ggatgggtat ttctttgcct atctgtgact tgcacatggt cttggcaatt 10860

attgatg 10867

<210> 131
<211> 7278
<212> DNA
<213> Artificial

<220>
<223> Final sequence of the targeted allele in a genome of mutant mice

<400> 131
atcttttttt atgtgtaaga cacaggtttt catgttagga gttaaagtca gttcagaaaa 60

tcttgagaaa atggagaggg ctcattatca gttgacgtgg catacagtgt cagattttct 120

gtttatcaag ctagtgagat taggggcaaa aagaggcttt agttgagagg aaagtaatta 180

atactatggt caccatccaa gagattggat cggagaataa gcatgagtag ttattgagat 240

ctgggtctga ctgcaggtag cgtggtcttc tagacgttta agtgggagat ttggagggga 300

tgaggaatga aggaacttca ggatagaaaa gggctgaagt caagttcagc tcctaaaatg 360

gatgtgggag caaactttga agataaactg aatgacccag aggatgaaac agcgcagatc 420

aaagaggggc ctggagctct gagaagagaa ggagactcat ccgtgttgag tttccacaag 480

tactgtcttg agttttgcaa taaaagtggg atagcagagt tgagtgagcc gtaggctgag 540

ttctctcttt tgtctcctaa gtttttatga ctacaaaaat cagtagtatg tcctgaaata 600

atcattaagc tgtttgaaag tatgactgct tgccatgtag ataccatggc ttgctgaata 660

atcagaagag gtgtgactct tattctaaaa tttgtcacaa aatgtcaaaa tgagagactc 720

tgtaggaacg agtccttgac agacagctca aggggttttt ttcctttgtc tcatttctac 780

atgaaagtaa atttgaaatg atctttttta ttataagagt agaaatacag ttgggtttga 840

actatatgtt ttaatggcca cggttttgta agacatttgg tcctttgttt tcccagttat 900

tactcgattg taattttata tcgccagcaa tggactgaaa cggtccgcaa cctcttcttt 960

acaactgggt gacctcgcgg ctgtgccagc catttggcgt tcaccctgcc gctaagggcc 1020

atgtgaaccc ccgcggtagc atcccttgct ccgcgtggac cactttcctg aggcacagtg 1080

ataggaacag agccactaat ctgaagagaa cagagatgtg acagactaca ctaatgtgag 1140

aaaaacaagg aaagggtgac ttattggaga tttcagaaat aaaatgcatt tattattata 1200

ttcccttatt ttaattttct attagggaat tagaaagggc ataaactgct ttatccagtg 1260

ttatattaaa agcttaatgt atataatctt ttagaggtaa aatctacagc cagcaaaagt 1320

catggtaaat attctttgac tgaactctca ctaaactcct ctaaattata tgtcatatta 1380

actggttaaa ttaatataaa tttgtgacat gaccttaact ggttaggtag gatatttttc 1440
ttcatgcaaa aatatgacta ataataattt agcacaaaaa tatttcccaa tactttaatt 1500
ctgtgataga aaaatgttta actcagctac tataatccca taattttgaa aactatttat 1560
tagcttttgt gtttgaccct tccctagcca aaggcaacta tttaaggacc ctttaaaact 1620
cttgaaacta ctttagagtc attaagttat ttaaccactt ttaattactt taaaatgatg 1680
tcaattccct tttaactatt aatttatttt aagggggaa aggctgctca taattctatt 1740
gtttttcttg gtaaagaact ctcagttttc gtttttacta cctctgtcac ccaagagttg 1800
gcatctcaac agaggggact ttccgagagg ccatctggca gttgcttaag atcagaagtg 1860
aagtctgcca gttcctccca ggcaggtggc ccagattaca gttgacctgt tctggtgtgg 1920
ctaaaaattg tcccatgtgg ttacaaacca ttagaccagg gtctgatgaa ttgctcagaa 1980
tatttctgga cacccaaata cagaccctgg cttaaggccc tgtccataca gtaggtttag 2040
cttggctaca ccaaaggaag ccatacagag gctaatatca gagtattctt ggaagagaca 2100
ggagaaaatg aaagccagtt tctgctctta ccttatgtgc ttgtgttcag actcccaaac 2160
atcaggagtg tcagataaac tggtctgaat ctctgtctga agcatggaac tgaaaagaat 2220
gtagtttcag ggaagaaagg caatagaagg aagcctgaga atatcttcaa agggtcagac 2280
tcaatttact ttctaaagaa gtagctagga actagggaat aacttagaaa caacaagatt 2340
gtatatatgt gcatcctggc cccattgttc cttatctgta gggataagcg tgcttttttg 2400
tgtgtctgta tataacataa ctgtttacac ataatacact gaaatggagc ccttccttgt 2460
tacttcatac catcctctgt gcttccttcc tcaggaactg tggctgcacc atctgtcttc 2520
atcttcccgc catctgatga gcagttgaaa tctggaactg cctctgttgt gtgcctgctg 2580
aataacttct atcccagaga ggccaaagta cagtggaagg tggataacgc cctccaatcg 2640
ggtaactccc aggagagtgt cacagagcag gacagcaagg acagcaccta cagcctcagc 2700
agcaccctga cgctgagcaa agcagactac gagaaacaca aagtctacgc ctgcgaagtc 2760
acccatcagg gcctgagctc gcccgtcaca aagagcttca acaggggaga gtgtggttcc 2820
ggttccggtt ccggttccct gcccacagag ggacatcatc atcatcatca ttgaagacaa 2880
acccgatctc gaggtcgact cgcgaaagct agcttataac ttcgtatagc atacattata 2940
cgaagttatc ccaattcatc gatatctaga tcggggtcct gagacgccac caccagctcc 3000
ccagctccat cctatcttcc cttctaaggt cttggaggct tccccacaag cgacctacca 3060
ctgttgcggt gctccaaacc tcctccccac ctccttctcc tcctcctccc tttccttggc 3120
ttttatcatg ctaatatttg cagaaaatat tcaataaagt gagtctttgc acttgagatc 3180
tctgtctttc ttactaaatg gtagtaatca gttgtttttc cagttacctg ggtttctctt 3240
ctaaagaagt taaatgttta gttgccctga aatccaccac acttaaagga taaataaaac 3300
cctccacttg ccctggttgg ctgtccacta catggcagtc ctttctaagg ttcacgagta 3360

ctattcatgg cttatttctc tgggccatgg taggtttgag gaggcatact tcctagtttt 3420
cttcccctaa gtcgtcaaag tcctgaaggg ggacagtctt tacaagcaca tgttctgtaa 3480
tctgattcaa cctacccagt aaacttggcg aagcaaagta gaatcattat cacaggaagc 3540
aaaggcaacc taaatgtgca agcaatagga aaatgtggaa gcccatcata gtacttggac 3600
ttcatctgct tttgtgcctt cactaagttt ttaaacatga gctggctcct atctgccatt 3660
ggcaaggctg ggcactaccc acaacctact tcaaggacct ctataccgtg agattacaca 3720
catacatcaa aatttgggaa aagttctacc aagctgagag ctgatcaccc cactcttagg 3780
tgcttatctc tgtacaccag aaaccttaag aagcaaccag tattgagaga ctcatttatg 3840
aaagtctaaa actggataca accaaaatgt ccaccaacag ttaaattatg acatgttcac 3900
aattgagcta ttacttaata aggagaatta ataaaataaa acttaagagc atagtttaat 3960
ctcataaaca agataataag caaaacaaaa catttttca tccatgtaag tttaaaagca 4020
ggtaaaattt aaaattaaga gagacataag ttttgaggta gcaagatgga aactctgggg 4080
cttggggaat gttctgtctc tctgtatggg atgtgaaagt tactattgtg gaattgggat 4140
ctatgttctt cctgtatata ttgtatactt cataataact tcacctaaag aaatatctaa 4200
tacccagtgc atacataaaa gaggatacaa ggaatgaatc atacgtcaag gccagaaaga 4260
caataaagta ggggatccag gatcaaatct cccacaacct tgagccttct actattctgc 4320
cttccagagc tcaaagtaca aaacacataa ttcaaacaca tgatccctcc ttggggtctc 4380
ttccttcatg catcgaatta gaaatagcca tgtataaaat gagatagaag agaccttcat 4440
caacaggtca aagaatatag gtaattttgt ctgggtatga agagcccacg tatcaaaggt 4500
tacattaggg aaggaagagg acactaacag tgactttcat tctcccctc ttcctggagg 4560
cccctgcatt tagtccctcg tgggctcatc cactcagcac acatttacta agcatcttct 4620
cagcctacac tctgaaggca gtgcagaata atgttagtgt cccttccccc agttaatatg 4680
cagtccagtt tccctgctcc ttccctttct cagtccacat aaggatgatg ggaaaggaca 4740
gtcaccaaat aggagaggc aacccctttgc cttcctacct cttgagaatg tacattatta 4800
tccacttttt gaacttcttt ttaattgctt tttttaatt tgtcttttca aatagcataa 4860
ccttgttcat ccatttctgg gaaccaaatt tatcaatcaa cagtgcctct aatctggcta 4920
ttaatacaaa aatgcctcct caaaatatat atgttcgagt cttatctaaa acagaaccca 4980
caataaaaaa gaagaaagaa tacatataag catttatata attctgagca accttgtgct 5040
ttgtgaaaaa aatataatct aatgtcacat gctgtattct ttttatttaa cactggtgaa 5100
attataccat tagagagaaa gaggacagat cactgatcct aggatctagg gatgttacag 5160
ataagaaaac aaatgtgaca aagagctgtc acaaggagga tcttcaaggt cacagaatca 5220
ctgtcttgat ttcagtggtg gttacataca tttaaatatg tgataaaatg ttgttgaact 5280

atattcatat attgtaccaa tgtcaaatgc ttaattttgg ctctatagta taattatgca 5340
ctaaataact atttggacaa agaaaatgat gtttacatca aaggtgaggc catatttgtt 5400
aggaacataa cttaaaaacc attttggata actaatgaaa agccattttg tgtgccttgg 5460
catatcatgc ctaagctgtc accagataga tctaataaga cctaagcctc agaagcaagc 5520
ccctgcccag caagcaggca gcacagataa gagctaaacc caggacaggc catgatatgc 5580
taatgaacta ccttcaaggt ggtgttgctg acctagtgaa ccagccccaa gctgtgagcc 5640
ccaatagcac aaagctactg cccaaagaaa ttatacaaaa attggaactt tgggaatggt 5700
gtgcaggatc gctctgctgt atgcctggaa cacagcttct ctatgttttg tattgatacc 5760
agtctagaag cttccaaaac tttctcactg aagaagattc cccatgtggg acccctacag 5820
actcttttgc ccaaacaact gcttccctcc tggtgtgata tctgttttgc ttttatgtta 5880
gcataatatt ataaggaatg tttgtgtgaa taaaccaaac atattttaaa agcaaatatt 5940
gtatgcacat cctaattgct aaaaagttta cagctaatag tcccatgctc tccacaatac 6000
tggatccaaa taagtcctaa tttcaatgtt gggcatcttt acagagagaa agacattaaa 6060
aatgaagaga catgcagaga gtgcaccatg ccatcgtgga gacagactga agtgacacaa 6120
ctgttagtca aagaggatta aggacttcca gaagccacca aaggaaggag gtatgaagtg 6180
gtttctccct cagagtatcc agaggagact aaaccaacca acaccttttt gcttaagact 6240
tcttgccttc aggactgtga gaaggtagct tcctattgtt ctaagcccca gtatgtggca 6300
ttttgttaag gtagagtcaa gaaaccaata aaatgcagac agacaaaagg atagctgagt 6360
tttccaggcc cttccttctt atttttggtt ttgttggtgg tggtggtggt ggtggtgatg 6420
gtggtggttt tgtttatgtt ttgtttgggg agtttttttgg ggtttttttg ggttttgttt 6480
ttgttgttgt tttgggggtt tttgttgttg ttgttgtttg ctttttttgtt ttttgttttt 6540
tgttttttttg agacagtgtt tctctgtata gccctggctg tcctggagtt ccttctatct 6600
ctaatgtcta catctcagag gggatcctct aatttcaaat gagcagtagc tctccatttt 6660
tagctcttat ttattcattt atttacttac ttacttattg tctgtagatg aaagaatttt 6720
ggagtgggaa agggttcatg agcccccagc aactaatgag gagctacaga caattgatgt 6780
ttctggggaa aggagactca gtttctttga gagtatagct tctgatgggt caaccatgtt 6840
cctgtggctg atgtcacacc caggagtatg cagacaacag aaactggagt taatgagttg 6900
ttttaaaaat aaaaaagggc atgaagcttg ggatagaaat taaggataaa tacaattaaa 6960
tacaggaaat tctgaaagaa ttaataaaaa catttctttt tttaaaaaaa aatccagaat 7020
tagctatgct tcttcaaaat tgcttctgga gaactttaca agttaaataa gttatattgt 7080
agaaaaggta gagaggagaa tagtggaaga gagagataag gagacttcaa aaggagtgga 7140
gggagataga ggaggagaaa gcagaagcaa tggctgatag acacaggata agagggaaca 7200
gaaaggagaa agaggaagcc aggatgggta tttctttgcc tatctgtgac ttgcacatgg 7260

tcttggcaat tattgatg 7278

## Description

### FIELD OF THE INVENTION:

The present invention relates to a fusion protein consisting of a constant domain (CL) of a light chain of an immunoglobulin fused at its C-terminal end to a polypeptide (P) characterized by the sequence:

LPXT(G)n (P)

wherein n is an integer number and X represents an amino acid other than tryptophan and cysteine.

### BACKGROUND OF THE INVENTION:

There is an increasing demand for the specific incorporation of novel functionalities into immunoglobulins. In the context, two examples of such functionalities may be provided to illustrate the potential of such functionalities. First, news optical detection approaches of fluorescently labeled proteins at the single molecular level have improved the spatial resolution down to the nanoscale. However, the very nature of such novel approaches - called nanoscopy - require the development of a new type of molecular probe that is both monovalent and conjugated to a single photo-responsive molecule. It is presently difficult to assemble such complex bioconjugates in a single-step by standard recombinant protein techniques. Second, in the field of vaccination, an important line of research aims at improving the immunogenicity of antigens by delivering them directly to dendritic cells. For that purpose, the candidate vaccinal antigen is covalently coupled to an antibody specific for a molecule expressed at the surface of the dendritic cell subset to be targeted. The covalent coupling between the vaccinal antigen and the antibody is generally achieved via chemistry (a process not applicable to certain mAbs due to the presence of chemically reactive groups within the antigen-binding site) or via engineering of the heavy chain of the antibody under study (a process that needs to be repeated "from scratch" for each mAb).

### SUMMARY OF THE INVENTION:

The present invention relates to a fusion protein consisting of a constant domain (CL) of a light chain of an immunoglobulin fused at its C-terminal end to a polypeptide (P) characterized by the sequence:

LPXT(G)n (P)

wherein n is an integer number and X represents an amino acid other than tryptophan and cysteine.
The invention also relates to an antibody wherein the constant domain of the light chain is replaced by a fusion protein according to the invention.
At last, the invention also relates to a transgenic non-human mammal genetically modified to express an antibody according to the invention.

### DETAILED DESCRIPTION OF THE INVENTION:

The inventors have created an innovative animal model (i.e. a mouse) intended to produce monoclonal antibodies (mAbs) that can be readily modified with natural (e.g. fluorescent proteins, vaccinal antigens...) or unnatural functionalities (e.g. quantum dots, see for example Popp et al. 2007) under physiological conditions. In particular, the genetic code of the animal is modified in a manner that the light chains of the immunoglobulins are fused to a sequence recognized by sortases. Sortases are transpeptidase enzymes that are found in most Grampositive bacteria (Antos et al., 2009a; Antos et al., 2009b; Tsukiji and Nagamune, 2009), and that have been recently shown to be capable of ligating distinct protein species. SrtA recognizes polypeptides that contain an LPETG sequence close to or at the C terminus of a given protein and catalyzes the cleavage of the amide bond between threonine and the glycine by means of an active-site cysteine residue. This process generates a covalent acyl-enzyme intermediate. Accordingly, the antibodies produced by the animal model may be easily conjugated to a protein or a polypeptide of interest by using a sortase.

#### *Definitions:*

Throughout the specification, several terms are employed and are defined in the following paragraphs.
The terms "protein", "polypeptide", and "peptide" are used herein interchangeably, and refer to amino acid sequences of a variety of lengths, either in their neutral (uncharged) forms or as salts, and either unmodified or modified by glycosylation, side-chain oxidation, or phosphorylation. In certain embodiments, the amino acid sequence is a full-length native protein. In other embodiments, the amino acid sequence is a smaller fragment of the full-length protein. In still other embodiments, the amino acid sequence is modified by additional substituents attached to the amino acid side chains, such as glycosyl units, lipids, or inorganic ions such as phosphates, as well as modifications relating to chemical conversions of the chains such as oxidation of sulfydryl groups. Thus, the term "protein" (or its equivalent terms) is intended to include the amino acid sequence of the full-length native protein, or a fragment thereof, subject to those modifications that do not significantly change its specific properties. In particular, the term "protein" encompasses protein isoforms, i.e., variants that are encoded by the same gene, but that differ in their pI or MW, or both. Such isoforms can differ in their amino acid sequence (e.g., as a result of allelic variation, alternative splicing or limited proteolysis), or in the alternative, may arise from differential post-translational modification (e.g., glycosylation, acylation, phosphorylation).

According to the present invention, "immunoglobulin" or "antibody" have the same meaning, and will be used equally in the present invention. In natural antibodies, the two heavy chains are linked to each other by disulfide bonds and each heavy chain is linked to a light chain by a disulfide bond. There are two types of light chain, lambda ($\lambda$) and kappa ($\kappa$). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, transplacental mobility, complement binding, and binding to Fc receptors. The Fv fragment is the N-terminal part of the Fab fragment of an immunoglobulin consisting of the variable domains of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. The CDR refers to amino acid sequences which together define the binding affinity and specificity of the natural Fv region of a native immunoglobulin binding site. The light and heavy chain variable domains of an immunoglobulin have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. FR refers to amino acid sequences interposed between CDRs.

The term "monoclonal antibody" or "mAb" as used herein refers to an antibody molecule of a single amino acid composition, that is directed against a specific antigen and that is produced by a single clone of B cells or hybridoma. Accordingly, the term "hybridoma" denotes a cell, which is obtained by subjecting a B cell prepared by immunizing an animal with an antigen to cell fusion with a myeloma cell derived from a mouse or the like which produces a desired monoclonal antibody having an antigen specificity.

The term "chimeric antibody" refers to an antibody which comprises a VH domain and a VL domain of an antibody and a CH domain and a CL domain of a human antibody. Methods for producing chimeric antibodies involve conventional recombinant DNA and gene transfection techniques are well known in the art (See Morrison SL. et al. (1984) and patent documents US5,202,238; and US5,204, 244).

According to the invention, the term "humanized antibody" refers to an antibody having variable region framework and constant regions from a human antibody but retains the CDRs of a murine antibody. In one embodiment, the antibody has variable region from mouse and constant region from human. The humanized antibody of the present invention may be produced by obtaining nucleic acid sequences encoding CDR domains, as previously described, constructing a humanized antibody expression vector by inserting them into an expression vector for animal cell having genes encoding (i) a heavy chain constant region identical to that of a human antibody and (ii) a light chain constant region identical to that of a human antibody, and expressing the genes by introducing the expression vector into an animal cell. The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, humanized antibody expression vector of the tandem type is preferred (Shitara K et al. 1994). Examples of tandem type humanized antibody expression vector include pKANTEX93 (WO 97/10354), pEE18 and the like. Methods for producing humanized antibodies based on conventional recombinant DNA and gene transfection techniques are well known in the art (See, e. g., Riechmann L. et al. 1988; Neuberger MS. et al. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM et al. (1994); Roguska MA. et al. (1994)), and chain shuffling (U.S. Pat. No.5,565,332). The general recombinant DNA technology for preparation of such antibodies is also known (see European Patent Application EP 125023 and International Patent Application WO 96/02576).

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond. The Fab of the present invention can be obtained by treating an antibody with a protease, papaine. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin. The F(ab')2 of the present invention can be obtained treating an antibody with a protease, pepsin. Also, the F(ab')2 can be produced by binding Fab' described below via a thioether bond or a disulfide bond.

The term "Fab' " refers to an antibody fragment having a molecular weight of about 50,000 and antigen binding activity, which is obtained by cutting a disulfide bond of the hinge region of the F(ab')2. The Fab' of the present invention can be obtained treating F(ab')2 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

The term "sortase A" is used herein to refer to a class A sortase, usually named SrtA in any particular bacterial species, e.g., SrtA from *S. aureus* (Mazmanian,S.K., Liu,G., Ton-That,H. and Schneewind,O. Staphylococcus aureus sortase, an enzyme that anchors surface proteins to the cell wall Science 285 (5428), 760-763 (1999)). An exemplary amino acid sequence for Srt A is accessible in GenBank under accession number AAD48437.

The term "immunoconjugate" is used herein to define the operative association of an antibody with another effective agent (e.g. a polypeptide of interest).

By "purified" and "isolated" it is meant, when referring to an antibody according to the invention or to a nucleotide sequence, that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the polypeptide; however, the molecule may include some additional bases or moieties which do not deleteriously affect the basic characteristics of the composition.

***Fusion protein of the invention***

A first object of the invention relates to a fusion protein consisting of a constant domain (CL) of a light chain of an immunoglobulin fused at its C-terminal end to a polypeptide (P) characterized by the sequence:

$$LPXT(G)_n \qquad (P)$$

wherein n is an integer number and X represents an amino acid other than tryptophan and cysteine.

In one embodiment, n represents 1, 2, 3, 4, 5 or 6.

In one embodiment, the CL domain is from a lambda (λ) or a kappa (κ) light chain. Preferably, the CL domain is from a kappa (κ) light chain.

In one embodiment, the CL domain is from a mouse or a human light chain.

In one embodiment, the polypeptide (P) comprises a sequence selected in the table A below.

| Amino acid X | n = 1 | n = 2 | n = 3 | n = 4 | n = 5 | n = 6 |
|---|---|---|---|---|---|---|
| A | LPATG SEQ ID NO: 1 | LPATGG SEQ ID NO: 2 | LPATGGG SEQ ID NO: 3 | LPATGGGG SEQ ID NO: 4 | LPATGGGGG SEQ ID NO: 5 | LPATGGGGGG SEQ ID NO: 6 |
| D | LPDTG SEQ ID NO: 7 | LPDTGG SEQ ID NO: 8 | LPDTGGG SEQ ID NO: 9 | LPDTGGGG SEQ ID NO: 10 | LPDTGGGGG SEQ ID NO: 11 | LPDTGGGGGG SEQ ID NO: 12 |
| E | LPETG SEQ ID NO: 13 | LPETGG SEQ ID NO: 14 | LPETGGG SEQ ID NO: 15 | LPETGGGG SEQ ID NO: 16 | LPETGGGGG SEQ ID NO: 17 | LPETGGGGGG SEQ ID NO: 18 |

(continued)

| Amino acid X | n = 1 | n = 2 | n = 3 | n = 4 | n = 5 | n = 6 |
|---|---|---|---|---|---|---|
| F | LPFTG SEQ ID NO: 19 | LPFTGG SEQ ID NO: 20 | LPFTGGG SEQ ID NO: 21 | LPFTGGGG SEQ ID NO: 22 | LPFTGGGGG SEQ ID NO: 23 | LPFTGGGGGG SEQ ID NO: 24 |
| G | LPGTG SEQ ID NO: 25 | LPGTGG SEQ ID NO: 26 | LPGTGGG SEQ ID NO: 27 | LPGTGGGG SEQ ID NO: 28 | LPGTGGGGG SEQ ID NO: 29 | LPGTGGGGGG SEQ ID NO: 30 |
| H | LPHTG SEQ ID NO: 31 | LPHTGG SEQ ID NO: 32 | LPHTGGG SEQ ID NO: 33 | LPHTGGGG SEQ ID NO: 34 | LPHTGGGGG SEQ ID NO: 35 | LPHTGGGGGG SEQ ID NO: 36 |
| I | LPITG SEQ ID NO: 37 | LPITGG SEQ ID NO: 38 | LPITGGG SEQ ID NO: 39 | LPITGGGG SEQ ID NO: 40 | LPITGGGGG SEQ ID NO: 41 | LPITGGGGGG SEQ ID NO: 42 |
| K | LPKTG SEQ ID NO: 43 | LPKTGG SEQ ID NO: 44 | LPKTGGG SEQ ID NO: 45 | LPKTGGGG SEQ ID NO: 46 | LPKTGGGGG SEQ ID NO: 47 | LPKTGGGGGG SEQ ID NO: 48 |
| L | LPLTG | LPLTGG | LPLTGGG | LPLTGGGG | LPLTGGGGG | LPLTGGGGGG |
| | SEQ ID NO: 49 | SEQ ID NO: 50 | SEQ ID NO: 51 | SEQ ID NO: 52 | SEQ ID NO: 53 | SEQ ID NO: 54 |
| M | LPMTG SEQ ID NO: 55 | LPMTGG SEQ ID NO: 56 | LPMTGGG SEQ ID NO: 57 | LPMTGGGG SEQ ID NO: 58 | LPMTGGGGG SEQ ID NO: 59 | LPMTGGGGGG SEQ ID NO: 60 |
| N | LPNTG SEQ ID NO: 61 | LPNTGG SEQ ID NO: 62 | LPNTGGG SEQ ID NO: 63 | LPNTGGGG SEQ ID NO: 64 | LPNTGGGGG SEQ ID NO: 65 | LPNTGGGGGG SEQ ID NO: 66 |
| P | LPPTG SEQ ID NO: 67 | LPPTGG SEQ ID NO: 68 | LPPTGGG SEQ ID NO: 69 | LPPTGGGG SEQ ID NO: 70 | LPPTGGGGG SEQ ID NO: 71 | LPPTGGGGGG SEQ ID NO: 72 |
| Q | LPQTG SEQ ID NO: 73 | LPQTGG SEQ ID NO: 74 | LPQTGGG SEQ ID NO: 75 | LPQTGGGG SEQ ID NO: 76 | LPQTGGGGG SEQ ID NO: 77 | LPQTGGGGGG SEQ ID NO: 78 |
| R | LPRTG SEQ ID NO: 79 | LPRTGG SEQ ID NO: 80 | LPRTGGG SEQ ID NO: 81 | LPRTGGGG SEQ ID NO: 82 | LPRTGGGGG SEQ ID NO: 83 | LPRTGGGGGG SEQ ID NO: 84 |
| S | LPSTG SEQ ID NO: 85 | LPSTGG SEQ ID NO: 86 | LPSTGGG SEQ ID NO: 87 | LPSTGGGG SEQ ID NO: 88 | LPSTGGGGG SEQ ID NO: 89 | LPSTGGGGGG SEQ ID NO: 90 |
| T | LPTTG SEQ ID NO: 91 | LPTTGG SEQ ID NO: 92 | LPATTGGG SEQ ID NO: 93 | LPTTGGGG SEQ ID NO: 94 | LPTTGGGGG SEQ ID NO: 95 | LPTTGGGGGG SEQ ID NO: 96 |
| V | LPVTG SEQ ID NO: 97 | LPVTGG SEQ ID NO: 98 | LPVTGGG SEQ ID NO: 99 | LPVTGGGG SEQ ID NO: 100 | LPVTGGGGG SEQ ID NO: 101 | LPVTGGGGGG SEQ ID NO: 102 |
| Y | LPYTG SEQ ID NO: 103 | LPYTGG SEQ ID NO: 104 | LPYTGGG SEQ ID NO: 105 | LPYTGGGG SEQ ID NO: 106 | LPYTGGGGG SEQ ID NO: 107 | LPYTGGGGGG SEQ ID NO: 108 |

According to the invention, the CL constant domain is fused at its C-terminal end to polypeptide (P) directly or indirectly.

As used herein, the term "directly" means that the last amino acid at the N-terminal end of the constant domain is fused directly to the first amino acid at the C-terminal end of the polypeptide (P). In other words, in this embodiment, the last amino acid of the N-terminal end of CL constant domain is directly linked by a covalent bond to the first amino acid of the C-terminal end of polypeptide (P).

As used herein, the term "indirectly" means that the last amino acid at the N-terminal end of the constant domain is fused to the first amino acid at the C-terminal end of the polypeptide (P) via a linker. As used herein, the term "linker" refers to a sequence of at least one amino acid that links the constant domain with polypeptide (P). Such a linker may be useful to prevent steric hindrances. Typically the linker (L) is characterized by the sequence:

$$(GS)_m \qquad (L)$$

wherein m is integer number which can be 1, 2, 3, 4 or 5.

In one embodiment, the linker may be GS (SEQ ID NO: 109), GSGS (SEQ ID NO: 110), GSGSGS (SEQ ID NO: 111), GSGSGSGS (SEQ ID NO: 112) or GSGSGSGSGS (SEQ ID NO: 113).

In one embodiment, the fusion protein according to the invention comprises at its C-terminal end a second polypeptide ("Tag"). In one embodiment, the polypeptide (Tag) is:

(H)p (Tag)

wherein p is integer number which can be 1 to 10.

In another particular embodiment, the tag is H (SEQ ID NO:114), HH (SEQ ID NO:115), HHH (SEQ ID NO:116), HHHH (SEQ ID NO:117), HHHHH (SEQ ID NO:118), HHHHHH (SEQ ID NO:119), HHHHHHH (SEQ ID NO:120), HHHHHHHH (SEQ ID NO:121), HHHHHHHHH (SEQ ID NO:122) or HHHHHHHHHH (SEQ ID NO:123).

In one embodiment, the fusion protein according to the invention is fused at its N-terminal end to a variable domain (VL) of a light of an immunoglobulin.

In one embodiment, the VL domain is from a humanised or a chimeric (mouse/antibody) chain.

***Nucleic acids, vectors and recombinant host cells of the invention:***

A further object of the present invention relates to a nucleic acid molecule encoding for a fusion protein according to the invention.

In one embodiment, the invention also relates to a nucleic acid molecule which comprises:

a. a cassette encoding for the constant domain of the fusion protein, and,
b. a cassette encoding for the polypeptide (P) of the fusion protein

In another embodiment, the nucleic acid molecule according to the invention comprises a cassette encoding for an antibiotic.

In one embodiment, the antibiotic is the neomycin or the ampicillin. In another particular embodiment, the antibiotic is the neomycin.

In one embodiment, the nucleic acid molecule according to the invention comprises a cassette encoding for a tag.

In one embodiment, the nucleic acid molecule according to the invention comprises a cassette encoding for a variable domain of a light chain of an immunoglobulin.

As used herein, a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

These nucleic acid molecules can be obtained by conventional methods well known to those skilled in the art.

Typically, said nucleic acid is a DNA or RNA molecule, which may be included in a suitable vector, such as a plasmid, cosmid, episome, artificial chromosome, phage or viral vector.

So, a further object of the present invention relates to an expression vector comprising a nucleic acid molecule according to the invention.

In a particular object, the present invention relates to a vector and an expression cassette in which a nucleic acid molecule encoding for a fusion protein of the invention is associated with suitable elements for controlling transcription (in particular promoter, enhancer and, optionally, terminator) and, optionally translation, and also the recombinant vectors into which a nucleic acid molecule in accordance with the invention is inserted. These recombinant vectors may, for example, be cloning vectors, or expression vectors.

As used herein, the terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

Any expression vector for animal cell can be used. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

Other examples of plasmids include replicating plasmids comprising an origin of replication, or integrative plasmids, such as for instance pUC, pcDNA, pBR, and the like.

Other examples of viral vectors include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

The invention also includes gene delivery systems comprising a nucleic acid molecule of the invention, which can be used in gene therapy in vivo or ex vivo. This includes for instance viral transfer vectors such as those derived from retrovirus, adenovirus, adeno associated virus, lentivirus, which are conventionally used in gene therapy. This also includes gene delivery systems comprising a nucleic acid molecule of the invention and a non-viral gene delivery vehicle. Examples of non viral gene delivery vehicles include liposomes and polymers such as polyethylenimines, cyclodextrins, histidine/lysine (HK) polymers, etc.

A subject of the present invention is also a prokaryotic or eukaryotic host cell genetically transformed with at least one nucleic acid molecule or expression vector according to the invention.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA has been "transformed".

In one embodiment, for expressing and producing a fusion protein of the invention, prokaryotic cells, in particular E. coli cells, will be chosen. Actually, according to the invention, it is not mandatory to produce the fusion protein of the invention in a eukaryotic context that will favour post-translational modifications (e;.g. glycosylation). Furthermore, prokaryotic cells have the advantages to produce protein in large amounts. If a eukaryotic context is needed, yeasts (e.g. saccharomyces strains) may be particularly suitable since they allow production of large amounts of proteins. Otherwise, typical eukaryotic cell lines such as CHO, BHK-21, COS-7, C127, PER.C6, YB2/0 or HEK293 could be used, for their ability to process to the right post-translational modifications of the immunoconjugate of the invention.

In one embodiment, the host cell is a pluripotent stem cell, in particular an embryonic stem cell (ES) or an induced pluripotent stem cell (IPS).

Accordingly, a further aspect of the invention relates to a host cell comprising a nucleic acid molecule encoding for fusion protein according to the invention or an expression vector according to the invention.

The construction of expression vectors in accordance with the invention, and the transformation of the host cells can be carried out using conventional molecular biology techniques. The fusion protein of the invention, can, for example, be obtained by culturing genetically transformed cells in accordance with the invention and recovering the fusion protein expressed by said cell, from the culture. They may then, if necessary, be purified by conventional procedures, known in themselves to those skilled in the art, for example by fractional precipitation, in particular ammonium sulfate precipitation, electrophoresis, gel filtration, affinity chromatography, etc.... In particular, conventional methods for preparing and purifying recombinant proteins may be used for producing the proteins in accordance with the invention.

A further aspect of the invention relates to a method for producing a fusion protein of the invention comprising the step consisting of: (i) culturing a transformed host cell according to the invention under conditions suitable to allow expression of said fusion protein; and (ii) recovering the expressed fusion protein.

***Antibodies of the invention***

A further object of the invention relates to an antibody wherein the constant domain of the light chain is replaced by a fusion protein according to the invention.

In one embodiment, the antibody is a monoclonal antibody.

In one embodiment the antibody is selected from the group consisting of human antibodies, humanized antibodies or chimeric antibodies.

A further object of the invention related to a fragment of an antibody according to the invention whis selected from the group consisting of Fab fragments, F(ab')2 fragments, or Fab' fragments.

The antibody according to the invention or the fragment thereof is directed against any antigen of interest.

In one embodiment, the antibody or the fragment thereof may be specific for an immune cell regulatory molecule such as CD3, CD4, CD8, CD25, CD28, CD26, CTLA-4, ICOS, or CD11a. Other suitable antigens include but are not limited to those associated with immune cells including T cell-associated molecules, such as TCR/CD3 or CD2; NK cell-associated targets such as NKG2D, Fc$\gamma$RIIIa (CD16), CD38, CD44, CD56, or CD69; granulocyte-associated targets such as Fc$\gamma$RI (CD64), Fc$\alpha$RI (CD89), and CR3 (CD11b/CD18); monocyte/macrophage-associated targets (such as Fc$\gamma$RI (CD64), Fc$\alpha$RI (CD89), CD3 (CD11b/CD18), or mannose receptor; dendritic cell-associated targets such as Fc$\gamma$RI (CD64) or mannose receptor; and erythrocyte-associated targets such as CRI (CD35). More particularly, the antibody of the invention or the fragment thereof is directed against a dendritic cell surface marker selected CD11c DEC-205, DC-SIGN (CD209) or Dectin-1, XCR1, Langerin (CD207), Clec9A, CD68 and CD36.

In one embodiment the antibody of the invention or the fragment thereof may be directed against a cancer antigen. Known cancer antigens include, without limitation, c-erbB-2 (erbB-2 is also known as c-neu or HER-2), which is particularly associated with breast, ovarian, and colon tumor cells, as well as neuroblastoma, lung cancer, thyroid cancer, pancreatic cancer, prostate cancer, renal cancer and cancers of the digestive tract. Another class of cancer antigens is oncofetal proteins of nonenzymatic function. These antigens are found in a variety of neoplasms, and are often referred to as "tumor-associated antigens." Carcinoembryonic antigen (CEA), and a-fetoprotein (AFP) are two examples of such cancer antigens. AFP levels rise in patients with hepatocellular carcinoma: 69% of patients with liver cancer express high levels of AFP in their serum. CEA is a serum glycoprotein of 200 kDa found in adenocarcinoma of colon, as well as cancers of the lung and genitourinary tract. Yet another class of cancer antigens is those antigens unique to a particular tumor, referred to sometimes as "tumor specific antigens," such as heat shock proteins (e.g., hsp70 or hsp90 proteins) from a particular type of tumor. Other targets include the MICA/B ligands of NKG2D. These molecules are expressed on many types of tumors, but not normally on healthy cells. Additional specific examples of cancer antigens include epithelial cell adhesion molecule (Ep-CAM/TACSTD1), mesothelin, tumor-associated glycoprotein 72 (TAG-72), gp100, Melan-A, MART-1, KDR, RCAS1, MDA7, cancer-associated viral vaccines (e.g., human papillomavirus antigens), prostate specific antigen (PSA, PSMA), RAGE (renal antigen), CAMEL (CTL-recognized antigen on melanoma), CT antigens (such as MAGE-B5, -B6, -C2, -C3, and D; Mage-12; CT10; NY-ESO-1, SSX-2, GAGE, BAGE, MAGE, and SAGE), mucin antigens (e.g., MUC1, mucin-CA125, etc.), cancer-associated ganglioside antigens, tyrosinase, gp75, C-myc, Mart1, MelanA, MUM-1, MUM-2, MUM-3, HLA-B7, Ep-CAM, tumor-derived heat shock proteins, and the like (see also, e.g., Acres et al., Curr Opin Mol Ther 2004 February, 6:40-7; Taylor-Papadimitriou et al., Biochim Biophys Acta. 1999 Oct. 8; 1455(2-3):301-13; Emens et al., Cancer Biol Ther. 2003 July-August; 2(4 Suppl 1):S161-8; and Ohshima et al., Int J Cancer. 2001 Jul. 1; 93(1):91-6). Other exemplary cancer antigen targets include CA 195 tumor-associated antigen-like antigen (see, e.g., U.S. Pat. No. 5,324,822) and female urine squamous cell carcinoma-like antigens (see, e.g., U.S. Pat. No. 5,306,811), and the breast cell cancer antigens described in U.S. Pat. No. 4,960,716. The antibody of the invention or the fragment thereof may target protein antigens, carbohydrate antigens, or glycosylated proteins. For example, the variable domain can target glycosylation groups of antigens that are preferentially produced by transformed (neoplastic or cancerous) cells, infected cells, and the like (cells associated with other immune system-related disorders). In one aspect, the antigen is a tumor-associated antigen. In an exemplary aspect, the antigen is O-acetylated-GD2 or glypican-3. In another particular aspect, the antigen is one of the Thomsen-Friedenreich (TF) antigens (TFAs). The antibody of the invention or the fragment thereof can also exhibit specificity for a cancer-associated protein. Such proteins can include any protein associated with cancer progression. Examples of such proteins include angiogenesis factors associated with tumor growth, such as vascular endothelial growth factors (VEGFs), fibroblast growth factors (FGFs), tissue factor (TF), epidermal growth factors (EGFs), and receptors thereof; factors associated with tumor invasiveness; and other receptors associated with cancer progression (e.g., one of the HER1-HER4 receptors).

In one embodiment, the antibody of the invention or the fragment thereof can be specific for a virus, a bacteria or parasite associated target. For example, the variable domain may be specific for a virus-associated target such as an HIV protein (e.g., gp120 or gp41), CMV or other viruses, such as hepatitis C virus (HCV). In another particular embodiment, the antibody of the invention or the fragment thereof may be specific for antigen derived from bacteria, rickettsiae, fungi, viruses, parasites, including Diphtheria, Pertussis, Tetanus, H. influenzae, S. pneumoniae, E. Coli, Klebsiella, S. aureus, S. epidermidis, N. meningiditis, Polio, Mumps, measles, rubella, Respiratory Syncytial Virus, Rabies, Ebola, Anthrax, Listeria, Hepatitis A, B, C, Human Immunodeficiency Virus I and 11, Herpes simplex types 1 and 2, CMV, EBV, Varicella Zoster, Malaria, Tuberculosis, Candida albicans, and other candida, Pneumocystis caringi, Mycoplasma, Influenzae virus A and B, Adenovirus, Group A streptococcus, Group B streptococcus, Pseudomonas aeryinosa, Rhinovirus, Leishmania, Parainfluenzae, types 1, 2 and 3, Coronaviruses, Salmonella, Shigella, Rotavirus, Toxoplasma, Enterovirusses, and Chlamydia trachomatis and pneumoniae.

In one embodiment, the antibody of the invention or the fragment thereof may target albumin.

In one embodiment the antibody of the invention or the fragment thereof may alternatively target a hapten, and more particularly low molecular weight hapten, and more preferably a radiolabeled low molecular weight hapten. Molecular weight haptens according to the invention may be selected from the group consisting of methotrexate, histamine succinyl glycine, DTPA (diethylene triaminepentaacetic acid); DOTA (1,4,7,10-tetraazacyclododecane-N,N',N",N'"-tetraacetic acid); and derivatives thereof (see, for example, U.S. Pat. Nos. 4,885,363; 5,087,440; 5,155,215; 5,188,816; 5,219,553; 5,262,532; and 5,358,704; and D. Meyer et al., Invest. Radiol. 1990, 25: S53-55). In particular embodiment, the hapten is labeled with a radionuclide. For example, useful diagnostic radionuclides include, but are not limited to, $^{110}$In, $^{177}$Lu, $^{18}$F, $^{52}$Fe, $^{62}$Cu, $^{64}$Cu, $^{67}$Cu, $^{67}$Ga, $^{68}$Ga, $^{89}$Zr, $^{94}$Tc, $^{150}$Re, $^{188}$Re, or other gamma-, beta-, or positron-emitters. Particularly useful therapeutic radionuclides include, but are not limited to $^{111}$In, $^{177}$Lu, $^{212}$Bi, $^{213}$Bi, $^{211}$At, $^{62}$Cu, $^{64}$Cu, $^{67}$Cu, $^{90}$Y, $^{125}$I, $^{131}$I, $^{32}$P, $^{33}$P, $^{47}$Sc, $^{111}$Ag, $^{67}$Ga, $^{142}$Pr, $^{153}$Sm, $^{161}$Tb, $^{166}$Dy, $^{166}$Ho, $^{186}$Re, $^{188}$Re, $^{189}$Re, $^{212}$Pb, $^{223}$Ra, $^{225}$Ac, $^{59}$Fe, $^{75}$Se, $^{77}$As, $^{89}$Sr, $^{99}$Mo, $^{105}$Rh, $^{109}$Pd, $^{143}$Pr, $^{149}$Pm, $^{169}$Er, $^{194}$Ir, $^{198}$Au, $^{199}$Au, and $^{211}$Pb.

Amino acid sequence modification(s) of the antibodies described herein are contemplated. For example, it may be desirable to improve the binding affinity and/or other biological properties of the antibody. It is known that when a humanized antibody is produced by simply grafting only CDRs in VH and VL of an antibody derived from a non-human animal in FRs of the VH and VL of a human antibody, the antigen binding activity is reduced in comparison with that of the original antibody derived from a non-human animal. It is considered that several amino acid residues of the VH and VL of the non-human antibody, not only in CDRs but also in FRs, are directly or indirectly associated with the antigen binding activity. Hence, substitution of these amino acid residues with different amino acid residues derived from FRs of the VH and VL of the human antibody would reduce of the binding activity. In order to resolve the problem, in antibodies grafted with human CDR, attempts have to be made to identify, among amino acid sequences of the FR of the VH and VL of human antibodies, an amino acid residue which is directly associated with binding to the antibody, or which interacts with an amino acid residue of CDR, or which maintains the three-dimensional structure of the antibody and which is directly associated with binding to the antigen. The reduced antigen binding activity could be increased by replacing the identified amino acids with amino acid residues of the original antibody derived from a non-human animal.

Modifications and changes may be made in the structure of the antibodies of the present invention, and in the DNA sequences encoding them, and still obtain a functional molecule that encodes an antibody with desirable characteristics.

In making the changes in the amino sequences, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

A further object of the present invention also encompasses function-conservative variants of the antibodies of the present invention.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared.

Two amino acid sequences are "substantially homologous" or "substantially similar" when greater than 80 %, preferably greater than 85 %, preferably greater than 90 % of the amino acids are identical, or greater than about 90 %, preferably greater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

For example, certain amino acids may be substituted by other amino acids in a protein structure without appreciable loss of activity. Since the interactive capacity and nature of a protein define the protein's biological functional activity, certain amino acid substitutions can be made in a protein sequence, and, of course, in its DNA encoding sequence, while nevertheless obtaining a protein with like properties. It is thus contemplated that various changes may be made in the antibodies sequences of the invention, or corresponding DNA sequences which encode said antibodies, without appreciable loss of their biological activity.

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

Engineered antibodies of the invention include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention. Another type of framework modification involves mutating one or more residues within the framework region, or even within one or more CDR regions, to remove T cell-epitopes to thereby reduce the potential immunogenicity of the antibody. This approach is also referred to as "deimmunization" and is described in further detail in U.S. Patent Publication No. 20030153043 by Carr et al.

In addition or alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the invention may be chemically modified (e.g., one or more chemical moieties can be attached to the antibody) or be modified to alter its glycosylation, again to alter one or more functional properties of the antibody. Each of these embodiments is described in further detail below. The numbering of residues in the Fc region is that of the EU index of Kabat.

In one embodiment, the hinge region of CH1 is modified such that the number of cysteine residues in the hinge region is altered, e.g., increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In another embodiment, the antibody is modified to increase its biological half-life. Various approaches are possible. For example, one or more of the following mutations can be introduced: T252L, T254S, T256F, as described in U.S. Patent No. 6,277,375 by Ward. Alternatively, to increase the biological half life, the antibody can be altered within the CH1 or CL region to contain a salvage receptor binding epitope taken from two loops of a CH2 domain of an Fc region of an IgG, as described in U.S. Patent Nos. 5,869,046 and 6,121 ,022 by Presta et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Patent Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in further detail in U.S. Patent Nos. 6,194,551 by Idusogie et al.

In another embodiment, one or more amino acid residues are altered to thereby alter the ability of the antibody to fix complement. This approach is described further in PCT Publication WO 94/29351 by Bodmer et al.

In yet another embodiment, the Fc region is modified to increase the ability of the antibody to mediate antibody dependent cellular cytotoxicity (ADCC) and/or to increase the affinity of the antibody for an Fc receptor by modifying one or more amino acids. This approach is described further in PCT Publication WO 00/42072 by Presta. Moreover, the binding sites on human IgGI for FcγRI, FcγRII, FcγRIII and FcRn have been mapped and variants with improved binding have been described (see Shields, R. L. et al., 2001 J. Biol. Chen. 276:6591-6604, W02010106180).

In still another embodiment, the glycosylation of an antibody is modified. For example, an aglycoslated antibody can be made (i.e., the antibody lacks glycosylation). Glycosylation can be altered to, for example, increase the affinity of the antibody for the antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861 by Co et al.

Additionally or alternatively, an antibody can be made that has an altered type of glycosylation, such as a hypofucosylated or non-fucosylated antibody having reduced amounts of or no fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, EP 1 ,176,195 by Hang et al. describes a cell line with a functionally disrupted FUT8 gene, which encodes a fucosyl transferase, such that antibodies expressed in such a cell line exhibit hypofucosylation or are devoid of fucosyl residues. Therefore, in one embodiment, the antibodies of the invention may be produced by recombinant expression in a cell line which exhibit hypofucosylation or non-fucosylation pattern, for example, a mammalian cell line with deficient expression of the FUT8 gene encoding fucosyltransferase. PCT Publication WO 03/035835 by Presta describes a variant CHO cell line, Lecl3 cells, with reduced ability to attach fucose to Asn(297)-linked carbohydrates, also resulting in hypofucosylation of antibodies expressed in that host cell (see also Shields, R.L. et al., 2002 J. Biol. Chem. 277:26733-26740). PCT Publication WO 99/54342 by Umana et al. describes cell lines engineered to express glycoprotein-modifying glycosyl transferases (e.g., beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., 1999 Nat. Biotech. 17:176-180). Eureka Therapeutics further describes genetically engineered CHO mammalian cells capable of producing antibodies with altered mammalian glycosylation pattern devoid of fucosyl residues (http://www.eurekainc.com/a&boutus/companyoverview.html). Alternatively, the antibodies of the invention can be produced in yeasts or filamentous fungi engineered for mammalian-like glycosylation pattern and capable of producing antibodies lacking fucose as glycosylation pattern (see for example EP1297172B1 ).

Another modification of the antibodies herein that is contemplated by the invention is pegylation. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1- C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the invention. See for example, EP O 154 316 by Nishimura et al. and EP 0 401 384 by Ishikawa et al.

***Transgenic non-human mammal of the invention***

A further object of the invention relates to a transgenic non-human mammal genetically modified to express an antibody according to the invention.

In one embodiment, the transgenic non-human mammal genetically modified expresses a human antibody wherein its light chain is replaced by a fusion protein according to the invention.

In one embodiment, said mammal is a rodent, for example a rat or a mouse.

In one specific embodiment, the invention relates to a method for producing a transgenic non-human mammal genetically modified, comprising the steps of:

a) providing a targeting vector comprising a nucleic acid molecule according to the invention;
b) introducing said expression vector into embryonic stem cells (ESC) obtained from said non-human mammal;
c) selecting ESC clones positive for homologous recombination of the targeting vector at the mammal light chain locus;
d) injecting ESC into blastocysts of corresponding non-human mammal to obtain chimeras;
e) growing resulting chimeras for germline transmission; and,
f) obtaining homozygous non-human mammal expressing an from crossing heterozygous animals.

Any ES cell can be used in accordance with the present invention. It is, however, preferred to use primary isolates of ES cells. Such isolates can be obtained directly from embryos, such as the CCE cell line, or from the clonal isolation ofES cells from the CCE cell line. Such clonal isolation can be accomplished according to the method of E. J. Robertson (In: Teratocarcinomas and Embryonic Stem Cells: A Practical Approach, (E. J. Robertson, Ed.), IRL Press, Oxford, 1987), which reference and method are incorporated herein by reference. The purpose of such clonal propagation is to obtain ES cells, which have a greater efficiency for differentiating into an animal. Clonally selected ES cells are approximately 10-fold more effective in producing transgenic animals than the progenitor cell line CCE. For the purposes of the recombination methods of the present invention, clonal selection provides no advantage.

An example of ES cell lines, which have been clonally derived from embryos, are the ES cell lines, AB1 (hprt.sup.+) or AB2.1 (hprt.sup.-). The ES cells are preferably cultured on stromal cells (such as STO cells (especially SNC4 STO cells) and/or primary embryonic fibroblast cells). Methods for the production and analysis of chimeric mice are well known by the skilled in the art. The stromal (and/or fibroblast) cells serve to eliminate the clonal overgrowth of abnormal ES cells. Most preferably, the cells are cultured in the presence of leukocyte inhibitory factor ("Lif"). Since the gene encoding Lif has been cloned, it is especially preferred to transform stromal cells with this gene, by means known in the art, and to then culture the ES cells on transformed stromal cells that secrete Lif into the culture medium.

The targeting vector should be designed for obtaining homologous recombination of the human light chain of an immunoglobulin coding sequence at the light chain locus of the corresponding animal. A preferred example of a targeting vector for a mouse is given in the example 1. More specifically, the targeting vector may comprise the following features:

1) a nucleic acid encoding for a fusion protein according to the invention, which includes:

   i. human light chain of an immunoglobulin;
   ii. sequence for initiation (promoter) and/or termination (terminator) of transcription (respectively downstream and upstream of human light chain of an immunoglobulin coding sequence), such as SV40 polyA as terminator;
   iii. optionally, selection marker cassette for selecting ESC clones for double homologous recombination event, such as Neomycin resistance cassette, said selection marker sequence being preferably flanked by LoxP sequence,

2) 5' downstream and 3'upstream flanking regions of a sequence coding for a peptidic sequence recognized by the sortase.

In one specific embodiment of the method, said non-human mammal is a mouse and said targeting vector comprises the coding sequence of human light chain of an immunoglobulin gene instead of the mouse light chain. In fact, the fully rearranged Kappa Light chain gene is made of a 3 exons : a Leader exon (that includes the ATG), a V exon that results from the rearrangement of a VL and a JL gene segments and of a C exon (terminated by a STOP codon). The vector shown in Figure 1 allowed us to substitute the CKappa exon of mouse origin by a CKappa exon of human origin. The replacement was extremely precise and started with the sequence coding for the first amino acid of the CK exon and ended up with the last amino acid of the CK exon.

Once the targeting vector has been constructed, it may be amplified and it is then introduced, for example by electroporation, into non-human mammalian embryonic stem (ES) cells, for example mouse or rat embryonic stem cells.

The selection step may include selecting clones resistant to the selection marker (if included in the targeting vector) and screen for clones having the proper insert in their genome, for example, using PCR amplification with primers upstream and downstream of the insert. A DNA fragment, an RNA fragment or synthetic oligodesoxynucleotide, or other nucleic acids may further be used as a probe.

As selective markers, positive selective markers such as neomycin resistant gene and hygromycin-resistant gene may be used. Negative selection markers include thymidine kinase gene or herpes simplex virus and fragment A of diphtheria toxin are known. Any of markers used for cell culture may be used in ES cells. Where a positive marker is used, it is necessary to insert the expression unit in an intron in the animal chromosomal DNA fragment of the targeting vector.

The selected clones are then reintroduced into a blastocyst and placed into a surrogate mother for gestation to generate the genetically modified animal, using standard blastocyst injection technology. The resulting chimera is then used for germline transmission of the genome with human light chain of an immunoglobulin.

Optionally, if a selection marker is used, the resistance cassette encoding such selection marker is flanked by LoxP sequences. Accordingly, the resistance cassette can be removed by crossing the chimeras with Cre recombinase expressing animals. Corresponding heterozygous knock-in animals are then intercrossed for obtaining homozygous animal lines which express the fusion protein according to the invention.

The invention thus also relates to the non-human mammal expressing the fusion protein according to the invention obtainable by a method as described above.

These animals according to the invention are useful for producing human antibody which express instead of the light chain of the immunoglobulin, the fusion protein according to the invention.

***Methods for producing antibodies of the invention***

The transgenic non-human mammal according to the invention is particularly suitable for producing the antibodies of the invention. Typically, the methods for producing the antibodies of the invention combine use of at least one transgenic non human mammal according to the invention and conventional methods for the production of monoclonal antibodies.

For example, aantibodies are prepared according to conventional methodology. Monoclonal antibodies may be generated using the method of Kohler and Milstein (Nature, 256:495, 1975). To prepare monoclonal antibodies useful in the invention, a transgenic non-human mammal according to the invention is immunized at suitable intervals (e.g., twice-weekly, weekly, twice-monthly or monthly) with an antigen of interest (e.g. a cancer antigen). The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The transgenic non-human mammal according to the invention may be immunized by subcutaneous, intraperitoneal, intramuscular, intravenous, intranasal or other routes. A transgenic non-human mammal according to the invention may be immunized with multiple forms of the antigen by multiple routes. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

***<u>Immunoconjugates of the invention:</u>***

The antibody according to the invention may be easily conjugated to a polypeptide of interest (Pi) by Sortase-Mediated Ligation (Tsukiji and Nagamune, 2009).

A further object of the invention relates to a method for preparing an immunoconjugate comprising i) contacting an antibody of the invention or a fragment thereof with a polypeptide of interest (Pi) optionally fused at its C (or N)-terminal end to a polypeptide (Pg) having the sequence $(G)_o$ wherein o is integer number which can be 1, 2, 3, 4, 5 or 6 in presence of an amount of Sortase, A and ii) recovering the immunoconjuate as obtained in step i). In one embodiment, the polypeptide of interest is at the Cter of the Light or Heavy chain.

In one embodiment, the contacting step may be performed in any recipient wherein the antibody or the fragment thereof is mixed with the polypeptide of interest (Pi) and with the amount of sortase A. The ligation may be performed in any convenient recipient (e.g., tubes such as microfuge tubes, flask, dish), microtiter plates (e.g., 96-well or 384-well plates), glass slides, silicon chips, filters, or any solid or semisolid support. Typically, the reaction mixture comprises an aqueous environment. Water with an appropriate buffer and/or salt content often may be utilized. The reaction mixture is maintained at any convenient temperature at which the ligation reaction can be performed. In some embodiments, the ligation is performed at a temperature ranging from about 15° C. to about 50° C. In certain embodiments, the temperature is room temperature (e.g., about 25° C.). Suitable ligation conditions comprise pH in the range of 6 to 8.5. In some embodiments, the antibody and the polypeptide (Pi) are present at a concentration ranging from about 10 pM to about 10 mM and the sortase A is at a concentration ranging from about 1 pM to about 500 pM.

Any polypeptide of interest (Pi) may be used in the ligation method as above described, provided that said polypeptide (Pi) comprises a $NH_2$-$CH_2$ moiety. In one embodiment, the polypeptide of interest (Pi) is fused to a polypeptide (Pg) having the sequence $(G)_o$ wherein o is integer number which can be 1, 2, 3, 4, 5 or 6.

The polypeptide (Pi) is isolated when utilized in a cell-free system. The polypeptide (Pi) may consist in a domain of a protein, such as in the N-terminus, C-terminus, extracellular region, intracellular region, transmembrane region, active site (e.g., nucleotide binding region or a substrate binding region), a domain (e.g., an SH2 or SH3 domain) or a post-translationally modified region (e.g., phosphorylated, glycosylated or ubiquinated region), for example. Typically, polypeptide (Pi) is 50 amino acids or fewer in length (e.g., 45, 40, 35, 30, 25, 20, or 15 amino acids or fewer in length) and proteins sometimes are 100 or fewer amino acids in length, or 200, 300, 400, 500, 600, 700, or 900 or fewer amino acids in length. In certain embodiments, the protein is a signal transduction factor, cell proliferation factor, apoptosis factor, angiogenesis factor, or cell interaction factor. Examples of cell interaction factors include but are not limited to cadherins (e.g., cadherins E, N, BR, P, R, and M; desmocollins; desmogleins; and protocadherins); connexins; integrins; proteoglycans ; immunoglobulins (e.g., ALCAM, NCAM-1 (CD56), CD44, intercellular adhesion molecules (e.g., ICAM-1 and ICAM-2), LFA-1, LFA-2, LFA-3, LECAM-1, VLA-4, ELAM and N-<BR>CAM); selectins (e.g., L-selectin (CD62L), E-selectin (CD62e), and P-selectin (CD62P)) ; agrin; CD34; and a cell surface protein that is cyclically internalized or internalized in response to ligand binding.

In one embodiment, the polypeptide of interest (Pi) is a reporter protein such as GFP (Green Fluorescent Protein), RFP (Red Fluorescent Protein), YFP (yellow Fluorescent Protein), BFP (Blue Fluorescent Protein).

In one embodiment, the polypeptide of interest is avidin, streptavidin, or biotin.

In one embodiment, the polypeptide of interest (Pi) is a serum protein, such as human serum albumin to increase half-life of the resulting molecule

In one embodiment, the polypeptide of interest (Pi) is an enzyme. Examples of enzymes that can be used to detectably label immunoconjugates include ß-galactosidase, glucose oxidase, peroxidase and alkaline phosphatase.

In one embodiment, the polypeptide of interest (Pi) is a toxin (e.g., a cytostatic or cytocidal agent such as, for example, abrin, ricin A, pseudomonas exotoxin, ribotoxin or diphtheria toxin).

In one embodiment, the polypeptide of interest (Pi) is a pro-drug converting enzyme. Exemplary pro-drug converting enzymes are carboxypeptidase G2, β-glucuronidase, penicillin-V-amidase, penicillin-G-amidase, ß-lactamase, β-glucosidase, nitroreductase and carboxypeptidase A.

In one embodiment, the polypeptide of interest (Pi) is an antigenic or a potentially antigenic polypeptide. This includes polypeptides against which it is desirable to obtain an antibody response or a T cell response, for instance antigenic polypeptides from pathogenic organisms, such as virus, bacteria, fungi, or protozoa, or from cancer antigens (i. e. antigens specifically associated with cancer cells); this includes also polypeptides containing sequences that one wishes to test for their ability to induce an antibody response or T cell reactivity, for instance candidate self-antigens, as well as polypeptides recognized by autoreactive lymphocytes in the context of autoimmune diseases such as type 1 diabetes so as to induce immune tolerance to the autoantigen, or by inducing regulatory T cells suppressing the autoimmune response.

A further object relates to immunoconjugate obtainable by the method as above described.

*Therapeutic uses:*

Immunoconjugates of the invention may be particularly suitable for the treatment of diseases such as cancers or infectious diseases.

Therefore, a further object of the invention relates to an immunoconjugate of the invention for use as a medicament.

More particularly, an aspect of the invention relates to an immunoconjugate of the invention for use in the treatment of any disease that can be treated with the mean of an antibody. In particular embodiment, the disease is selected from the group consisting of cancer, infectious diseases, autoimmune diseases, inflammatory diseases and cardiovascular diseases.

In the context of the invention, the term "treating" or "treatment", as used herein, means reversing, alleviating, inhibiting the progress of, or preventing the disorder or condition to which such term applies, or one or more symptoms of such disorder or condition.

As used herein, the term "infectious disease" is intended to encompass any disease which results from an infection mediated by a virus, a bacteria or a parasite. Therefore the term includes but is not limited to infection with virus such as human immunodeficiency virus, Hepatitis B virus, hepatitis C virus, with parasites such as Plasmodium Falciparum (causative agent for Malaria), or with bacteria such as mycobacterium tuberculosis.

As used herein, the term "cancer" is intended to encompass primary and metastatic solid tumors, including bile duct cancer (e.g. periphilar cancer, distal bile duct cancer, intrahepatic bile duct cancer), bladder cancer, bone cancer (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating, lobular carcinoma, lobular carcinoma in, situ, gynecomastia), Castleman disease (e.g. giant lymph node hyperplasia, angiofollicular lymph node hyperplasia), cervical cancer, colorectal cancer, endometrial cancer (e.g. endometrial adenocarcinoma, adenocanthoma, papillary serous adnocarcinroma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin’s disease, non-Hodgkin’s lymphoma, Kaposi’s sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma, thyroid lymphoma), vaginal cancer, vulvar cancer, and uterine cancer (e.g. uterine leiomyosarcoma). In one embodiment, the cancer is a colorectal cancer.. The term cancer also relates to tumors arising from hematopoietic malignancies such as leukemias as well both Hodgkin's and non-Hodgkin's lymphomas.

As used herein, the term "autoimmune disease" means a disease resulting from an immune response against a self tissue or tissue component, including both self antibody responses and cell-mediated responses. The term autoimmune disease, as used herein, encompasses organ-specific autoimmune diseases, in which an autoimmune response is directed against a single tissue, such as type I diabetes mellitus (T1D), Crohn's disease, ulcerative colitis, myasthenia gravis, vitiligo, Graves' disease, Hashimoto's disease, Addison's disease and autoimmune gastritis and autoimmune hepatitis. The term autoimmune disease also encompasses non-organ specific autoimmune diseases, in which an autoimmune response is directed against a component present in several or many organs throughout the body. Such autoimmune diseases include, for example, rheumatoid disease, systemic lupus erythematosus, progressive systemic sclerosis and variants, polymyositis and dermatomyositis. Additional autoimmune diseases include pernicious anemia including some of autoimmune gastritis, primary biliary cirrhosis, autoimmune thrombocytopenia, Sjogren's syndrome, multiple sclerosis and psoriasis. One skilled in the art understands that the methods of the invention can be applied to these or other autoimmune diseases, as desired.

As used herein, an "inflammatory disease" refers to a disease caused by or resulting from or resulting in inflammation. The term "inflammatory disease" may also refer to a dysregulated inflammatory reaction that causes an exaggerated response by macrophages, granulocytes, and/or T-lymphocytes leading to abnormal tissue damage and cell death. An "inflammatory disease" can be either an acute or chronic inflammatory condition and can result from infections or non-infectious causes. Inflammatory diseases include, without limitation, atherosclerosis, arteriosclerosis, autoimmune disorders, multiple sclerosis, systemic lupus erythematosus, polymyalgia rheumatica (PMR), gouty arthritis, degenerative arthritis, tendonitis, bursitis, psoriasis, cystic fibrosis, arthrosteitis, rheumatoid arthritis, inflammatory arthritis, Sjogren's Syndrome, giant cell arteritis, progressive systemic sclerosis (scleroderma), ankylosing spondylitis, polymyositis, dermatomyosifis, pemphigus, pemphigoid, diabetes (e.g., Type I), myasthenia gravis, Hashimoto's thyroditis, Graves' disease, Goodpasture's disease, mixed connective tissue disease, sclerosing cholangitis, inflammatory bowel disease, Crohn's Disease, ulcerative colitis, pernicious anemia, inflammatory dermatoses, usual interstitial pneumonitis (UIP), asbestosis, silicosis, bronchiectasis, berylliosis, talcosis, pneumoconiosis, sarcoidosis, desquamative interstitial pneumonia, lymphoid interstitial pneumonia, giant cell interstitial pneumonia, cellular interstitial pneumonia, extrinsic allergic alveolitis, Wegener's granulomatosis and related forms of angiitis (temporal arteritis and polyarteritis nodosa), inflammatory dermatoses, hepatitis, delayed-type hypersensitivity reactions (e.g., poison ivy dermatitis), pneumonia, respiratory tract inflammation, Adult Respiratory Distress Syndrome (ARDS), encephalitis, immediate hypersensitivity reactions, asthma, hayfever, allergies, acute anaphylaxis, rheumatic fever, glomerulonephritis, pyelonephritis, cellulitis, cystitis, chronic cholecystitis, ischemia (ischemic injury), allograft rejection, host-versus-graft rejection, appendicitis, arteritis, blepharitis, bronchiolitis, bronchitis, cervicitis, cholangitis, chorioamnionitis, conjunctivitis, dacryoadenitis, dermatomyositis, endocarditis, endometritis, enteritis, enterocolitis, epicondylitis, epididymitis, fasciitis, fibrositis, gastritis, gastroenteritis, gingivitis, ileitis, iritis, laryngitis, myelitis, myocarditis, nephritis, omphalitis, oophoritis, orchitis, osteitis, otitis, pancreatitis, parotitis, pericarditis, pharyngitis, pleuritis, phlebitis, pneumonitis, proctitis, prostatitis, rhinitis, salpingitis, sinusitis, stomatitis, synovitis, testitis, tonsillitis, urethritis, urocystitis, uveitis, vaginitis, vasculitis, vulvitis, and vulvovaginitis, angitis, chronic bronchitis, osteomylitis, optic neuritis, temporal arteritis, transverse myelitis, necrotizing fascilitis, and necrotizing enterocolitis. In one embodiment, the inflammatory disease is selected from the group consisting of arthritis, atherosclerosis, arteriosclerosis, ischemic injury, giant cell arteritis, inflammatory bowel disease, allergy, asthma, diabetes, lupus, multiple sclerosis, cystic fibrosis, hepatitis and psoriasis. In yet another embodiment, the inflammatory disease is selected from the group consisting of lupus, arthritis, and atherosclerosis.

The term "cardiovascular disease" or the like term refers to diseases related to the heart and the blood vessels or the circulation, such as atherosclerosis, ischemic heart disease to or cerebrovascular disease such as coronary artery disease including angina pectoris and myocardial infarction, stroke, vascular heart disease and peripheral vascular disorders such as peripheral arterial disease and occlusive arterial diseases. Cardiovascular disease can be any disorder that affects the heart or the vasculature. CVD, as the term is used herein, can refer to any disease that affects the cardiovascular system, in particular, CVD conditions include, but are not limited to: coronary (ischemic) heart disease; angina pectoris; arrhythmia; cardiac fibrosis; congenital cardiovascular disease; coronary artery disease; peripheral vascular disease; dilated cardiomyopathy; heart attack (myocardial infarction); cerebrovascular disease (stroke); atherosclerosis; heart failure; hypertrophic cardiomyopathy; systemic hypertension from any cause; edematous disorders caused by liver or renal disease; mitral regurgitation; myocardial tumors; myocarditis; rheumatic fever; Kawasaki disease; Takaysu arteritis; cor pulmonale; primary pulmonary hypertension; amyloidosis; hemachromatosis; toxic effects on the heart due to poisoning; Chaga's disease; heart transplantation; cardiac rejection after heart transplantation; cardiomyopathy of chachexia; arrhythmogenic right ventricular dysplasia; cardiomyopathy of pregnancy; Marfan Syndrome; Turner Syndrome; Loeys-Dietz Syndrome; familial biscuspid aortic valve or any inherited disorder of the heart or vasculature, or combinations thereof. These and other CVD conditions have related causes, mechanisms, and treatments. In practice, CVD can be treated by cardiologists, thoracic surgeons, vascular surgeons, neurologists, and interventional radiologists, depending on the organ system that is being treated. There is considerable overlap in the specialties, and it is common for certain procedures to be performed by different types of specialists in the same hospital.

In one embodiment, an immunoconjugate of the invention is particularly suitable for the treatment of cancer. Typically, the immunoconjugate may consist of an antibody specific for a cancer antigen and litigated to a toxin, so that the immunoconjugate targets the cancer, is then internalized and finally releases the toxin. Alternatively, immunoconjugate may consist of an antibody specific for an cancer antigen and litigated to a pro-drug converting enzyme, so that the immunoconjugate is administered in combination with a prodrug, the immunoconjugate targets the cancer and the conversion of the drug is realised in close contact with the cancer cell.

In one embodiment, an immunoconjugate of the invention may be particularly useful for vaccination purposes.

Stimulation of T cell responses indeed requires that relevant antigens gain access to the appropriate intracellular compartments so as to be broken down into peptides, which can then be loaded on major histocompatibility complex (MHC) class I and/or class II molecules (BRYANT & PLOEGH, Curr Opin Immunol, 16, 96-102, 2004; SHASTRI et al., Annu Rev Immunol, 20, 463-93, 2002). Moreover, peptide presentation by cells providing efficient costimulation is important for priming and boosting T cell responses, a requirement met best by dendritic cells (DCs) (BANCHEREAU & PALUCKA, Nat Rev Immunol, 5, 296-306, 2005). The latter also possess the unique functional capacity of presenting via their MHC class I molecules peptides derived from internalized material, a property referred to as cross-presentation (GUERMONPREZ et al., Annu Rev Immunol, 20, 621-67, 2002). Therefore, exploitation of the unique antigen presentation and T cell priming of DCs is an important objective of current vaccine technologies (ADEMA et al., Curr Opin Immunol, 17, 170-4, 2005; BERZOFSKY et al., J Clin Invest, 114, 450-62, 2004; NESTLE et al., Curr Opin Immunol, 17, 163-9, 2005). Antigen targeting to professional APCs should have several important advantages, including reduced consumption of recombinant antigen, increased efficiency of T cell stimulation, and diminished undesired tolerizing effects resulting from antigen presentation by non-professional APCs.

Accordingly, an immunoconjugate consisting of a an antibody specific for a cell surface marker of dendritic cells selected from the group consisting of CD11c DEC-205, DC-SIGN (CD209) or Dectin-1 XCR1, Langerin (CD207), Clec9A, CD68 or CD36. litigated with an antigenic polypeptide as above described is particularly suitable for vaccinating a subject in need thereof.

The immunoconjugate of the invention may be administered in the form of a pharmaceutical composition, as defined below.

Preferably, the immunoconjugates of the invention are administered in a therapeutically effective amount.

The term "therapeutically effective amount" means a sufficient amount of the active ingredients of the invention to treat cancer or infectious disease at a reasonable benefit/risk ratio applicable to any medical treatment.

It will be understood that the total daily usage of the immunoconjugates of the invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific antigen binding formats employed; the duration of the treatment; drugs used in combination or coincidental with the specific antigen binding formats employed; and like factors well known in the medical arts. For example, it is well within the skill of the art to start doses of the antigen binding formats at levels lower than those required to achieve the desired therapeutic effect and to gradually increase the dosage until the desired effect is achieved.

***Diagnostic methods and uses of the invention:***

Immunoconjugates of the invention may also be particularly suitable for diagnosing purposes.

In one embodiment, the immunoconjugate of the invention may be included in an immunoassay.

Typically, the immunoconjugate consists of an antibody litigated with a detectable moiety such as steptavidin, biotin, a fluorescent polypeptide (e.g. GFP (Green Fluorescent Protein), RFP (Red Fluorescent Protein), YFP (yellow Fluorescent Protein), BFP (Blue Fluorescent Protein)), an enzyme that can be used to detect the immunoconjugate (e.g. β-galactosidase, glucose oxidase, peroxidase and alkaline phosphatase) or a protein linked with a radioisotope (such as Be-7, Mg-28, Co-57, Zn-65, Cu-67, Ge-68, Sr-82, Rb-83, Tc-95m, Tc-96, Pd-103, Cd-109, and Xe-127).

Such immunoconjugates are useful in diagnostic methods. Diagnostic methods include, for example, nuclear medicinal methods for locating a disease in a subject, and methods for detecting specific biomarkers in a biological sample from a subject. The immunoconjugates also are useful in immunoassays. For example, the immunoconjugates are useful in flow cytometry techniques and for detecting a cellular location of a specific protein or peptide in a cell. For example the location of a protein in a cell sometimes is detected by a fluorescence imaging technique. Imaging techniques include, for example, using a standard light microscope or a confocal microscope (e.g., U.S. Pat. Nos. 5,283,433 and 5,296,703 (Tsien)). Appropriate light microscopes are commercially available and are useful for probing cells in two dimensions (i. e., the height of a cell often is not resolved), and confocal microscopy is useful for probing cells in three-dimensions. In some embodiments, cells expressing the protein are subjected to a known flow cytometry methods, such as flow microfluorimetry (FMF) and fluorescence activated cell sorting (FACS); U.S. Pat. No. 6,090,919 (Cormack, et al.); U.S. Pat. No. 6,461,813 (Lorens); and U.S. Pat. No. 6,455, 263 (Payan)). For use in these procedures, the protein often is expressed on the cell surface.

A "biological sample" encompasses a variety of sample types obtained from a subject and can be used in a diagnostic or monitoring assay. Biological samples include but are not limited to blood and other liquid samples of biological origin, solid tissue samples such as a biopsy specimen or tissue cultures or cells derived therefrom, and the progeny thereof. For example, biological samples include cells obtained from a tissue sample collected from an individual suspected of having a cancer disease. Therefore, biological samples encompass clinical samples, cells in culture, cell supernatants, cell lysates, serum, plasma, biological fluid, and tissue samples.

***Pharmaceutical compositions and kits of the invention:***

The invention also relates to pharmaceutical compositions comprising an immunoconjugate of the invention.

Therefore, immunoconjugates of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce any adverse, allergic or other untoward reaction when administered to a mammal, especially a human, as appropriate. A pharmaceutically acceptable carrier or excipient refers to a non-toxic solid, semisolid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antigen binding format may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An immunoconjugate of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the required amount of the active compounds in the appropriate solvent with various/several of the other ingredients enumerated above, as required, followed by filtered sterilization. Generally, dispersions are prepared by incorporating the various sterilized active ingredients into a sterile vehicle which contains the basic dispersion medium and the other required ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The immunoconjugates of the invention may be formulated within a therapeutic mixture to comprise about 0.0001 to 1.0 milligrams, or about 0.001 to 1.0 milligrams, or about 0.01 to 1.0 milligrams, or about 0.1 to 1.0 milligrams or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; time release capsules; and any other form currently used.

In certain embodiments, the use of liposomes and/or nanoparticles is contemplated for the introduction of antigen binding formats into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 μm) are generally designed using polymers able to be degraded in vivo. Biodegradable polyalkyl-cyanoacrylate nanoparticles that meet these requirements are contemplated for use in the present invention, and such particles may be easily made.

Liposomes are formed from phospholipids that once dispersed in an aqueous medium spontaneously form multilamellar concentric bilayer vesicles (also termed multilamellar vesicles (MLVs)). MLVs generally have diameters ranging from 25 nm to 4 μm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

### *Kits:*

Finally, the invention also provides kits for preparing an immunoconjugate according to the invention comprising an antibody of the invention or a fragment thereof and an amount of sortase A. The polypeptide of interest (Pi) may also be comprised in the kit. Kits of the invention may contain an antibody coupled to a solid support, e.g., a tissue culture plate or beads (e.g., sepharose beads). Kits can be provided which contain antibodies for detection and quantification in vitro, e.g. in an ELISA or a Western blot.

The invention will be further illustrated by the following figures and examples. However, these examples and figures should not be interpreted in any way as limiting the scope of the present invention.

**FIGURES:**

**Figure1:** Targeting vector bearing knock-in mutation (ET-subcloning of 1016_Synthese_Copoc_(GS)4- LPETGG-(H)6_V2 cassette (SEQ ID NO: 124) in entry vector named1016_COPOC_hCk_LPETGG_04). Targeting vector allowing to substitute the mouse CK exon with a human CK exon containing at its 3' end a sequence coding for a (GS)4LPETGG(H)6 sequence (SEQ ID NO: 124).

**Figure 2:** Targeted region between the 5' and 3' homologous arms (fragment of 10-16 RP23-435I4 Ig-Ck WT Locus v2).

**Figure 3:** Targeted allele in ES cells genome (fragment of 10-16 RP23-435I4 Ig-Ck Mutant ES Cells Locus v2).

**Figure 4:** Locus after deletion of neomycin selection cassette via the Cre recombinase (fragment of 10-16 RP23-435I4 Ig-Ck LPETGG Mice Locus v2).

**Figure 5:** Focus on the modified gene region.

**Figure 6:** Analyze of the B cells found in the blood of a hCk-LPTEG expressing homozygous mouse by Flow cytometry. The B cells of a wild-type mouse and of a hCk-LPTEG expressing homozygous mouse were defined as CD45.1+ CD19+ and stained with a flurescent anti-human CK antibody. Most B cells from the hCk-LPTEG expressing homozygous mouse express high levels of hCK at their cell surface.

**EXAMPLES:**

EXAMPLE 1: Development of a transgenic non-human mammal genetically modified to express an antibody according to the invention.

The aim of is to develop a mouse line carrying allele suitable for the generation of a hCk Knock-in model.

The development of the targeting vector will be conducted using:

- Ready to use selection cassettes validated in ES cells at our plateforme
- loxP sites validated in vitro and in vivo.

The targeting vector will display the following features:

1. Mouse gene name:

Murine Ig-Ck identified on ENSMUSG00000076609 (data base ensembl, igkc gene).

2. Type of genetic modification:

Knock-In (KI)

3. Agreed targeting strategy:

Generation of knock-in mouse model by replacement of mouse Ck gene by human Ck gene in open reading frame with linker sequence (GS)4-LPETGG-(H)6-STOP. The model will be generated by homologous recombination in embryonic stem (ES) cells.

In order to increase following homologous recombination in Ck mouse locus, we propose to extend one homology arm by DTA negative selection marker that will be removed in ES cells recombined in the locus of a given targeted gene.

4. Summary of subcloning steps for the generation of targeting vector:

1. Generation of 5' and 3' homology arms. The choice of these homologous sequences has been established by using BAC RP23-43514. These homology arms contain in total 6797 pb of Ck homologous genomic sequences (2494pb for5' homology arm et 4303pb 3'homology arm).

2. The murine cK coding exon was substituted by the hcK exon:

a. Murine cK sequence (SEQ ID NO: 125):

GCTGATGCTGCACCAACTGTATCCATCTTCCCACCATCCAGTGAGCAGTTAACATCTGGAG
GTGCCTCAGTCGTGTGCTTCTTGAACAACTTCTACCCCAAAGACATCAATGTCAAGTGGAAGATTG
ATGGCAGTGAACGACAAAATGGCGTCCTGAACAGTTGGACTGATCAGGACAGCAAAGACAGCACC
TACAGCATGAGCAGCACCCTCACGTTGACCAAGGACGAGTATGAACGACATAACAGCTATACCTGT
GAGGCCACTCACAAGACATCAACTTCACCCATTGTCAAGAGCTTCAACAGGAATGAGTGTTAG

b. human cK sequence (SEQ ID NO: 126):

ACTGTGGCTGCACCATCTGTCTTCATCTTCCCGCCATCTGATGAGCAGTTGAAATCTGGAAC
TGCCTCTGTTGTGTGCCTGCTGAATAACTTCTATCCCAGAGAGGCCAAAGTACAGTGGAAGGTGGA
TAACGCCCTCCAATCGGGTAACTCCCAGGAGAGTGTCACAGAGCAGGACAGCAAGGACAGCACCT
ACAGCCTCAGCAGCACCCTGACGCTGAGCAAAGCAGACTACGAGAAACACAAAGTCTACGCCTGC
GAAGTCACCCATCAGGGCCTGAGCTCGCCCGTCACAAAGAGCTTCAACAGGGGAGAGTGT

3. Design and generation of vector named 1016_Synthese_Copoc_(GS)4- LPETGG-(H)6 _V2. This vector will permit the replacement of a native sequence of hCk by following sequence hCk-(GS)4-LPETGG-(H)6:

a. human cK following sequence (GS)4- LPETGG-(H)6:

ACTGTGGCTGCACCATCTGTCTTCATCTTCCCGCCATCTGATGAGCAGTTGAAATCTGGAAC
TGCCTCTGTTGTGTGCCTGCTGAATAACTTCTATCCCAGAGAGGCCAAAGTACAGTGGAAGGTGGA
TAACGCCCTCCAATCGGGTAACTCCCAGGAGAGTGTCACAGAGCAGGACAGCAAGGACAGCACCT
ACAGCCTCAGCAGCACCCTGACGCTGAGCAAAGCAGACTACGAGAAACACAAAGTCTACGCCTGC
GAAGTCACCCATCAGGGCCTGAGCTCGCCCGTCACAAAGAGCTTCAACAGGGGAGAGTGTGGTTC
CGGTTCCGGTTCCGGTTCCCTGCCCGAGACAGGAGGACATCATCATCATCATCAT (SEQ ID NO: 127)

4. ET-subcloning of 1016_Synthese_Copoc_(GS)4- LPETGG-(H)6_V2 cassette in entry vector named1016_COPOC_hCk_LPETGG_04

5. Targeting vector bearing knock-in mutation:

See figure 1

6. Sequence of the targeting vector (SEQ ID NO: 128):

CGCGCCGCGGCCGCGGGAATTCGATTATCGAATTCTACCGGGTAGGGGAGGCGCTTTTCCC
AAGGCAGTCTGGAGCATGCGCTTTAGCAGCCCCGCTGGGCACTTGGCGCTACACAAGTGGCCTCTG
GCCTCGCACACATTCCACATCCACCGGTAGGCGCCAACCGGCTCCGTTCTTTGGTGGCCCCTTCGCG
CCACCTTCTACTCCTCCCCTAGTCAGGAAGTTCCCCCCCGCCCCGCAGCTCGCGTCGTGCAGACGTG
ACAAATGGAAGTAGCACGTCTCACTAGTCTCGTGCAGATGGACAGCACCGCTGAGCAATGGAAGC
GGGTAGGCCTTTGGGGCAGCGGCCAATAGCAGCTTTGCTCCTTCGCTTTCTGGGCTCAGAGGCTGG
GAAGGGGTGGGTCCGGGGGCGGGCTCAGGGGCGGGCTCAGGGGCGGGGCGGGCGCCCGAAGGTC
CTCCGGAGGCCCGGCATTCTGCACGCTTCAAAAGCGCACGTCTGCCGCGCTGTTCTCCTCTTCCTCA
TCTCCGGGCCTTTCGACCTGCAGGTCCTCGCCATGGATCCTGATGATGTTGTTGATTCTTCTAAATC
TTTTGTGATGGAAAACTTTTCTTCGTACCACGGGACTAAACCTGGTTATGTAGATTCCATTCAAAAA
GGTATACAAAAGCCAAAATCTGGTACACAAGGAAATTATGACGATGATTGGAAAGGGTTTTATAG
TACCGACAATAAATACGACGCTGCGGGATACTCTGTAGATAATGAAAACCCGCTCTCTGGAAAAGC
TGGAGGCGTGGTCAAAGTGACGTATCCAGGACTGACGAAGGTTCTCGCACTAAAAGTGGATAATG
CCGAAACTATTAAGAAAGAGTTAGGTTTAAGTCTCACTGAACCGTTGATGGAGCAAGTCGGAACG
GAAGAGTTTATCAAAAGGTTCGGTGATGGTGCTTCGCGTGTAGTGCTCAGCCTTCCCTTCGCTGAG
GGGAGTTCTAGCGTTGAATATATTAATAACTGGGAACAGGCGAAAGCGTTAAGCGTAGAACTTGA
GATTAATTTTGAAACCCGTGGAAAACGTGGCCAAGATGCGATGTATGAGTATATGGCTCAAGCCTG
TGCAGGAAATCGTGTCAGGCGATCTCTTTGTGAAGGAACCTTACTTCTGTGGTGTGACATAATTGG
ACAAACTACCTACAGAGATTTAAAGCTCTAAGGTAAATATAAAATTTTTAAGTGTATAATGTGTTA
AACTACTGATTCTAATTGTTTGTGTATTTTAGATTCCAACCTATGGAACTGATGAATGGGAGCAGTG
GTGGAATGCAGATCCTAGAGCTCGCTGATCAGCCTCGACTGTGCCTTCTAGTTGCCAGCCATCTGTT
GTTTGCCCCTCCCCCGTGCCTTCCTTGACCCTGGAAGGTGCCACTCCCACTGTCCTTTCCTAATAAA
ATGAGGAAATTGCATCGCATTGTCTGAGTAGGTGTCATTCTATTCTGGGGGGTGGGGTGGGGCAGG
ACAGCAAGGGGGAGGATTGGGAAGACAATAGCAGGCATGCTGGGGATGCGGTGGGCTCTATGGCT
TCTGAGGCGGAAAGAACCAGCTGGGGCTCGACGAGAACGAAGCGCTAACCGTTTTTATCAGGCTCT
GGGAGGCAGAATAAATGATCATATCGTCAATTATTACCTCCACGGGGAGAGCCTGAGCAAACTGG
CCTCAGGCATTTGAGAAGCACACGGTCACACTGCTTCCGGTAGTCAATAAACCGGTAAACCAGCAA

TAGACATAAGCGGCTATTTAACGACCCTGCCCTGAACCGACGACCGGGTCGAATTTGCTTTCGAAT
TTCTGCCATTCATCCGCTTATTATCACTTATTCAGGCGTAGCAACCAGGCGTTTAAGGGCACCAATA
ACTGCCTTAAAAAAATTACGCCCCGCCCTGCCACTCATCGCAGTACTGTTGTAATTCATTAAGCATT
CTGCCGACATGGAAGCCATCACAAACGGCATGATGAACCTGAATCGCCAGCGGCATCAGCACCTT
GTCGCCTTGCGTATAATATTTGCCCATGGTGAAAACGGGGGCGAAGAAGTTGTCCATATTGGCCAC
GTTTAAATCAAAACTGGTGAAACTCACCCAGGGATTGGCTGAGACGAAAAACATATTCTCAATAAA
CCCTTTAGGGAAATAGGCCAGGTTTTCACCGTAACACGCCACATCTTGCGAATATATGTGTAGAAA
CTGCCGGAAATCGTCGTGGTATTCACTCCAGAGCGATGAAAACGTTTCAGTTTGCTCATGGAAAAC
GGTGTAACAAGGGTGAACACTATCCCATATCACCAGCTCACCGTCTTTCATTGCCATACGGAATTC
CGGATGAGCATTCATCAGGCGGGCAAGAATGTGAATAAAGGCCGGATAAAACTTGTGCTTATTTTT
CTTTACGGTCTTTAAAAAGGCCGTAATATCCAGCTGAACGGTCTGGTTATAGGTACATTGAGCAAC
TGACTGAAATGCCTCAAAATGTTCTTTACGATGCCATTGGGATATATCAACGGTGGTATATCCAGT
GATTTTTTTCTCCATTTTAGCTTCCTTAGCTCCTGAAAATCTCGATAACTCAAAAAATACGCCCGGT
AGTGATCTTATTTCATTATGGTGAAAGTTGGAACCTCTTACGTGCCGATCAACGTCTCATTTTCGCC
AAAAGTTGGCCCAGGGCTTCCCGGTATCAACAGGGACACCAGGATTTATTTATTCTGCGAAGTGAT
CTTCCGTCACAGGTATTTATTCGGCGCAAAGTGCGTCGGGTGATGCTGCCAACTTACTGATTTAGTG
TATGATGGTGTTTTTGAGGTGCTCCAGTGGCTTCTGTTTCTATCAGCTGTCCCTCCTGTTCAGCTACT
GACGGGGTGGTGCGTAACGGCAAAAGCACCGCCGGACATCAGCGCTGATAATCACTAGTGAATTC
GCGGCCGCATCTTTTTTTATGTGTAAGACACAGGTTTTCATGTTAGGAGTTAAAGTCAGTTCAGAAA
ATCTTGAGAAAATGGAGAGGGCTCATTATCAGTTGACGTGGCATACAGTGTCAGATTTTCTGTTTAT
CAAGCTAGTGAGATTAGGGGCAAAAAGAGGCTTTAGTTGAGAGGAAAGTAATTAATACTATGGTC
ACCATCCAAGAGATTGGATCGGAGAATAAGCATGAGTAGTTATTGAGATCTGGGTCTGACTGCAGG
TAGCGTGGTCTTCTAGACGTTTAAGTGGGAGATTTGGAGGGGATGAGGAATGAAGGAACTTCAGG
ATAGAAAAGGGCTGAAGTCAAGTTCAGCTCCTAAAATGGATGTGGGAGCAAACTTTGAAGATAAA
CTGAATGACCCAGAGGATGAAACAGCGCAGATCAAAGAGGGGCCTGGAGCTCTGAGAAGAGAAG
GAGACTCATCCGTGTTGAGTTTCCACAAGTACTGTCTTGAGTTTTGCAATAAAAGTGGGATAGCAG
AGTTGAGTGAGCCGTAGGCTGAGTTCTCTCTTTTGTCTCCTAAGTTTTTATGACTACAAAAATCAGT
AGTATGTCCTGAAATAATCATTAAGCTGTTTGAAAGTATGACTGCTTGCCATGTAGATACCATGGCT
TGCTGAATAATCAGAAGAGGTGTGACTCTTATTCTAAAATTTGTCACAAAATGTCAAAATGAGAGA
CTCTGTAGGAACGAGTCCTTGACAGACAGCTCAAGGGGTTTTTTTCCTTTGTCTCATTTCTACATGA
AAGTAAATTTGAAATGATCTTTTTTATTATAAGAGTAGAAATACAGTTGGGTTTGAACTATATGTTT
TAATGGCCACGGTTTTGTAAGACATTTGGTCCTTTGTTTTCCCAGTTATTACTCGATTGTAATTTTAT
ATCGCCAGCAATGGACTGAAACGGTCCGCAACCTCTTCTTTACAACTGGGTGACCTCGCGGCTGTG
CCAGCCATTTGGCGTTCACCCTGCCGCTAAGGGCCATGTGAACCCCCGCGGTAGCATCCCTTGCTCC
GCGTGGACCACTTTCCTGAGGCACAGTGATAGGAACAGAGCCACTAATCTGAAGAGAACAGAGAT
GTGACAGACTACACTAATGTGAGAAAAACAAGGAAAGGGTGACTTATTGGAGATTTCAGAAATAA
AATGCATTTATTATTATATTCCCTTATTTTAATTTTCTATTAGGGAATTAGAAAGGGCATAAACTGC
TTTATCCAGTGTTATATTAAAAGCTTAATGTATATAATCTTTTAGAGGTAAAATCTACAGCCAGCAA
AAGTCATGGTAAATATTCTTTGACTGAACTCTCACTAAACTCCTCTAAATTATATGTCATATTAACT
GGTTAAATTAATATAAATTTGTGACATGACCTTAACTGGTTAGGTAGGATATTTTTCTTCATGCAA

AATATGACTAATAATAATTTAGCACAAAAATATTTCCCAATACTTTAATTCTGTGATAGAAAAATG
TTTAACTCAGCTACTATAATCCCATAATTTTGAAAACTATTTATTAGCTTTTGTGTTTGACCCTTCCC
TAGCCAAAGGCAACTATTTAAGGACCCTTTAAAACTCTTGAAACTACTTTAGAGTCATTAAGTTATT
TAACCACTTTTAATTACTTTAAAATGATGTCAATTCCCTTTTAACTATTAATTTATTTTAAGGGGGG
AAAGGCTGCTCATAATTCTATTGTTTTTCTTGGTAAAGAACTCTCAGTTTTCGTTTTTACTACCTCTG
TCACCCAAGAGTTGGCATCTCAACAGAGGGGACTTTCCGAGAGGCCATCTGGCAGTTGCTTAAGAT
CAGAAGTGAAGTCTGCCAGTTCCTCCCAGGCAGGTGGCCCAGATTACAGTTGACCTGTTCTGGTGT
GGCTAAAAATTGTCCCATGTGGTTACAAACCATTAGACCAGGGTCTGATGAATTGCTCAGAATATT
TCTGGACACCCAAATACAGACCCTGGCTTAAGGCCCTGTCCATACAGTAGGTTTAGCTTGGCTACA
CCAAAGGAAGCCATACAGAGGCTAATATCAGAGTATTCTTGGAAGAGACAGGAGAAAATGAAAGC
CAGTTTCTGCTCTTACCTTATGTGCTTGTGTTCAGACTCCCAAACATCAGGAGTGTCAGATAAACTG
GTCTGAATCTCTGTCTGAAGCATGGAACTGAAAAGAATGTAGTTTCAGGGAAGAAAGGCAATAGA
AGGAAGCCTGAGAATATCTTCAAAGGGTCAGACTCAATTTACTTTCTAAAGAAGTAGCTAGGAACT
AGGGAATAACTTAGAAACAACAAGATTGTATATATGTGCATCCTGGCCCCATTGTTCCTTATCTGTA
GGGATAAGCGTGCTTTTTTGTGTGTCTGTATATAACATAACTGTTTACACATAATACACTGAAATGG
AGCCCTTCCTTGTTACTTCATACCATCCTCTGTGCTTCCTTCCTCAGGAACTGTGGCTGCACCATCTG
TCTTCATCTTCCCGCCATCTGATGAGCAGTTGAAATCTGGAACTGCCTCTGTTGTGTGCCTGCTGAA
TAACTTCTATCCCAGAGAGGCCAAAGTACAGTGGAAGGTGGATAACGCCCTCCAATCGGGTAACTC
CCAGGAGAGTGTCACAGAGCAGGACAGCAAGGACAGCACCTACAGCCTCAGCAGCACCCTGACGC
TGAGCAAAGCAGACTACGAGAAACACAAAGTCTACGCCTGCGAAGTCACCCATCAGGGCCTGAGC
TCGCCCGTCACAAAGAGCTTCAACAGGGGAGAGTGTGGTTCCGGTTCCGGTTCCGGTTCCCTGCCC
GAGACAGGAGGACATCATCATCATCATCATTGAAGACAAACCCGATCTCGAGGTCGACTCGCGAA
AGCTAGCTTATAACTTCGTATAGCATACATTATACGAAGTTATGGATCTCCATGGGCCAGGCAAAT
ATCCCTTACCAGCCTCACAGAGACCTCCCCCACCCCCCGCAACCCTAGAGTTCTTTTACTAGTGAGG
GACAAGTGGACAATGGTGCTGTTGTGGGCCCCACCCTGTGTCCCCTGTGCCCACAGTGGTCACTCT
GCTTGGCAGGCAGGTGTTGCAGGCTGGCTGCTCCAGGCCCTGGCAGGAGGTACTGAAGGACCTGGT
AGGCTCAGATGCCCTGGATGCCAAGGCACTGCTGGAGTACTTCCAACCGGTCAGCCAGTGGCTGGA
AGAGCAGAATCAGCGGAATGGCGAAGTCCTAGGCTGGCCAGAGAATCAGTGGCGTCCACCGTTAC
CCGACAACTATCCAGAGGGCATTGGTAAAGCTCTGAGTGAGGGTGGACTGGGACCAAGAGAAGTC
CTGGCCTCTGGCCTCTGGCTTCTGGGTCAAAGCCTCAGCATCCTGGTCACTTTGCTGCCAGCTGAGC
CCCAGTGTCCTTTGCTTCAGTGCCAAGCCACCCCTGGGCTCATCCTCAGGGCCCTAAGCAGAAATG
GGTATGTCTTTCTCTCAGGGTCCTAGAGACAGTGTGCCCAAGCCTGAGGGCCCTTGGGGTCAGGCT
GGCTGGCACATTGCTCTATGAGGTCACACTGCAGGCTTGGCTCTTATTGGCCGGTGATGGGAGCTT
CAGGGCTCTGCTTTCCTGCGGCCATGCCCAAGAAGAAGAGGAAGGTGTCCAATTTACTGACCGTAC
ACCAAAATTTGCCTGCATTACCGGTCGATGCAACGAGTGATGAGGTTCGCAAGAACCTGATGGACA
TGTTCAGGGATCGCCAGGCGTTTTCTGAGCATACCTGGAAAATGCTTCTGTCCGTTTGCCGGTCGTG
GGCGGCATGGTGCAAGTTGAATAACCGGAAATGGTTTCCCGCAGAACCTGAAGATGTTCGCGATTA
TCTTCTATATCTTCAGGCGCGCGGTCTGGCAGTAAAAACTATCCAGCAACATTTGGGCCAGCTAAA
CATGCTTCATCGTCGGTCCGGGCTGCCACGACCAAGTGACAGCAATGCTGTTTCACTGGTTATGCG
GCGGATCCGAAAAGAAAACGTTGATGCCGGTGAACGTGCAAAACAGGCTCTAGCGTTCGAACGCA

CTGATTTCGACCAGGTTCGTTCACTCATGGAAAATAGCGATCGCTGCCAGGATATACGTAATCTGG
CATTTCTGGGGATTGCTTATAACACCCTGTTACGTATAGCCGAAATTGCCAGGATCAGGGTTAAAG
ATATCTCACGTACTGACGGTGGGAGAATGTTAATCCATATTGGCAGAACGAAAACGCTGGTTAGCA
CCGCAGGTGTAGAGAAGGCACTTAGCCTGGGGGTAACTAAACTGGTCGAGCGATGGATTTCCGTCT
CTGGTGTAGCTGATGATCCGAATAACTACCTGTTTTGCCGGGTCAGAAAAAATGGTGTTGCCGCGC
CATCTGCCACCAGCCAGCTATCAACTCGCGCCCTGGAAGGGATTTTTGAAGCAACTCATCGATTGA
TTTACGGCGCTAAGGTAAATATAAAATTTTTAAGTGTATAATGTGTTAAACTACTGATTCTAATTGT
TTGTGTATTTTAGGATGACTCTGGTCAGAGATACCTGGCCTGGTCTGGACACAGTGCCCGTGTCGG
AGCCGCGCGAGATATGGCCCGCGCTGGAGTTTCAATACCGGAGATCATGCAAGCTGGTGGCTGGA
CCAATGTAAATATTGTCATGAACTATATCCGTAACCTGGATAGTGAAACAGGGGCAATGGTGCGCC
TGCTGGAAGATGGCGATTAGCCATTAACGCGTAAATGATTGCTATAATTATTTGATATTTATGGTGA
CATATGAGAAAGGATTTCAACATCGACGGAAAATATGTAGTGCTGTCTGTAAGCACTAATATTCAG
TCGCCAGCCGTCATTGTCACTGTAAAGCTGAGCGGCAATAAAAAGACAGAATAAAACGCACGGGT
GTTGGGTCGTTTGTTCGGTCGAGCTCGCGAAAGCTTCTACCGGGTAGGGGAGGCGCTTTTCCCAAG
GCAGTCTGGAGCATGCGCTTTAGCAGCCCCGCTGGGCACTTGGCGCTACACAAGTGGCCTCTGGCC
TCGCACACATTCCACATCCACCGGTAGGCGCCAACCGGCTCCGTTCTTTGGTGGCCCCGTCGCGCC
ACCTTCTACTCCTCCCCTAGTCAGGAAGTTCCCCCCCGCCCCGCAGCTCGCGTCGTGCAGGACGTGA
CAAATGGAAGTAGCACGTCTCACTAGTCTCGTGCAGATGGACAGCACCGCTGAGCAATGGAAGCG
GGTAGGCCTTTGGGGCAGCGGCCAATAGCAGCTTTGCTCCTTCGCTTTCTGGGCTCAGAGGCTGGG
AAGGGGTGGGTCCGGGGGCGGGCTCAGGGGCGGGCTCAGGGGCGGGGCGGGCGCCCGAAGGTCC
TCCGGAGGCCCGGCATTCTGCACGCTTCAAAAGCGCACGTCTGCCGCGCTGTTCTCCTCTTCCTCAT
CTCCGGGCCTTTCGACCTGCAGCAGCACGTGTTGACAATTAATCATCGGCATAGTATATCGGCATA
GTATAATACGACAAGGTGAGGAACTAAACCATGGGATCGGCCATTGAACAAGATGGATTGCACGC
AGGTTCTCCGGCCGCTTGGGTGGAGAGGCTATTCGGCTATGACTGGGCACAACAGACGATCGGCTG
CTCTGATGCCGCCGTGTTCCGGCTGTCAGCGCAGGGGCGCCCGGTTCTTTTTGTCAAGACCGACCTG
TCCGGTGCCCTGAATGAACTGCAGGACGAGGCAGCGCGGCTATCGTGGCTGGCCACGACGGGCGT
TCCTTGCGCAGCTGTGCTCGACGTTGTCACTGAAGCGGGAAGGGACTGGCTGCTATTGGGCGAAGT
GCCGGGGCAGGATCTCCTGTCATCTCACCTTGCTCCTGCCGAGAAAGTATCCATCATGGCTGATGC
AATGCGGCGGCTGCATACGCTTGATCCGGCTACCTGCCCATTCGACCACCAAGCGAAACATCGCAT
CGAGCGAGCACGTACTCGGATGGAAGCCGGTCTTGTCGATCAGGATGATCTGGACGAAGAGCATC
AGGGGCTCGCGCCAGCCGAACTGTTCGCCAGGCTCAAGGCGCGCATGCCCGACGGCGAGGATCTC
GTCGTGACCCATGGCGATGCCTGCTTGCCGAATATCATGGTGGAAAATGGCCGCTTTTCTGGATTC
ATCGACTGTGGCCGGCTGGGTGTGGCGGACCGCTATCAGGACATAGCGTTGGCTACCCGTGATATT
GCTGAAGAGCTTGGCGGCGAATGGGCTGACCGCTTCCTCGTGCTTTACGGTATCGCCGCCCCCGAT
TCGCAGCGCATCGCCTTCTATCGCCTTCTTGACGAGTTCTTCTGAGCGGGACTCTGGGGTTCGAATA
AAGACCGACCAAGCGACGTCTGAGAGCTCCCTGGCGAATTCGGTACCAATAAAAGAGCTTTATTTT
CATGATCTGTGTGTTGGTTTTTGTGTGCGGCGCGATAACTTCGTATAGCATACATTATACGAAGTTA
TCCCAATTCATCGATATCTAGATCGGGGTCCTGAGACGCCACCACCAGCTCCCCAGCTCCATCCTAT
CTTCCCTTCTAAGGTCTTGGAGGCTTCCCCACAAGCGACCTACCACTGTTGCGGTGCTCCAAACCTC
CTCCCCACCTCCTTCTCCTCCTCCTCCCTTTCCTTGGCTTTTATCATGCTAATATTTGCAGAAAATAT

TCAATAAAGTGAGTCTTTGCACTTGAGATCTCTGTCTTTCTTACTAAATGGTAGTAATCAGTTGTTTT
TCCAGTTACCTGGGTTTCTCTTCTAAAGAAGTTAAATGTTTAGTTGCCCTGAAATCCACCACACTTA
AAGGATAAATAAAACCCTCCACTTGCCCTGGTTGGCTGTCCACTACATGGCAGTCCTTTCTAAGGTT
CACGAGTACTATTCATGGCTTATTTCTCTGGGCCATGGTAGGTTTGAGGAGGCATACTTCCTAGTTT
TCTTCCCCTAAGTCGTCAAAGTCCTGAAGGGGGACAGTCTTTACAAGCACATGTTCTGTAATCTGAT
TCAACCTACCCAGTAAACTTGGCGAAGCAAAGTAGAATCATTATCACAGGAAGCAAAGGCAACCT
AAATGTGCAAGCAATAGGAAAATGTGGAAGCCCATCATAGTACTTGGACTTCATCTGCTTTTGTGC
CTTCACTAAGTTTTTAAACATGAGCTGGCTCCTATCTGCCATTGGCAAGGCTGGGCACTACCCACAA
CCTACTTCAAGGACCTCTATACCGTGAGATTACACACATACATCAAAATTTGGGAAAAGTTCTACC
AAGCTGAGAGCTGATCACCCCACTCTTAGGTGCTTATCTCTGTACACCAGAAACCTTAAGAAGCAA
CCAGTATTGAGAGACTCATTTATGAAAGTCTAAAACTGGATACAACCAAAATGTCCACCAACAGTT
AAATTATGACATGTTCACAATTGAGCTATTACTTAATAAGGAGAATTAATAAAATAAAACTTAAGA
GCATAGTTTAATCTCATAAACAAGATAATAAGCAAAACAAAACATTTTTTCATCCATGTAAGTTTA
AAAGCAGGTAAAATTTAAAATTAAGAGAGACATAAGTTTTGAGGTAGCAAGATGGAAACTCTGGG
GCTTGGGGAATGTTCTGTCTCTCTGTATGGGATGTGAAAGTTACTATTGTGGAATTGGGATCTATGT
TCTTCCTGTATATATTGTATACTTCATAATAACTTCACCTAAAGAAATATCTAATACCCAGTGCATA
CATAAAAGAGGATACAAGGAATGAATCATACGTCAAGGCCAGAAAGACAATAAAGTAGGGGATC
CAGGATCAAATCTCCCACAACCTTGAGCCTTCTACTATTCTGCCTTCCAGAGCTCAAAGTACAAAA
CACATAATTCAAACACATGATCCCTCCTTGGGGTCTCTTCCTTCATGCATCGAATTAGAAATAGCCA
TGTATAAAATGAGATAGAAGAGACCTTCATCAACAGGTCAAAGAATATAGGTAATTTTGTCTGGGT
ATGAAGAGCCCACGTATCAAAGGTTACATTAGGGAAGGAAGAGGACACTAACAGTGACTTTCATT
CTCCCCCTCTTCCTGGAGGCCCCTGCATTTAGTCCCTCGTGGGCTCATCCACTCAGCACACATTTAC
TAAGCATCTTCTCAGCCTACACTCTGAAGGCAGTGCAGAATAATGTTAGTGTCCCTTCCCCCAGTTA
ATATGCAGTCCAGTTTCCCTGCTCCTTCCCTTTCTCAGTCCACATAAGGATGATGGGAAAGGACAGT
CACCAAATAGGAGAGGGCAACCCTTTGCCTTCCTACCTCTTGAGAATGTACATTATTATCCACTTTT
TGAAACTTCTTTTAATTGCTTTTTTTTAATTTGTCTTTTCAAATAGCATAACCTTGTTCATCCATTTCT
GGGAACCAAATTTATCAATCAACAGTGCCTCTAATCTGGCTATTAATACAAAAATGCCTCCTCAAA
ATATATATGTTCGAGTCTTATCTAAAACAGAACCCACAATAAAAAAGAAGAAAGAATACATATAA
GCATTTATATAATTCTGAGCAACCTTGTGCTTTGTGAAAAAAATATAATCTAATGTCACATGCTGTA
TTCTTTTTATTTAACACTGGTGAAATTATACCATTAGAGAGAAAGAGGACAGATCACTGATCCTAG
GATCTAGGGATGTTACAGATAAGAAAACAAATGTGACAAAGAGCTGTCACAAGGAGGATCTTCAA
GGTCACAGAATCACTGTCTTGATTTCAGTGGTGGTTACATACATTTAAATATGTGATAAAATGTTGT
TGAACTATATTCATATATTGTACCAATGTCAAATGCTTAATTTTGGCTCTATAGTATAATTATGCAC
TAAATAACTATTTGGACAAAGAAAATGATGTTTACATCAAAGGTGAGGCCATATTTGTTAGGAACA
TAACTTAAAAACCATTTTGGATAACTAATGAAAAGCCATTTTGTGTGCCTTGGCATATCATGCCTAA
GCTGTCACCAGATAGATCTAATAAGACCTAAGCCTCAGAAGCAAGCCCCTGCCCAGCAAGCAGGC
AGCACAGATAAGAGCTAAACCCAGGACAGGCCATGATATGCTAATGAACTACCTTCAAGGTGGTG
TTGCTGACCTAGTGAACCAGCCCCAAGCTGTGAGCCCCAATAGCACAAAGCTACTGCCCAAAGAA
ATTATACAAAAATTGGAACTTTGGGAATGGTGTGCAGGATCGCTCTGCTGTATGCCTGGAACACAG
CTTCTCTATGTTTTGTATTGATACCAGTCTAGAAGCTTCCAAAACTTTCTCACTGAAGAAGATTCCC

CATGTGGGACCCCTACAGACTCTTTTGCCCAAACAACTGCTTCCCTCCTGGTGTGATATCTGTTTTG
CTTTTATGTTAGCATAATATTATAAGGAATGTTTGTGTGAATAAACCAAACATATTTTAAAAGCAA
ATATTGTATGCACATCCTAATTGCTAAAAAGTTTACAGCTAATAGTCCCATGCTCTCCACAATACTG
GATCCAAATAAGTCCTAATTTCAATGTTGGGCATCTTTACAGAGAGAAAGACATTAAAAATGAAGA
GACATGCAGAGAGTGCACCATGCCATCGTGGAGACAGACTGAAGTGACACAACTGTTAGTCAAAG
AGGATTAAGGACTTCCAGAAGCCACCAAAGGAAGGAGGTATGAAGTGGTTTCTCCCTCAGAGTAT
CCAGAGGAGACTAAACCAACCAACACCTTTTTGCTTAAGACTTCTTGCCTTCAGGACTGTGAGAAG
GTAGCTTCCTATTGTTCTAAGCCCCAGTATGTGGCATTTTGTTAAGGTAGAGTCAAGAAACCAATA
AAATGCAGACAGACAAAAGGATAGCTGAGTTTTCCAGGCCCTTCCTTCTTATTTTTGGTTTTGTTGG
TGGTGGTGGTGGTGGTGGTGATGGTGGTGGTTTTGTTTATGTTTTGTTTGGGGAGTTTTTTGGGGTTT
TTTTGGGTTTTGTTTTTGTTGTTGTTTTGGGGGTTTTTGTTGTTGTTGTTGTTTGCTTTTTTGTTTTTTG
TTTTTTGTTTTTTTGAGACAGTGTTTCTCTGTATAGCCCTGGCTGTCCTGGAGTTCCTTCTATCTCTA
ATGTCTACATCTCAGAGGGGATCCTCTAATTTCAAATGAGCAGTAGCTCTCCATTTTTAGCTCTTAT
TTATTCATTTATTTACTTACTTACTTATTGTCTGTAGATGAAAGAATTTTGGAGTGGGAAAGGGTTC
ATGAGCCCCCAGCAACTAATGAGGAGCTACAGACAATTGATGTTTCTGGGGAAAGGAGACTCAGT
TTCTTTGAGAGTATAGCTTCTGATGGGTCAACCATGTTCCTGTGGCTGATGTCACACCCAGGAGTAT
GCAGACAACAGAAACTGGAGTTAATGAGTTGTTTTAAAAATAAAAAAGGGCATGAAGCTTGGGAT
AGAAATTAAGGATAAATACAATTAAATACAGGAAATTCTGAAAGAATTAATAAAAACATTTCTTTT
TTTAAAAAAAAATCCAGAATTAGCTATGCTTCTTCAAAATTGCTTCTGGAGAACTTTACAAGTTAA
ATAAGTTATATTGTAGAAAAGGTAGAGAGGAGAATAGTGGAAGAGAGAGATAAGGAGACTTCAA
AAGGAGTGGAGGGAGATAGAGGAGGAGAAAGCAGAAGCAATGGCTGATAGACACAGGATAAGAG
GGAACAGAAAGGAGAAAGAGGAAGCCAGGATGGGTATTTCTTTGCCTATCTGTGACTTGCACATG
GTCTTGGCAATTATTGATGCCTCGAGGGGGGGCCCGGTACCCAGCTTTTGTTCCCTTTAGTGAGGGT
TAATTGCGCGCTTGGCGTAATCATGGTCATAGCTGTTTCCTGTGTGAAATTGTTATCCGCTCACAAT
TCCACACAACATACGAGCCGGGAGCATAAAGTGTAAAGCCTGGGGTGCCTAATGAGTGAGCTAAC
TCACATTAATTGCGTTGCGCTCACTGCCCGCTTTCCAGTCGGGAAACCTGTCGTGCCAGCTGCATTA
ATGAATCGGCCAACGCGCGGGGAGAGGCGGTTTGCGTATTGGGCGCTCTTCCGCTTCCTCGCTCAC
TGACTCGCTGCGCTCGGTCGTTCGGCTGCGGCGAGCGGTATCAGCTCACTCAAAGGCGGTAATACG
GTTATCCACAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGACGAGCATCAC
AAAAATCGACGCTCAAGTCAGAGGTGGCGAAACCCGACAGGACTATAAAGATACCAGGCGTTTCC
CCCTGGAAGCTCCCTCGTGCGCTCTCCTGTTCCGACCCTGCCGCTTACCGGATACCTGTCCGCCTTT
CTCCCTTCGGGAAGCGTGGCGCTTTCTCATAGCTCACGCTGTAGGTATCTCAGTTCGGTGTAGGTCG
TTCGCTCCAAGCTGGGCTGTGTGCACGAACCCCCCGTTCAGCCCGACCGCTGCGCCTTATCCGGTA
ACTATCGTCTTGAGTCCAACCCGGTAAGACACGACTTATCGCCACTGGCAGCAGCCACTGGTAACA
GGATTAGCAGAGCGAGGTATGTAGGCGGTGCTACAGAGTTCTTGAAGTGGTGGCCTAACTACGGCT
ACACTAGAAGGACAGTATTTGGTATCTGCGCTCTGCTGAAGCCAGTTACCTTCGGAAAAAGAGTTG
GTAGCTCTTGATCCGGCAAACAAACCACCGCTGGTAGCGGTGGTTTTTTTGTTTGCAAGCAGCAGA
TTACGCGCAGAAAAAAAGGATCTCAAGAAGATCCTTTGATCTTTTCTACGGGGTCTGACGCTCAGT
GGAACGAAAACTCACGTTAAGGGATTTTGGTCATGAGATTATCAAAAAGGATCTTCACCTAGATCC

TTTTAAATTAAAAATGAAGTTTTAAATCAATCTAAAGTATATATGAGTAAACTTGGTCTGACAGTTA CCAATGCTTAATCAGTGAGGCACCTATCTCAGCGATCTGTCTATTTCGTTCATCCATAGTTGCCTGA CTCCCCGTCGTGTAGATAACTACGATACGGGAGGGCTTACCATCTGGCCCCAGTGCTGCAATGATA CCGCGAGACCCACGCTCACCGGCTCCAGATTTATCAGCAATAAACCAGCCAGCCGGAAGGGCCGA GCGCAGAAGTGGTCCTGCAACTTTATCCGCCTCCATCCAGTCTATTAATTGTTGCCGGGAAGCTAG AGTAAGTAGTTCGCCAGTTAATAGTTTGCGCAACGTTGTTGCCATTGCTACAGGCATCGTGGTGTCA CGCTCGTCGTTTGGTATGGCTTCATTCAGCTCCGGTTCCCAACGATCAAGGCGAGTTACATGATCCC CCATGTTGTGCAAAAAAGCGGTTAGCTCCTTCGGTCCTCCGATCGTTGTCAGAAGTAAGTTGGCCG CAGTGTTATCACTCATGGTTATGGCAGCACTGCATAATTCTCTTACTGTCATGCCATCCGTAAGATG CTTTTCTGTGACTGGTGAGTACTCAACCAAGTCATTCTGAGAATAGTGTATGCGGCGACCGAGTTG CTCTTGCCCGGCGTCAATACGGGATAATACCGCGCCACATAGCAGAACTTTAAAAGTGCTCATCAT TGGAAAACGTTCTTCGGGGCGAAAACTCTCAAGGATCTTACCGCTGTTGAGATCCAGTTCGATGTA ACCCACTCGTGCACCCAACTGATCTTCAGCATCTTTTACTTTCACCAGCGTTTCTGGGTGAGCAAAA ACAGGAAGGCAAAATGCCGCAAAAAAGGGAATAAGGGCGACACGGAAATGTTGAATACTCATACT CTTCCTTTTTCAATATTATTGAAGCATTTATCAGGGTTATTGTCTCATGAGCGGATACATATTTGAAT GTATTTAGAAAAATAAACAAATAGGGGTTCCGCGCACATTTCCCCGAAAAGTGCCACCTAAATTGT AAGCGTTAATATTTTGTTAAAATTCGCGTTAAATTTTTGTTAAATCAGCTCATTTTTTAACCAATAG GCCGAAATCGGCAAAATCCCTTATAAATCAAAAGAATAGACCGAGATAGGGTTGAGTGTTGTTCCA GTTTGGAACAAGAGTCCACTATTAAAGAACGTGGACTCCAACGTCAAAGGGCGAAAAACCGTCTA TCAGGGCGATGGCCCACTACGTGAACCATCACCCTAATCAAGTTTTTTGGGGTCGAGGTGCCGTAA AGCACTAAATCGGAACCCTAAAGGGAGCCCCCGATTTAGAGCTTGACGGGGAAAGCCGGCGAACG TGGCGAGAAAGGAAGGGAAGAAAGCGAAAGGAGCGGGCGCTAGGGCGCTGGCAAGTGTAGCGGT CACGCTGCGCGTAACCACCACACCCGCCGCGCTTAATGCGCCGCTACAGGGCGCGTCCCATTCGCC ATTCAGGCTGCGCAACTGTTGGGAAGGGCGATCGGTGCGGGCCTCTTCGCTATTACGCCAGCTGGC GAAAGGGGGATGTGCTGCAAGGCGATTAAGTTGGGTAACGCCAGGGTTTTCCCAGTCACGACGTTG TAAAACGACGGCCAGTGAGCGCGCGTAATACGACTCACTATAGGGCGAATTGGAGCTCCACCGCG GTGGCAGCCGCTGG

5. <u>Mouse gene organization of wild type locus of IgCk gene region:</u>

Sequence of the targeted region between 5' and 3' homologous arms (figure 2, SEQ ID NO: 129) with:

- *XXXX*: 5' homologous arm
- ***XXXX*:** mouse cK sequence
- <u>*XXXX*</u>: 3' homologous arm

*ATCTTTTTTTATGTGTAAGACACAGGTTTTCATGTTAGGAGTTAAAGTCAGTTCAGAAAATCTTGAG AAAATGGAGAGGGCTCATTATCAGTTGACGTGGCATACAGTGTCAGATTTTCTGTTTATCAAGCTAGTGAG*

*ATTAGGGGCAAAAAGAGGCTTTAGTTGAGAGGAAAGTAATTAATACTATGGTCACCATCCAAGAGATTGGA*
*TCGGAGAATAAGCATGAGTAGTTATTGAGATCTGGGTCTGACTGCAGGTAGCGTGGTCTTCTAGACGTTTA*
*AGTGGGAGATTTGGAGGGGATGAGGAATGAAGGAACTTCAGGATAGAAAAGGGCTGAAGTCAAGTTCAG*
*CTCCTAAAATGGATGTGGGAGCAAACTTTGAAGATAAACTGAATGACCCAGAGGATGAAACAGCGCAGAT*
*CAAAGAGGGGCCTGGAGCTCTGAGAAGAGAAGGAGACTCATCCGTGTTGAGTTTCCACAAGTACTGTCTT*
*GAGTTTTGCAATAAAAGTGGGATAGCAGAGTTGAGTGAGCCGTAGGCTGAGTTCTCTCTTTTGTCTCCTAA*
*GTTTTTATGACTACAAAAATCAGTAGTATGTCCTGAAATAATCATTAAGCTGTTTGAAAGTATGACTGCTTGC*
*CATGTAGATACCATGGCTTGCTGAATAATCAGAAGAGGTGTGACTCTTATTCTAAAATTTGTCACAAAATGT*
*CAAAATGAGAGACTCTGTAGGAACGAGTCCTTGACAGACAGCTCAAGGGGTTTTTTCCTTTGTCTCATTTC*
*TACATGAAAGTAAATTTGAAATGATCTTTTTTATTATAAGAGTAGAAATACAGTTGGGTTTGAACTATATGTTT*
*TAATGGCCACGGTTTTGTAAGACATTTGGTCCTTTGTTTTCCCAGTTATTACTCGATTGTAATTTTATATCGC*
*CAGCAATGGACTGAAACGGTCCGCAACCTCTTCTTTACAACTGGGTGACCTCGCGGCTGTGCCAGCCATT*
*TGGCGTTCACCCTGCCGCTAAGGGCCATGTGAACCCCCGCGGTAGCATCCCTTGCTCCGCGTGGACCAC*
*TTTCCTGAGGCACAGTGATAGGAACAGAGCCACTAATCTGAAGAGAACAGAGATGTGACAGACTACACTAA*
*TGTGAGAAAAACAAGGAAAGGGTGACTTATTGGAGATTTCAGAAATAAAATGCATTTATTATTATATTCCCTT*
*ATTTTAATTTTCTATTAGGGAATTAGAAAGGGCATAAACTGCTTTATCCAGTGTTATATTAAAAGCTTAATGTA*
*TATAATCTTTTAGAGGTAAAATCTACAGCCAGCAAAAGTCATGGTAAATATTCTTTGACTGAACTCTCACTAA*
*ACTCCTCTAAATTATATGTCATATTAACTGGTTAAATTAATATAAATTTGTGACATGACCTTAACTGGTTAGGT*
*AGGATATTTTTCTTCATGCAAAAATATGACTAATAATAATTTAGCACAAAAATATTTCCCAATACTTTAATTCT*
*GTGATAGAAAAATGTTTAACTCAGCTACTATAATCCCATAATTTTGAAAACTATTTATTAGCTTTTGTGTTTGA*
*CCCTTCCCTAGCCAAAGGCAACTATTTAAGGACCCTTTAAAACTCTTGAAACTACTTTAGAGTCATTAAGTT*
*ATTTAACCACTTTTAATTACTTTAAAATGATGTCAATTCCCTTTTAACTATTAATTTATTTTAAGGGGGGAAAG*
*GCTGCTCATAATTCTATTGTTTTTCTTGGTAAAGAACTCTCAGTTTTCGTTTTTACTACCTCTGTCACCCAAG*
*AGTTGGCATCTCAACAGAGGGGACTTTCCGAGAGGCCATCTGGCAGTTGCTTAAGATCAGAAGTGAAGTC*
*TGCCAGTTCCTCCCAGGCAGGTGGCCCAGATTACAGTTGACCTGTTCTGGTGTGGCTAAAAATTGTCCCAT*
*GTGGTTACAAACCATTAGACCAGGGTCTGATGAATTGCTCAGAATATTTCTGGACACCCAAATACAGACCC*
*TGGCTTAAGGCCCTGTCCATACAGTAGGTTTAGCTTGGCTACACCAAAGGAAGCCATACAGAGGCTAATAT*
*CAGAGTATTCTTGGAAGAGACAGGAGAAAATGAAAGCCAGTTTCTGCTCTTACCTTATGTGCTTGTGTTCA*
*GACTCCCAAACATCAGGAGTGTCAGATAAACTGGTCTGAATCTCTGTCTGAAGCATGGAACTGAAAAGAAT*
*GTAGTTTCAGGGAAGAAAGGCAATAGAAGGAAGCCTGAGAATATCTTCAAAGGGTCAGACTCAATTTACTT*
*TCTAAAGAAGTAGCTAGGAACTAGGGAATAACTTAGAAACAACAAGATTGTATATATGTGCATCCTGGCCC*
*CATTGTTCCTTATCTGTAGGGATAAGCGTGCTTTTTTGTGTGTCTGTATATAACATAACTGTTTACACATAAT*
*ACACTGAAATGGAGCCCTTCCTTGTTACTTCATACCATCCTCTGTGCTTCCTTCCTCAG*GG**GCTGATGCT**
**GCACCAACTGTATCCATCTTCCCACCATCCAGTGAGCAGTTAACATCTGGAGGTGCCTCAGTC**
**GTGTGCTTCTTGAACAACTTCTACCCCAAAGACATCAATGTCAAGTGGAAGATTGATGGCAGT**
**GAACGACAAAATGGCGTCCTGAACAGTTGGACTGATCAGGACAGCAAAGACAGCACCTACAG**
**CATGAGCAGCACCCTCACGTTGACCAAGGACGAGTATGAACGACATAACAGCTATACCTGTG**
**AGGCCACTCACAAGACATCAACTTCACCCATTGTCAAGAGCTTCAACAGGAATGAGTGTTAG**
AGACAAAGGTCCTGAGACGCCACCACCAGCTCCCCAGCTCCATCCTATCTTCCCTTCTAAGGTCTTG
GAGGCTTCCCCACAAGCGACCTACCACTGTTGCGGTGCTCCAAACCTCCTCCCCACCTCCTTCTCCT

CCTCCTCCCTTTCCTTGGCTTTTATCATGCTAATATTTGCAGAAAATATTCAATAAAGTGAGTCTTTG CACTTGAGATCTCTGTCTTTCTTACTAAATGGTAGTAATCAGTTGTTTTTCCAGTTACCTGGGTTTCT CTTCTAAAGAAGTTAAATGTTTAGTTGCCCTGAAATCCACCACACTTAAAGGATAAATAAAACCCT CCACTTGCCCTGGTTGGCTGTCCACTACATGGCAGTCCTTTCTAAGGTTCACGAGTACTATTCATGG CTTATTTCTCTGGGCCATGGTAGGTTTGAGGAGGCATACTTCCTAGTTTTCTTCCCCTAAGTCGTCA AAGTCCTGAAGGGGGACAGTCTTTACAAGCACATGTTCTGTAATCTGATTCAACCTACCCAGTAAA CTTGGCGAAGCAAAGTAGAATCATTATCACAGGAAGCAAAGGCAACCTAAATGTGCAAGCAATAG GAAAATGTGGAAGCCCATCATAGTACTTGGACTTCATCTGCTTTTGTGCCTTCACTAAGTTTTTAAA CATGAGCTGGCTCCTATCTGCCATTGGCAAGGCTGGGCACTACCCACAACCTACTTCAAGGACCTC TATACCGTGAGATTACACACATACATCAAAATTTGGGAAAAGTTCTACCAAGCTGAGAGCTGATCA CCCCACTCTTAGGTGCTTATCTCTGTACACCAGAAACCTTAAGAAGCAACCAGTATTGAGAGACTC ATTTATGAAAGTCTAAAACTGGATACAACCAAAATGTCCACCAACAGTTAAATTATGACATGTTCA CAATTGAGCTATTACTTAATAAGGAGAATTAATAAAATAAAACTTAAGAGCATAGTTTAATCTCAT AAACAAGATAATAAGCAAAACAAAACATTTTTTCATCCATGTAAGTTTAAAAGCAGGTAAAATTTA AAATTAAGAGAGACATAAGTTTTGAGGTAGCAAGATGGAAACTCTGGGGCTTGGGGAATGTTCTGT CTCTCTGTATGGGATGTGAAAGTTACTATTGTGGAATTGGGATCTATGTTCTTCCTGTATATATTGT ATACTTCATAATAACTTCACCTAAAGAAATATCTAATACCCAGTGCATACATAAAAGAGGATACAA GGAATGAATCATACGTCAAGGCCAGAAAGACAATAAAGTAGGGGATCCAGGATCAAATCTCCCAC AACCTTGAGCCTTCTACTATTCTGCCTTCCAGAGCTCAAAGTACAAAACACATAATTCAAACACAT GATCCCTCCTTGGGGTCTCTTCCTTCATGCATCGAATTAGAAATAGCCATGTATAAAATGAGATAG AAGAGACCTTCATCAACAGGTCAAAGAATATAGGTAATTTTGTCTGGGTATGAAGAGCCCACGTAT CAAAGGTTACATTAGGGAAGGAAGAGGACACTAACAGTGACTTTCATTCTCCCCCTCTTCCTGGAG GCCCCTGCATTTAGTCCCTCGTGGGCTCATCCACTCAGCACACATTTACTAAGCATCTTCTCAGCCT ACACTCTGAAGGCAGTGCAGAATAATGTTAGTGTCCCTTCCCCCAGTTAATATGCAGTCCAGTTTCC CTGCTCCTTCCCTTTCTCAGTCCACATAAGGATGATGGGAAAGGACAGTCACCAAATAGGAGAGGG CAACCCTTTGCCTTCCTACCTCTTGAGAATGTACATTATTATCCACTTTTTGAAACTTCTTTTAATTG CTTTTTTTTAATTTGTCTTTTCAAATAGCATAACCTTGTTCATCCATTTCTGGGAACCAAATTTATCA ATCAACAGTGCCTCTAATCTGGCTATTAATACAAAAATGCCTCCTCAAAATATATATGTTCGAGTCT TATCTAAAACAGAACCCACAATAAAAAAGAAGAAAGAATACATATAAGCATTTATATAATTCTGA GCAACCTTGTGCTTTGTGAAAAAAATATAATCTAATGTCACATGCTGTATTCTTTTTATTTAACACT GGTGAAATTATACCATTAGAGAGAAAGAGGACAGATCACTGATCCTAGGATCTAGGGATGTTACA GATAAGAAAACAAATGTGACAAAGAGCTGTCACAAGGAGGATCTTCAAGGTCACAGAATCACTGT CTTGATTTCAGTGGTGGTTACATACATTTAAATATGTGATAAAATGTTGTTGAACTATATTCATATA TTGTACCAATGTCAAATGCTTAATTTTGGCTCTATAGTATAATTATGCACTAAATAACTATTTGGAC AAAGAAAATGATGTTTACATCAAAGGTGAGGCCATATTTGTTAGGAACATAACTTAAAAACCATTT TGGATAACTAATGAAAAGCCATTTTGTGTGCCTTGGCATATCATGCCTAAGCTGTCACCAGATAGA TCTAATAAGACCTAAGCCTCAGAAGCAAGCCCCTGCCCAGCAAGCAGGCAGCACAGATAAGAGCT AAACCCAGGACAGGCCATGATATGCTAATGAACTACCTTCAAGGTGGTGTTGCTGACCTAGTGAAC CAGCCCCAAGCTGTGAGCCCCAATAGCACAAAGCTACTGCCCAAAGAAATTATACAAAAATTGGA ACTTTGGGAATGGTGTGCAGGATCGCTCTGCTGTATGCCTGGAACACAGCTTCTCTATGTTTTGTAT

TGATACCAGTCTAGAAGCTTCCAAAACTTTCTCACTGAAGAAGATTCCCCATGTGGGACCCCTACA
GACTCTTTTGCCCAAACAACTGCTTCCCTCCTGGTGTGATATCTGTTTTGCTTTTATGTTAGCATAAT
ATTATAAGGAATGTTTGTGTGAATAAACCAAACATATTTTAAAAGCAAATATTGTATGCACATCCT
AATTGCTAAAAAGTTTACAGCTAATAGTCCCATGCTCTCCACAATACTGGATCCAAATAAGTCCTA
ATTTCAATGTTGGGCATCTTTACAGAGAGAAAGACATTAAAAATGAAGAGACATGCAGAGAGTGC
ACCATGCCATCGTGGAGACAGACTGAAGTGACACAACTGTTAGTCAAAGAGGATTAAGGACTTCC
AGAAGCCACCAAAGGAAGGAGGTATGAAGTGGTTTCTCCCTCAGAGTATCCAGAGGAGACTAAAC
CAACCAACACCTTTTTGCTTAAGACTTCTTGCCTTCAGGACTGTGAGAAGGTAGCTTCCTATTGTTC
TAAGCCCCAGTATGTGGCATTTTGTTAAGGTAGAGTCAAGAAACCAATAAAATGCAGACAGACAA
AAGGATAGCTGAGTTTTCCAGGCCCTTCCTTCTTATTTTTGGTTTTGTTGGTGGTGGTGGTGGT
GGTGATGGTGGTGGTTTTGTTTATGTTTTGTTTGGGGAGTTTTTTGGGGTTTTTTTGGGTTTTGTTTTT
GTTGTTGTTTTGGGGGTTTTTGTTGTTGTTGTTGTTTGCTTTTTTGTTTTTTGTTTTTTGTTTTTTTGAG
ACAGTGTTTCTCTGTATAGCCCTGGCTGTCCTGGAGTTCCTTCTATCTCTAATGTCTACATCTCAGAG
GGGATCCTCTAATTTCAAATGAGCAGTAGCTCTCCATTTTTAGCTCTTATTTATTCATTTATTTACTT
ACTTACTTATTGTCTGTAGATGAAAGAATTTTGGAGTGGGAAAGGGTTCATGAGCCCCCAGCAACT
AATGAGGAGCTACAGACAATTGATGTTTCTGGGGAAAGGAGACTCAGTTTCTTTGAGAGTATAGCT
TCTGATGGGTCAACCATGTTCCTGTGGCTGATGTCACACCCAGGAGTATGCAGACAACAGAAACTG
GAGTTAATGAGTTGTTTTAAAAATAAAAAAGGGCATGAAGCTTGGGATAGAAATTAAGGATAAAT
ACAATTAAATACAGGAAATTCTGAAAGAATTAATAAAAACATTTCTTTTTTTAAAAAAAAATCCAG
AATTAGCTATGCTTCTTCAAAATTGCTTCTGGAGAACTTTACAAGTTAAATAAGTTATATTGTAGAA
AAGGTAGAGAGGAGAATAGTGGAAGAGAGAGATAAGGAGACTTCAAAAGGAGTGGAGGGAGATA
GAGGAGGAGAAAGCAGAAGCAATGGCTGATAGACACAGGATAAGAGGGAACAGAAAGGAGAAA
GAGGAAGCCAGGATGGGTATTTCTTTGCCTATCTGTGACTTGCACATGGTCTTGGCAATTATTGATG

6. ES cells locus after homologous recombination with the targeting vector:

Sequence of the targeted allele in ES cells genome (figure 3, SEQ ID NO: 130) with:

- *XXXX*: 5' homologous arm
- ***XXXX*:** human cK sequence
- *GGTTCCGGTTCCGGTTCCGGTTCC*: sequence coding for (GS)4
- ***CTGCCCGAGACAGGAGGA:*** sequence coding for LPETGG
- CATCATCATCATCATCAT: sequence coding for (H)6 Tag
- XXXX : sequence of the "Self delete NeoR cassette"
- ***ATAACTTCGTATAGCATACATTATACGAAGTTAT :*** LoxP site
- XXXX: 3' homology arm

*ATCTTTTTTTATGTGTAAGACACAGGTTTTCATGTTAGGAGTTAAAGTCAGTTCAGAAAATCTTGAG*
*AAAATGGAGAGGGCTCATTATCAGTTGACGTGGCATACAGTGTCAGATTTTCTGTTTATCAAGCTAGTGAG*
*ATTAGGGGCAAAAAGAGGCTTTAGTTGAGAGGAAAGTAATTAATACTATGGTCACCATCCAAGAGATTGGA*
*TCGGAGAATAAGCATGAGTAGTTATTGAGATCTGGGTCTGACTGCAGGTAGCGTGGTCTTCTAGACGTTTA*
*AGTGGGAGATTTGGAGGGGATGAGGAATGAAGGAACTTCAGGATAGAAAAGGGCTGAAGTCAAGTTCAG*
*CTCCTAAAATGGATGTGGGAGCAAACTTTGAAGATAAACTGAATGACCCAGAGGATGAAACAGCGCAGAT*
*CAAAGAGGGGCCTGGAGCTCTGAGAAGAGAAGGAGACTCATCCGTGTTGAGTTTCCACAAGTACTGTCTT*
*GAGTTTTGCAATAAAAGTGGGATAGCAGAGTTGAGTGAGCCGTAGGCTGAGTTCTCTCTTTTGTCTCCTAA*
*GTTTTTATGACTACAAAAATCAGTAGTATGTCCTGAAATAATCATTAAGCTGTTTGAAAGTATGACTGCTTGC*
*CATGTAGATACCATGGCTTGCTGAATAATCAGAAGAGGTGTGACTCTTATTCTAAAATTTGTCACAAAATGT*
*CAAAATGAGAGACTCTGTAGGAACGAGTCCTTGACAGACAGCTCAAGGGGTTTTTTCCTTTGTCTCATTTC*
*TACATGAAAGTAAATTTGAAATGATCTTTTTTATTATAAGAGTAGAAATACAGTTGGGTTTGAACTATATGTTT*
*TAATGGCCACGGTTTTGTAAGACATTTGGTCCTTTGTTTTCCCAGTTATTACTCGATTGTAATTTTATATCGC*
*CAGCAATGGACTGAAACGGTCCGCAACCTCTTCTTTACAACTGGGTGACCTCGCGGCTGTGCCAGCCATT*
*TGGCGTTCACCCTGCCGCTAAGGGCCATGTGAACCCCCGCGGTAGCATCCCTTGCTCCGCGTGGACCAC*
*TTTCCTGAGGCACAGTGATAGGAACAGAGCCACTAATCTGAAGAGAACAGAGATGTGACAGACTACACTAA*
*TGTGAGAAAAACAAGGAAAGGGTGACTTATTGGAGATTTCAGAAATAAAATGCATTTATTATTATATTCCCTT*
*ATTTTAATTTTCTATTAGGGAATTAGAAAGGGCATAAACTGCTTTATCCAGTGTTATATTAAAAGCTTAATGTA*
*TATAATCTTTTAGAGGTAAAATCTACAGCCAGCAAAAGTCATGGTAAATATTCTTTGACTGAACTCTCACTAA*
*ACTCCTCTAAATTATATGTCATATTAACTGGTTAAATTAATATAAATTTGTGACATGACCTTAACTGGTTAGGT*
*AGGATATTTTTCTTCATGCAAAAATATGACTAATAATAATTTAGCACAAAAATATTTCCCAATACTTTAATTCT*
*GTGATAGAAAAATGTTTAACTCAGCTACTATAATCCCATAATTTTGAAAACTATTTATTAGCTTTTGTGTTTGA*
*CCCTTCCCTAGCCAAAGGCAACTATTTAAGGACCCTTTAAAACTCTTGAAACTACTTTAGAGTCATTAAGTT*
*ATTTAACCACTTTTAATTACTTTAAAATGATGTCAATTCCCTTTTAACTATTAATTTATTTTAAGGGGGGAAAG*
*GCTGCTCATAATTCTATTGTTTTTCTTGGTAAAGAACTCTCAGTTTTCGTTTTTACTACCTCTGTCACCCAAG*
*AGTTGGCATCTCAACAGAGGGGACTTTCCGAGAGGCCATCTGGCAGTTGCTTAAGATCAGAAGTGAAGTC*
*TGCCAGTTCCTCCCAGGCAGGTGGCCCAGATTACAGTTGACCTGTTCTGGTGTGGCTAAAAATTGTCCCAT*
*GTGGTTACAAACCATTAGACCAGGGTCTGATGAATTGCTCAGAATATTTCTGGACACCCAAATACAGACCC*
*TGGCTTAAGGCCCTGTCCATACAGTAGGTTTAGCTTGGCTACACCAAAGGAAGCCATACAGAGGCTAATAT*
*CAGAGTATTCTTGGAAGAGACAGGAGAAAATGAAAGCCAGTTTCTGCTCTTACCTTATGTGCTTGTGTTCA*
*GACTCCCAAACATCAGGAGTGTCAGATAAACTGGTCTGAATCTCTGTCTGAAGCATGGAACTGAAAAGAAT*
*GTAGTTTCAGGGAAGAAAGGCAATAGAAGGAAGCCTGAGAATATCTTCAAAGGGTCAGACTCAATTTACTT*
*TCTAAAGAAGTAGCTAGGAACTAGGGAATAACTTAGAAACAACAAGATTGTATATATGTGCATCCTGGCCC*
*CATTGTTCCTTATCTGTAGGGATAAGCGTGCTTTTTTGTGTGTCTGTATATAACATAACTGTTTACACATAAT*
*ACACTGAAATGGAGCCCTTCCTTGTTACTTCATACCATCCTCTGTGCTTCCTTCCTCAG*GA**ACTGTGGCT**
**GCACCATCTGTCTTCATCTTCCCGCCATCTGATGAGCAGTTGAAATCTGGAACTGCCTCTGTT**
**GTGTGCCTGCTGAATAACTTCTATCCCAGAGAGGCCAAAGTACAGTGGAAGGTGGATAACGC**
**CCTCCAATCGGGTAACTCCCAGGAGAGTGTCACAGAGCAGGACAGCAAGGACAGCACCTACA**
**GCCTCAGCAGCACCCTGACGCTGAGCAAAGCAGACTACGAGAAACACAAAGTCTACGCCTGC**
**GAAGTCACCCATCAGGGCCTGAGCTCGCCCGTCACAAAGAGCTTCAACAGGGGAGAGTGT***GG*

*TTCCGGTTCCGGTTCCGGTTCC*___CTGCCCGAGACAGGAGGA___CATCATCATCATCATCATTGAAGACAA
ACCCGATCTCGAGGTCGACTCGCGAAAGCTAGCTT___ATAACTTCGTATAGCATACATTATACGAAGTT___
___AT___GGATCTCCATGGCCAGGCAAATATCCCTTACCAGCCTCACAGAGACCTCCCCCACCCCCCGCA
ACCCTAGAGTTCTTTTACTAGTGAGGGACAAGTGGACAATGGTGCTGTTGTGGGCCCCACCCTGTG
TCCCCTGTGCCCACAGTGGTCACTCTGCTTGGCAGGCAGGTGTTGCAGGCTGGCTGCTCCAGGCCCT
GGCAGGAGGTACTGAAGGACCTGGTAGGCTCAGATGCCCTGGATGCCAAGGCACTGCTGGAGTAC
TTCCAACCGGTCAGCCAGTGGCTGGAAGAGCAGAATCAGCGGAATGGCGAAGTCCTAGGCTGGCC
AGAGAATCAGTGGCGTCCACCGTTACCCGACAACTATCCAGAGGGCATTGGTAAAGCTCTGAGTGA
GGGTGGACTGGGACCAAGAGAAGTCCTGGCCTCTGGCCTCTGGCTTCTGGGTCAAAGCCTCAGCAT
CCTGGTCACTTTGCTGCCAGCTGAGCCCCAGTGTCCTTTGCTTCAGTGCCAAGCCACCCCTGGGCTC
ATCCTCAGGGCCCTAAGCAGAAATGGGTATGTCTTTCTCTCAGGGTCCTAGAGACAGTGTGCCCAA
GCCTGAGGGCCCTTGGGGTCAGGCTGGCTGGCACATTGCTCTATGAGGTCACACTGCAGGCTTGGC
TCTTATTGGCCGGTGATGGGAGCTTCAGGGCTCTGCTTTCCTGCGGCCATGCCCAAGAAGAAGAGG
AAGGTGTCCAATTTACTGACCGTACACCAAAATTTGCCTGCATTACCGGTCGATGCAACGAGTGAT
GAGGTTCGCAAGAACCTGATGGACATGTTCAGGGATCGCCAGGCGTTTTCTGAGCATACCTGGAAA
ATGCTTCTGTCCGTTTGCCGGTCGTGGGCGGCATGGTGCAAGTTGAATAACCGGAAATGGTTTCCC
GCAGAACCTGAAGATGTTCGCGATTATCTTCTATATCTTCAGGCGCGCGGTCTGGCAGTAAAAACT
ATCCAGCAACATTTGGGCCAGCTAAACATGCTTCATCGTCGGTCCGGGCTGCCACGACCAAGTGAC
AGCAATGCTGTTTCACTGGTTATGCGGCGGATCCGAAAAGAAAACGTTGATGCCGGTGAACGTGCA
AAACAGGCTCTAGCGTTCGAACGCACTGATTTCGACCAGGTTCGTTCACTCATGGAAAATAGCGAT
CGCTGCCAGGATATACGTAATCTGGCATTTCTGGGGATTGCTTATAACACCCTGTTACGTATAGCCG
AAATTGCCAGGATCAGGGTTAAAGATATCTCACGTACTGACGGTGGGAGAATGTTAATCCATATTG
GCAGAACGAAAACGCTGGTTAGCACCGCAGGTGTAGAGAAGGCACTTAGCCTGGGGGTAACTAAA
CTGGTCGAGCGATGGATTTCCGTCTCTGGTGTAGCTGATGATCCGAATAACTACCTGTTTTGCCGGG
TCAGAAAAAATGGTGTTGCCGCGCCATCTGCCACCAGCCAGCTATCAACTCGCGCCCTGGAAGGGA
TTTTTGAAGCAACTCATCGATTGATTTACGGCGCTAAGGTAAATATAAAATTTTTAAGTGTATAATG
TGTTAAACTACTGATTCTAATTGTTTGTGTATTTTAGGATGACTCTGGTCAGAGATACCTGGCCTGG
TCTGGACACAGTGCCCGTGTCGGAGCCGCGCGAGATATGGCCCGCGCTGGAGTTTCAATACCGGAG
ATCATGCAAGCTGGTGGCTGGACCAATGTAAATATTGTCATGAACTATATCCGTAACCTGGATAGT
GAAACAGGGGCAATGGTGCGCCTGCTGGAAGATGGCGATTAGCCATTAACGCGTAAATGATTGCT
ATAATTATTTGATATTTATGGTGACATATGAGAAAGGATTTCAACATCGACGGAAAATATGTAGTG
CTGTCTGTAAGCACTAATATTCAGTCGCCAGCCGTCATTGTCACTGTAAAGCTGAGCGGCAATAAA
AAGACAGAATAAAACGCACGGGTGTTGGGTCGTTTGTTCGGTCGAGCTCGCGAAAGCTTCTACCGG
GTAGGGGAGGCGCTTTTCCCAAGGCAGTCTGGAGCATGCGCTTTAGCAGCCCCGCTGGGCACTTGG
CGCTACACAAGTGGCCTCTGGCCTCGCACACATTCCACATCCACCGGTAGGCGCCAACCGGCTCCG
TTCTTTGGTGGCCCCGTCGCGCCACCTTCTACTCCTCCCCTAGTCAGGAAGTTCCCCCCCGCCCCGC
AGCTCGCGTCGTGCAGGACGTGACAAATGGAAGTAGCACGTCTCACTAGTCTCGTGCAGATGGACA
GCACCGCTGAGCAATGGAAGCGGGTAGGCCTTTGGGGCAGCGGCCAATAGCAGCTTTGCTCCTTCG
CTTTCTGGGCTCAGAGGCTGGGAAGGGGTGGGTCCGGGGGCGGGCTCAGGGGCGGGCTCAGGGGC
GGGGCGGGCGCCCGAAGGTCCTCCGGAGGCCCGGCATTCTGCACGCTTCAAAAGCGCACGTCTGCC

GCGCTGTTCTCCTCTTCCTCATCTCCGGGCCTTTCGACCTGCAGCAGCACGTGTTGACAATTAATCA
TCGGCATAGTATATCGGCATAGTATAATACGACAAGGTGAGGAACTAAACCATGGGATCGGCCATT
GAACAAGATGGATTGCACGCAGGTTCTCCGGCCGCTTGGGTGGAGAGGCTATTCGGCTATGACTGG
GCACAACAGACGATCGGCTGCTCTGATGCCGCCGTGTTCCGGCTGTCAGCGCAGGGGCGCCCGGTT
CTTTTTGTCAAGACCGACCTGTCCGGTGCCCTGAATGAACTGCAGGACGAGGCAGCGCGGCTATCG
TGGCTGGCCACGACGGGCGTTCCTTGCGCAGCTGTGCTCGACGTTGTCACTGAAGCGGGAAGGGAC
TGGCTGCTATTGGGCGAAGTGCCGGGGCAGGATCTCCTGTCATCTCACCTTGCTCCTGCCGAGAAA
GTATCCATCATGGCTGATGCAATGCGGCGGCTGCATACGCTTGATCCGGCTACCTGCCCATTCGAC
CACCAAGCGAAACATCGCATCGAGCGAGCACGTACTCGGATGGAAGCCGGTCTTGTCGATCAGGA
TGATCTGGACGAAGAGCATCAGGGGCTCGCGCCAGCCGAACTGTTCGCCAGGCTCAAGGCGCGCA
TGCCCGACGGCGAGGATCTCGTCGTGACCCATGGCGATGCCTGCTTGCCGAATATCATGGTGGAAA
ATGGCCGCTTTTCTGGATTCATCGACTGTGGCCGGCTGGGTGTGGCGGACCGCTATCAGGACATAG
CGTTGGCTACCCGTGATATTGCTGAAGAGCTTGGCGGCGAATGGGCTGACCGCTTCCTCGTGCTTTA
CGGTATCGCCGCCCCCGATTCGCAGCGCATCGCCTTCTATCGCCTTCTTGACGAGTTCTTCTGAGCG
GGACTCTGGGGTTCGAATAAAGACCGACCAAGCGACGTCTGAGAGCTCCCTGGCGAATTCGGTACC
AATAAAAGAGCTTTATTTTCATGATCTGTGTGTTGGTTTTTGTGTGCGGCGCG***ATAACTTCGTATAGC***
***ATACATTATACGAAGTTAT***CCCAATTCATCGATATCTAGATCGGGGTCCTGAGACGCCACCACCAGC
TCCCCAGCTCCATCCTATCTTCCCTTCTAAGGTCTTGGAGGCTTCCCCACAAGCGACCTACCACTGT
TGCGGTGCTCCAAACCTCCTCCCCACCTCCTTCTCCTCCTCCTCCCTTTCCTTGGCTTTTATCATGCT
AATATTTGCAGAAAATATTCAATAAAGTGAGTCTTTGCACTTGAGATCTCTGTCTTTCTTACTAAAT
GGTAGTAATCAGTTGTTTTTCCAGTTACCTGGGTTTCTCTTCTAAAGAAGTTAAATGTTTAGTTGCC
CTGAAATCCACCACACTTAAAGGATAAATAAAACCCTCCACTTGCCCTGGTTGGCTGTCCACTACA
TGGCAGTCCTTTCTAAGGTTCACGAGTACTATTCATGGCTTATTTCTCTGGGCCATGGTAGGTTTGA
GGAGGCATACTTCCTAGTTTTCTTCCCCTAAGTCGTCAAAGTCCTGAAGGGGGACAGTCTTTACAA
GCACATGTTCTGTAATCTGATTCAACCTACCCAGTAAACTTGGCGAAGCAAAGTAGAATCATTATC
ACAGGAAGCAAAGGCAACCTAAATGTGCAAGCAATAGGAAAATGTGGAAGCCCATCATAGTACTT
GGACTTCATCTGCTTTTGTGCCTTCACTAAGTTTTTAAACATGAGCTGGCTCCTATCTGCCATTGGC
AAGGCTGGGCACTACCCACAACCTACTTCAAGGACCTCTATACCGTGAGATTACACACATACATCA
AAATTTGGGAAAAGTTCTACCAAGCTGAGAGCTGATCACCCCACTCTTAGGTGCTTATCTCTGTAC
ACCAGAAACCTTAAGAAGCAACCAGTATTGAGAGACTCATTTATGAAAGTCTAAAACTGGATACA
ACCAAAATGTCCACCAACAGTTAAATTATGACATGTTCACAATTGAGCTATTACTTAATAAGGAGA
ATTAATAAAATAAAACTTAAGAGCATAGTTTAATCTCATAAACAAGATAATAAGCAAAACAAAAC
ATTTTTTCATCCATGTAAGTTTAAAAGCAGGTAAAATTTAAAATTAAGAGAGACATAAGTTTTGAG
GTAGCAAGATGGAAACTCTGGGGCTTGGGGAATGTTCTGTCTCTCTGTATGGGATGTGAAAGTTAC
TATTGTGGAATTGGGATCTATGTTCTTCCTGTATATATTGTATACTTCATAATAACTTCACCTAAAG
AAATATCTAATACCCAGTGCATACATAAAAGAGGATACAAGGAATGAATCATACGTCAAGGCCAG
AAAGACAATAAAGTAGGGGATCCAGGATCAAATCTCCCACAACCTTGAGCCTTCTACTATTCTGCC
TTCCAGAGCTCAAAGTACAAAACACATAATTCAAACACATGATCCCTCCTTGGGGTCTCTTCCTTCA
TGCATCGAATTAGAAATAGCCATGTATAAAATGAGATAGAAGAGACCTTCATCAACAGGTCAAAG
AATATAGGTAATTTTGTCTGGGTATGAAGAGCCCACGTATCAAAGGTTACATTAGGGAAGGAAGA

GGACACTAACAGTGACTTTCATTCTCCCCCTCTTCCTGGAGGCCCCTGCATTTAGTCCCTCGTGGGC
TCATCCACTCAGCACACATTTACTAAGCATCTTCTCAGCCTACACTCTGAAGGCAGTGCAGAATAA
TGTTAGTGTCCCTTCCCCCAGTTAATATGCAGTCCAGTTTCCCTGCTCCTTCCCTTTCTCAGTCCACA
TAAGGATGATGGGAAAGGACAGTCACCAAATAGGAGAGGGCAACCCTTTGCCTTCCTACCTCTTGA
GAATGTACATTATTATCCACTTTTTGAAACTTCTTTTAATTGCTTTTTTTTAATTTGTCTTTTCAAATA
GCATAACCTTGTTCATCCATTTCTGGGAACCAAATTTATCAATCAACAGTGCCTCTAATCTGGCTAT
TAATACAAAAATGCCTCCTCAAAATATATATGTTCGAGTCTTATCTAAAACAGAACCCACAATAAA
AAAGAAGAAAGAATACATATAAGCATTTATATAATTCTGAGCAACCTTGTGCTTTGTGAAAAAAAT
ATAATCTAATGTCACATGCTGTATTCTTTTTATTTAACACTGGTGAAATTATACCATTAGAGAGAAA
GAGGACAGATCACTGATCCTAGGATCTAGGGATGTTACAGATAAGAAAACAAATGTGACAAAGAG
CTGTCACAAGGAGGATCTTCAAGGTCACAGAATCACTGTCTTGATTTCAGTGGTGGTTACATACATT
TAAATATGTGATAAAATGTTGTTGAACTATATTCATATATTGTACCAATGTCAAATGCTTAATTTTG
GCTCTATAGTATAATTATGCACTAAATAACTATTTGGACAAAGAAAATGATGTTTACATCAAAGGT
GAGGCCATATTTGTTAGGAACATAACTTAAAAACCATTTTGGATAACTAATGAAAAGCCATTTTGT
GTGCCTTGGCATATCATGCCTAAGCTGTCACCAGATAGATCTAATAAGACCTAAGCCTCAGAAGCA
AGCCCCTGCCCAGCAAGCAGGCAGCACAGATAAGAGCTAAACCCAGGACAGGCCATGATATGCTA
ATGAACTACCTTCAAGGTGGTGTTGCTGACCTAGTGAACCAGCCCCAAGCTGTGAGCCCCAATAGC
ACAAAGCTACTGCCCAAAGAAATTATACAAAAATTGGAACTTTGGGAATGGTGTGCAGGATCGCTC
TGCTGTATGCCTGGAACACAGCTTCTCTATGTTTTGTATTGATACCAGTCTAGAAGCTTCCAAAACT
TTCTCACTGAAGAAGATTCCCCATGTGGGACCCCTACAGACTCTTTTGCCCAAACAACTGCTTCCCT
CCTGGTGTGATATCTGTTTTGCTTTTATGTTAGCATAATATTATAAGGAATGTTTGTGTGAATAAAC
CAAACATATTTTAAAAGCAAATATTGTATGCACATCCTAATTGCTAAAAAGTTTACAGCTAATAGT
CCCATGCTCTCCACAATACTGGATCCAAATAAGTCCTAATTTCAATGTTGGGCATCTTTACAGAGAG
AAAGACATTAAAAATGAAGAGACATGCAGAGAGTGCACCATGCCATCGTGGAGACAGACTGAAGT
GACACAACTGTTAGTCAAAGAGGATTAAGGACTTCCAGAAGCCACCAAAGGAAGGAGGTATGAAG
TGGTTTCTCCCTCAGAGTATCCAGAGGAGACTAAACCAACCAACACCTTTTTGCTTAAGACTTCTTG
CCTTCAGGACTGTGAGAAGGTAGCTTCCTATTGTTCTAAGCCCCAGTATGTGGCATTTTGTTAAGGT
AGAGTCAAGAAACCAATAAAATGCAGACAGACAAAAGGATAGCTGAGTTTTCCAGGCCCTTCCTT
CTTATTTTTGGTTTTGTTGGTGGTGGTGGTGGTGGTGGTGATGGTGGTGGTTTTGTTTATGTTTTGTT
TGGGGAGTTTTTTGGGGTTTTTTTGGGTTTTGTTTTTGTTGTTGTTTTGGGGGTTTTTGTTGTTGTTGT
TGTTTGCTTTTTTGTTTTTTGTTTTTTGTTTTTTTGAGACAGTGTTTCTCTGTATAGCCCTGGCTGTCC
TGGAGTTCCTTCTATCTCTAATGTCTACATCTCAGAGGGGATCCTCTAATTTCAAATGAGCAGTAGC
TCTCCATTTTTAGCTCTTATTTATTCATTTATTTACTTACTTACTTATTGTCTGTAGATGAAAGAATTT
TGGAGTGGGAAAGGGTTCATGAGCCCCCAGCAACTAATGAGGAGCTACAGACAATTGATGTTTCTG
GGGAAAGGAGACTCAGTTTCTTTGAGAGTATAGCTTCTGATGGGTCAACCATGTTCCTGTGGCTGA
TGTCACACCCAGGAGTATGCAGACAACAGAAACTGGAGTTAATGAGTTGTTTTAAAAATAAAAAA
GGGCATGAAGCTTGGGATAGAAATTAAGGATAAATACAATTAAATACAGGAAATTCTGAAAGAAT
TAATAAAAACATTTCTTTTTTTAAAAAAAAATCCAGAATTAGCTATGCTTCTTCAAAATTGCTTCTG
GAGAACTTTACAAGTTAAATAAGTTATATTGTAGAAAAGGTAGAGAGGAGAATAGTGGAAGAGAG
AGATAAGGAGACTTCAAAAGGAGTGGAGGGAGATAGAGGAGGAGAAAGCAGAAGCAATGGCTGA

TAGACACAGGATAAGAGGGAACAGAAAGGAGAAAGAGGAAGCCAGGATGGGTATTTCTTTGCCTATCTGTGACTTGCACATGGTCTTGGCAATTATTGATG

7. Targeted locus after deletion of neomycin selection cassette:

Locus after deletion of neomycin selection cassette (figure 4) with a focus on the modified gene region (figure 5).

Final sequence (SEQ ID NO: 131) of the targeted allele in a genome of mutant mice with:

- *XXXX* : 5' homologous arm
- **XXXX:** human cK sequence
- *GGTTCCGGTTCCGGTTCCGGTTCC*: sequence coding for (GS)4
- ***CTGCCCGAGACAGGAGGA:*** sequence coding for LPETGG
- CATCATCATCATCATCAT: sequence coding for (H)6 Tag
- ***ATAACTTCGTATAGCATACATTATACGAAGTTAT*** **:** LoxP site
- XXXX : residual cloning sequences of the "Self delete NeoR cassette"
- XXXX: 3' homologous arm

*ATCTTTTTTTATGTGTAAGACACAGGTTTTCATGTTAGGAGTTAAAGTCAGTTCAGAAAATCTTGAGAAAATGGAGAGGGCTCATTATCAGTTGACGTGGCATACAGTGTCAGATTTTCTGTTTATCAAGCTAGTGAGATTAGGGGCAAAAAGAGGCTTTAGTTGAGAGGAAAGTAATTAATACTATGGTCACCATCCAAGAGATTGGATCGGAGAATAAGCATGAGTAGTTATTGAGATCTGGGTCTGACTGCAGGTAGCGTGGTCTTCTAGACGTTTAAGTGGGAGATTTGGAGGGGATGAGGAATGAAGGAACTTCAGGATAGAAAAGGGCTGAAGTCAAGTTCAGCTCCTAAAATGGATGTGGGAGCAAACTTTGAAGATAAACTGAATGACCCAGAGGATGAAACAGCGCAGATCAAAGAGGGGCCTGGAGCTCTGAGAAGAGAAGGAGACTCATCCGTGTTGAGTTTCCACAAGTACTGTCTTGAGTTTTGCAATAAAAGTGGGATAGCAGAGTTGAGTGAGCCGTAGGCTGAGTTCTCTCTTTTGTCTCCTAAGTTTTTATGACTACAAAAATCAGTAGTATGTCCTGAAATAATCATTAAGCTGTTTGAAAGTATGACTGCTTGCCATGTAGATACCATGGCTTGCTGAATAATCAGAAGAGGTGTGACTCTTATTCTAAAATTTGTCACAAAATGTCAAAATGAGAGACTCTGTAGGAACGAGTCCTTGACAGACAGCTCAAGGGGTTTTTTTCCTTTGTCTCATTTCTACATGAAAGTAAATTTGAAATGATCTTTTTTATTATAAGAGTAGAAATACAGTTGGGTTTGAACTATATGTTTTAATGGCCACGGTTTTGTAAGACATTTGGTCCTTTGTTTTCCCAGTTATTACTCGATTGTAATTTTATATCGCCAGCAATGGACTGAAACGGTCCGCAACCTCTTCTTTACAACTGGGTGACCTCGCGGCTGTGCCAGCCATTTGGCGTTCACCCTGCCGCTAAGGGCCATGTGAACCCCCGCGGTAGCATCCCTTGCTCCGCGTGGACCACTTTCCTGAGGCACAGTGATAGGAACAGAGCCACTAATCTGAAGAGAACAGAGATGTGACAGACTACACTAATGTGAGAAAAACAAGGAAAGGGTGACTTATTGGAGATTTCAGAAATAAAATGCATTTATTATTATATTCCCTTATTTTAATTTTCTATTAGGGAATTAGAAAGGGCATAAACTGCTTTATCCAGTGTTATATTAAAAGCTTAATGTATATAATCTTTTAGAGGTAAAATCTACAGCCAGCAAAAGTCATGGTAAATATTCTTTGACTGAACTCTCACTAA*

*ACTCCTCTAAATTATATGTCATATTAACTGGTTAAATTAATATAAATTTGTGACATGACCTTAACTGGTTAGGT*
*AGGATATTTTTCTTCATGCAAAAATATGACTAATAATAATTTAGCACAAAAATATTTCCCAATACTTTAATTCT*
*GTGATAGAAAAATGTTTAACTCAGCTACTATAATCCCATAATTTTGAAAACTATTTATTAGCTTTTGTGTTTGA*
*CCCTTCCCTAGCCAAAGGCAACTATTTAAGGACCCTTTAAAACTCTTGAAACTACTTTAGAGTCATTAAGTT*
*ATTTAACCACTTTTAATTACTTTAAAATGATGTCAATTCCCTTTTAACTATTAATTTATTTTAAGGGGGGAAAG*
*GCTGCTCATAATTCTATTGTTTTTCTTGGTAAAGAACTCTCAGTTTTCGTTTTTACTACCTCTGTCACCCAAG*
*AGTTGGCATCTCAACAGAGGGGACTTTCCGAGAGGCCATCTGGCAGTTGCTTAAGATCAGAAGTGAAGTC*
*TGCCAGTTCCTCCCAGGCAGGTGGCCCAGATTACAGTTGACCTGTTCTGGTGTGGCTAAAAATTGTCCCAT*
*GTGGTTACAAACCATTAGACCAGGGTCTGATGAATTGCTCAGAATATTTCTGGACACCCAAATACAGACCC*
*TGGCTTAAGGCCCTGTCCATACAGTAGGTTTAGCTTGGCTACACCAAAGGAAGCCATACAGAGGCTAATAT*
*CAGAGTATTCTTGGAAGAGACAGGAGAAAATGAAAGCCAGTTTCTGCTCTTACCTTATGTGCTTGTGTTCA*
*GACTCCCAAACATCAGGAGTGTCAGATAAACTGGTCTGAATCTCTGTCTGAAGCATGGAACTGAAAAGAAT*
*GTAGTTTCAGGGAAGAAAGGCAATAGAAGGAAGCCTGAGAATATCTTCAAAGGGTCAGACTCAATTTACTT*
*TCTAAAGAAGTAGCTAGGAACTAGGGAATAACTTAGAAACAACAAGATTGTATATATGTGCATCCTGGCCC*
*CATTGTTCCTTATCTGTAGGGATAAGCGTGCTTTTTTGTGTGTCTGTATATAACATAACTGTTTACACATAAT*
*ACACTGAAATGGAGCCCTTCCTTGTTACTTCATACCATCCTCTGTGCTTCCTTCCTCAG*GA**ACTGTGGCT**
**GCACCATCTGTCTTCATCTTCCCGCCATCTGATGAGCAGTTGAAATCTGGAACTGCCTCTGTT**
**GTGTGCCTGCTGAATAACTTCTATCCCAGAGAGGCCAAAGTACAGTGGAAGGTGGATAACGC**
**CCTCCAATCGGGTAACTCCCAGGAGAGTGTCACAGAGCAGGACAGCAAGGACAGCACCTACA**
**GCCTCAGCAGCACCCTGACGCTGAGCAAAGCAGACTACGAGAAACACAAAGTCTACGCCTGC**
**GAAGTCACCCATCAGGGCCTGAGCTCGCCCGTCACAAAGAGCTTCAACAGGGGAGAGTGT***GG*
*TTCCGGTTCCGGTTCCGGTTCC****CTGCCCACAGAGGGA***CATCATCATCATCATCATTGAAGACAAACC
CGATCTCGAGGTCGACTCGCGAAAGCTAGCTT***ATAACTTCGTATAGCATACATTATACGAAGTTAT***C
CCAATTCATCGATATCTAGATCGGGGTCCTGAGACGCCACCACCAGCTCCCCAGCTCCATCCTATCT
TCCCTTCTAAGGTCTTGGAGGCTTCCCCACAAGCGACCTACCACTGTTGCGGTGCTCCAAACCTCCT
CCCCACCTCCTTCTCCTCCTCCTCCCTTTCCTTGGCTTTTATCATGCTAATATTTGCAGAAAATATTC
AATAAAGTGAGTCTTTGCACTTGAGATCTCTGTCTTTCTTACTAAATGGTAGTAATCAGTTGTTTTTC
CAGTTACCTGGGTTTCTCTTCTAAAGAAGTTAAATGTTTAGTTGCCCTGAAATCCACCACACTTAAA
GGATAAATAAAACCCTCCACTTGCCCTGGTTGGCTGTCCACTACATGGCAGTCCTTTCTAAGGTTCA
CGAGTACTATTCATGGCTTATTTCTCTGGGCCATGGTAGGTTTGAGGAGGCATACTTCCTAGTTTTC
TTCCCCTAAGTCGTCAAAGTCCTGAAGGGGGACAGTCTTTACAAGCACATGTTCTGTAATCTGATTC
AACCTACCCAGTAAACTTGGCGAAGCAAAGTAGAATCATTATCACAGGAAGCAAAGGCAACCTAA
ATGTGCAAGCAATAGGAAAATGTGGAAGCCCATCATAGTACTTGGACTTCATCTGCTTTTGTGCCTT
CACTAAGTTTTTAAACATGAGCTGGCTCCTATCTGCCATTGGCAAGGCTGGGCACTACCCACAACC
TACTTCAAGGACCTCTATACCGTGAGATTACACACATACATCAAAATTTGGGAAAAGTTCTACCAA
GCTGAGAGCTGATCACCCCACTCTTAGGTGCTTATCTCTGTACACCAGAAACCTTAAGAAGCAACC
AGTATTGAGAGACTCATTTATGAAAGTCTAAAACTGGATACAACCAAAATGTCCACCAACAGTTAA
ATTATGACATGTTCACAATTGAGCTATTACTTAATAAGGAGAATTAATAAAATAAAACTTAAGAGC
ATAGTTTAATCTCATAAACAAGATAATAAGCAAAACAAAACATTTTTTCATCCATGTAAGTTTAAA
AGCAGGTAAAATTTAAAATTAAGAGAGACATAAGTTTTGAGGTAGCAAGATGGAAACTCTGGGGC

TTGGGGAATGTTCTGTCTCTCTGTATGGGATGTGAAAGTTACTATTGTGGAATTGGGATCTATGTTC
TTCCTGTATATATTGTATACTTCATAATAACTTCACCTAAAGAAATATCTAATACCCAGTGCATACA
TAAAAGAGGATACAAGGAATGAATCATACGTCAAGGCCAGAAAGACAATAAAGTAGGGGATCCA
GGATCAAATCTCCCACAACCTTGAGCCTTCTACTATTCTGCCTTCCAGAGCTCAAAGTACAAAACA
CATAATTCAAACACATGATCCCTCCTTGGGGTCTCTTCCTTCATGCATCGAATTAGAAATAGCCATG
TATAAAATGAGATAGAAGAGACCTTCATCAACAGGTCAAAGAATATAGGTAATTTTGTCTGGGTAT
GAAGAGCCCACGTATCAAAGGTTACATTAGGGAAGGAAGAGGACACTAACAGTGACTTTCATTCT
CCCCCTCTTCCTGGAGGCCCCTGCATTTAGTCCCTCGTGGGCTCATCCACTCAGCACACATTTACTA
AGCATCTTCTCAGCCTACACTCTGAAGGCAGTGCAGAATAATGTTAGTGTCCCTTCCCCCAGTTAAT
ATGCAGTCCAGTTTCCCTGCTCCTTCCCTTTCTCAGTCCACATAAGGATGATGGGAAAGGACAGTCA
CCAAATAGGAGAGGGCAACCCTTTGCCTTCCTACCTCTTGAGAATGTACATTATTATCCACTTTTTG
AAACTTCTTTTAATTGCTTTTTTTTAATTTGTCTTTTCAAATAGCATAACCTTGTTCATCCATTTCTGG
GAACCAAATTTATCAATCAACAGTGCCTCTAATCTGGCTATTAATACAAAAATGCCTCCTCAAAAT
ATATATGTTCGAGTCTTATCTAAAACAGAACCCACAATAAAAAAGAAGAAAGAATACATATAAGC
ATTTATATAATTCTGAGCAACCTTGTGCTTTGTGAAAAAAATATAATCTAATGTCACATGCTGTATT
CTTTTTATTTAACACTGGTGAAATTATACCATTAGAGAGAAAGAGGACAGATCACTGATCCTAGGA
TCTAGGGATGTTACAGATAAGAAAACAAATGTGACAAAGAGCTGTCACAAGGAGGATCTTCAAGG
TCACAGAATCACTGTCTTGATTTCAGTGGTGGTTACATACATTTAAATATGTGATAAAATGTTGTTG
AACTATATTCATATATTGTACCAATGTCAAATGCTTAATTTTGGCTCTATAGTATAATTATGCACTA
AATAACTATTTGGACAAAGAAAATGATGTTTACATCAAAGGTGAGGCCATATTTGTTAGGAACATA
ACTTAAAAACCATTTTGGATAACTAATGAAAAGCCATTTTGTGTGCCTTGGCATATCATGCCTAAGC
TGTCACCAGATAGATCTAATAAGACCTAAGCCTCAGAAGCAAGCCCCTGCCCAGCAAGCAGGCAG
CACAGATAAGAGCTAAACCCAGGACAGGCCATGATATGCTAATGAACTACCTTCAAGGTGGTGTTG
CTGACCTAGTGAACCAGCCCCAAGCTGTGAGCCCCAATAGCACAAAGCTACTGCCCAAAGAAATT
ATACAAAAATTGGAACTTTGGGAATGGTGTGCAGGATCGCTCTGCTGTATGCCTGGAACACAGCTT
CTCTATGTTTTGTATTGATACCAGTCTAGAAGCTTCCAAAACTTTCTCACTGAAGAAGATTCCCCAT
GTGGGACCCCTACAGACTCTTTTGCCCAAACAACTGCTTCCCTCCTGGTGTGATATCTGTTTTGCTTT
TATGTTAGCATAATATTATAAGGAATGTTTGTGTGAATAAACCAAACATATTTTAAAAGCAAATAT
TGTATGCACATCCTAATTGCTAAAAAGTTTACAGCTAATAGTCCCATGCTCTCCACAATACTGGATC
CAAATAAGTCCTAATTTCAATGTTGGGCATCTTTACAGAGAGAAAGACATTAAAAATGAAGAGAC
ATGCAGAGAGTGCACCATGCCATCGTGGAGACAGACTGAAGTGACACAACTGTTAGTCAAAGAGG
ATTAAGGACTTCCAGAAGCCACCAAAGGAAGGAGGTATGAAGTGGTTTCTCCCTCAGAGTATCCAG
AGGAGACTAAACCAACCAACACCTTTTTGCTTAAGACTTCTTGCCTTCAGGACTGTGAGAAGGTAG
CTTCCTATTGTTCTAAGCCCCAGTATGTGGCATTTTGTTAAGGTAGAGTCAAGAAACCAATAAAAT
GCAGACAGACAAAAGGATAGCTGAGTTTTCCAGGCCCTTCCTTCTTATTTTTGGTTTTGTTGGTGGT
GGTGGTGGTGGTGGTGATGGTGGTGGTTTTGTTTATGTTTTGTTTGGGGAGTTTTTTGGGGTTTTTTT
GGGTTTTGTTTTTGTTGTTGTTTTGGGGGTTTTTGTTGTTGTTGTTGTTTGCTTTTTTGTTTTTTGTTTT
TTGTTTTTTTGAGACAGTGTTTCTCTGTATAGCCCTGGCTGTCCTGGAGTTCCTTCTATCTCTAATGT
CTACATCTCAGAGGGGATCCTCTAATTTCAAATGAGCAGTAGCTCTCCATTTTTAGCTCTTATTTAT
TCATTTATTTACTTACTTACTTATTGTCTGTAGATGAAAGAATTTTGGAGTGGGAAAGGGTTCATGA

GCCCCCAGCAACTAATGAGGAGCTACAGACAATTGATGTTTCTGGGGAAAGGAGACTCAGTTTCTTTGAGAGTATAGCTTCTGATGGGTCAACCATGTTCCTGTGGCTGATGTCACACCCAGGAGTATGCAGACAACAGAAACTGGAGTTAATGAGTTGTTTTAAAAATAAAAAAGGGCATGAAGCTTGGGATAGAAATTAAGGATAAATACAATTAAATACAGGAAATTCTGAAAGAATTAATAAAAACATTTCTTTTTTTAAAAAAAAATCCAGAATTAGCTATGCTTCTTCAAAATTGCTTCTGGAGAACTTTACAAGTTAAATAAGTTATATTGTAGAAAAGGTAGAGAGGAGAATAGTGGAAGAGAGAGATAAGGAGACTTCAAAAGGAGTGGAGGGAGATAGAGGAGGAGAAAGCAGAAGCAATGGCTGATAGACACAGGATAAGAGGGAACAGAAAGGAGAAAGAGGAAGCCAGGATGGGTATTTCTTTGCCTATCTGTGACTTGCACATGGTCTTGGCAATTATTGATG

**EXAMPLE 2:** Development of a transgenic non-human mammal genetically modified to express an antibody according to the invention.

The B cells found in the blood of a wild-type mouse and of a mouse homozygous for the allele-hCk LPETG were identified on the basis of their CD19+ CD45R+ phenotype and analyzed for the presence of BCR-expressing chain hCk-LPETG at their cell surface. In contrast to wild-type mice, all B cells of the mouse hCk-LPETG express human CK light chains as expected (figure 6).

**REFERENCES:**

Throughout this application, various references describe the state of the art to which this invention pertains. The disclosures of these references are hereby incorporated by reference into the present disclosure.

Tsukiji S. and Nagamune T. Sortase-Mediated Ligation: A Gift from Gram-Positive Bacteria to Protein Engineering. ChemBioChem, Volume 10, Issue 5, pages 787-798, March 23, 2009.

Popp MW, Antos JM, Grotenbreg GM, Spooner E, Ploegh HL. Sortagging: a versatile method for protein labeling. Nat Chem Biol. 2007 Nov;3(11):707-8. Epub 2007 Sep 23.

## Claims

1. A fusion protein consisting of a constant domain (CL) of a light chain of an immunoglobulin fused at its C-terminal end to a polypeptide (P) **characterized by** the sequence:

LPXT(G)n (P)

wherein n is an integer number and X represents an amino acid other than tryptophan and cysteine.

2. A fusion protein according to the claim 1 wherein n is 1, 2, 3, 4, 5 or 6.

3. A fusion protein according to any claims 1 to 2 which comprises at its C-terminal end a tag.

4. A fusion protein according to the claim 3 wherein the peptidic sequence of the tag is (H)n with n which can be 1 to 10.

5. A nucleic acid molecule encoding for a fusion protein according to claims 1 to 4.

6. An expression vector comprising a nucleic acid molecule according to claim 5.

7. A prokaryotic or eukaryotic host cell genetically transformed with at least one nucleic acid molecule according to claim 5 or an expression vector according to claim 6.

8. An antibody wherein the constant domain of the light chain is replaced by a fusion protein according to any claims 1 to 4.

9. A transgenic non-human mammal genetically modified to express an antibody according to claim 8.

10. An antibody according to the claim 8 conjugated to a polypeptide of interest (Pi).

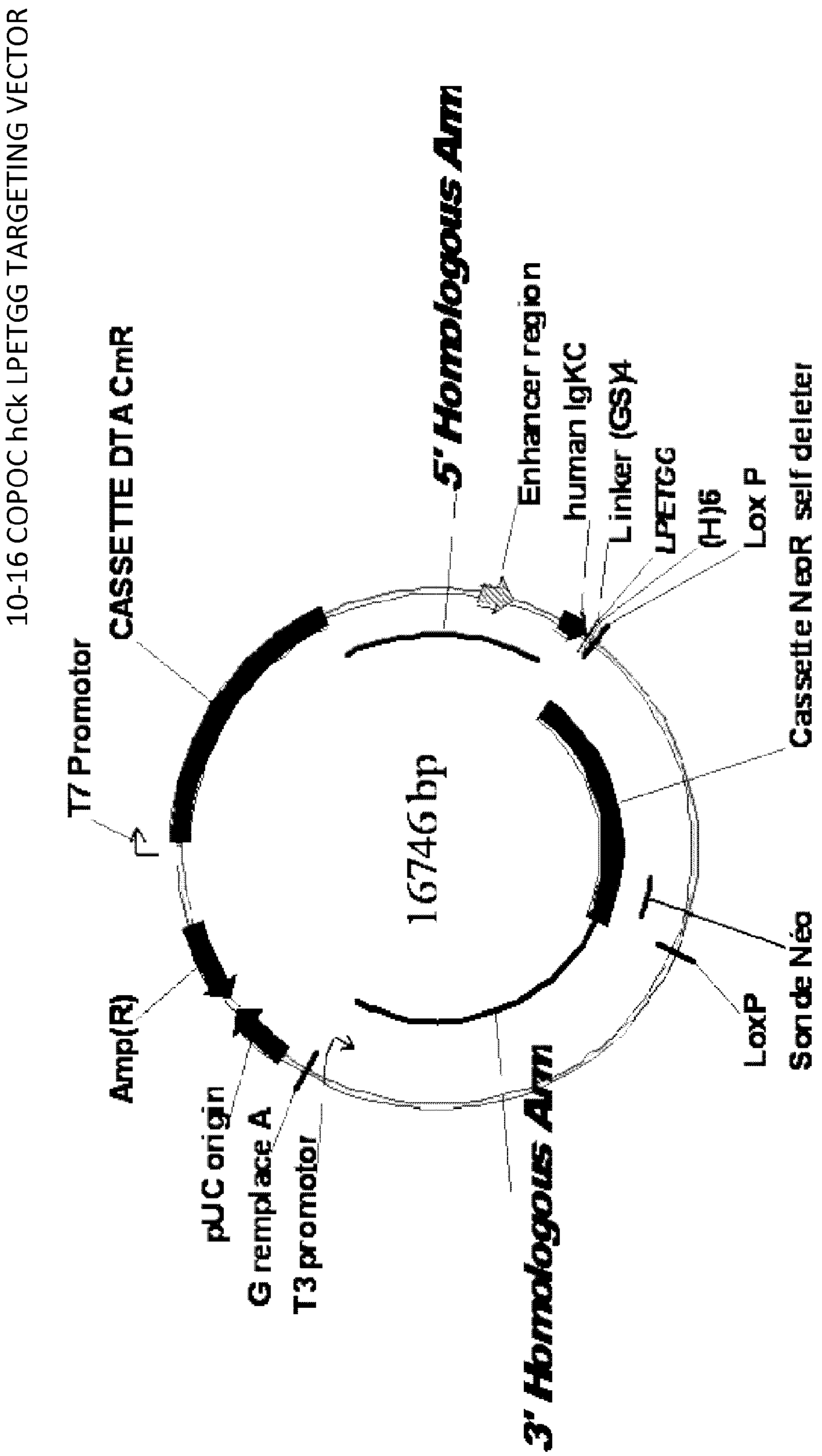
10-16 COPOC hCk LPETGG TARGETING VECTOR
CASSETTE DTA CmR
T7 Promotor
5' Homologous Arm
Enhancer region
human IgKC
Linker (GS)4
LPETGG
(H)6
Lox P
Cassette NeoR self deleter
16746 bp
Amp(R)
pUC origin
G remplace A
T3 promotor
3' Homologous Arm
LoxP
Sonde Néo

Figure 1

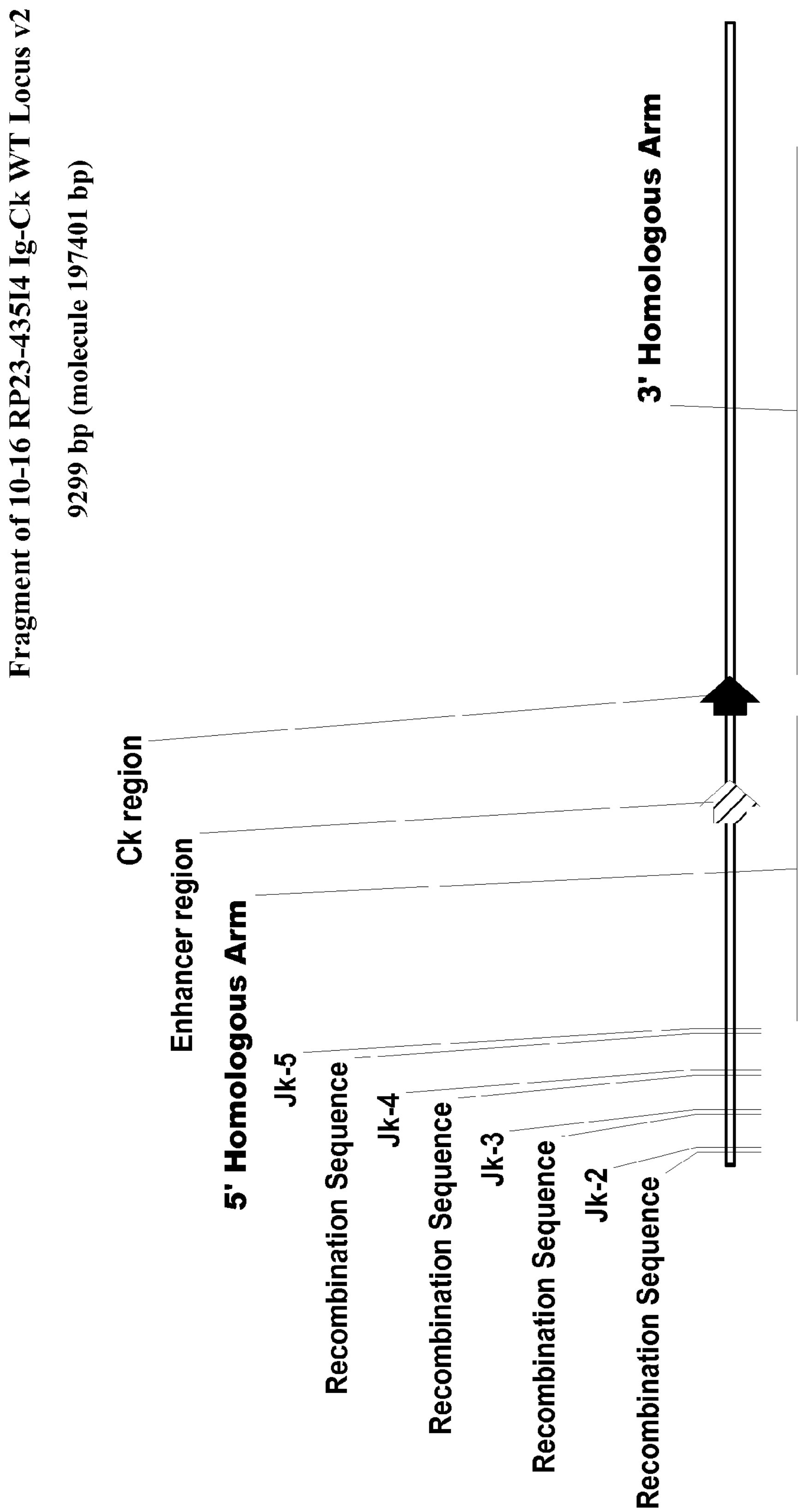
Fragment of 10-16 RP23-435I4 Ig-Ck WT Locus v2
9299 bp (molecule 197401 bp)
3' Homologous Arm
Ck region
Enhancer region
5' Homologous Arm
Jk-5
Recombination Sequence
Jk-4
Recombination Sequence
Jk-3
Recombination Sequence
Jk-2
Recombination Sequence

Figure 2

**Fragment of 10-16 RP23-435I4 Ig-Ck Mutant ES Cells Locus v2**
**12562 bp (molecule 201140 bp)**

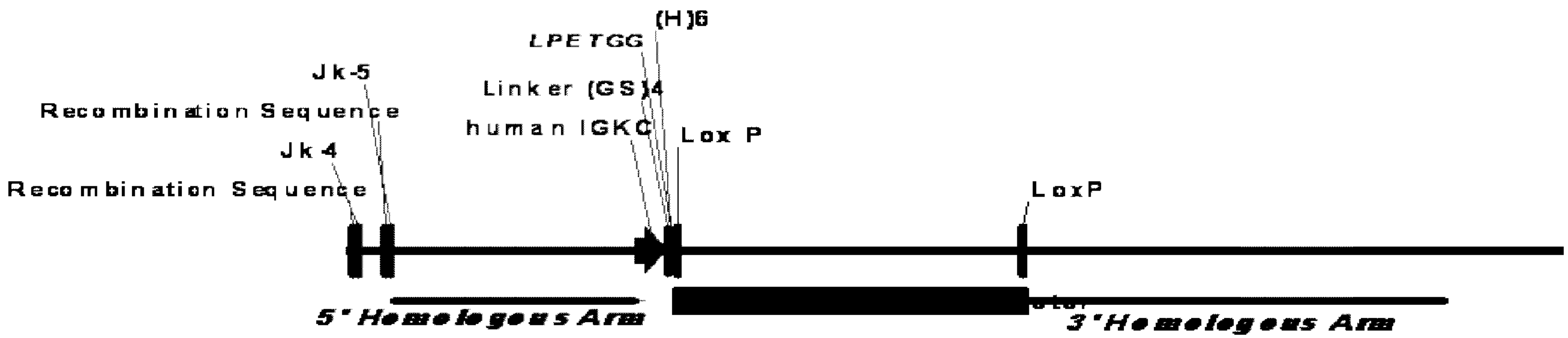


Figure 3

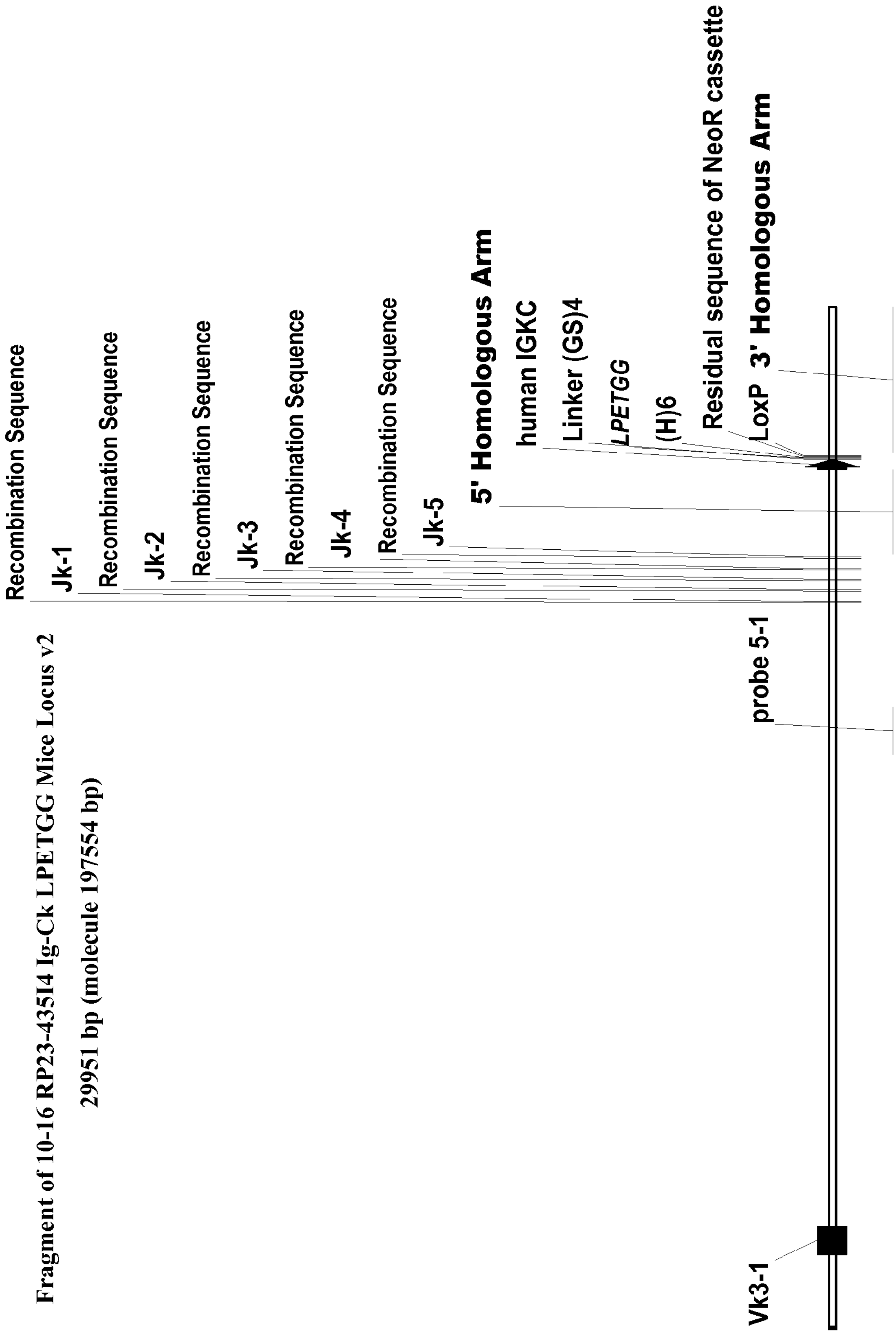
Fragment of 10-16 RP23-435I4 Ig-Ck LPETGG Mice Locus v2
29951 bp (molecule 197554 bp)
Recombination Sequence
Jk-1
Recombination Sequence
Jk-2
Recombination Sequence
Jk-3
Recombination Sequence
Jk-4
Recombination Sequence
Jk-5
5' Homologous Arm
human IGKC
Linker (GS)4
LPETGG
(H)6
Residual sequence of NeoR cassette
LoxP
3' Homologous Arm
probe 5-1
Vk3-1

Figure 4

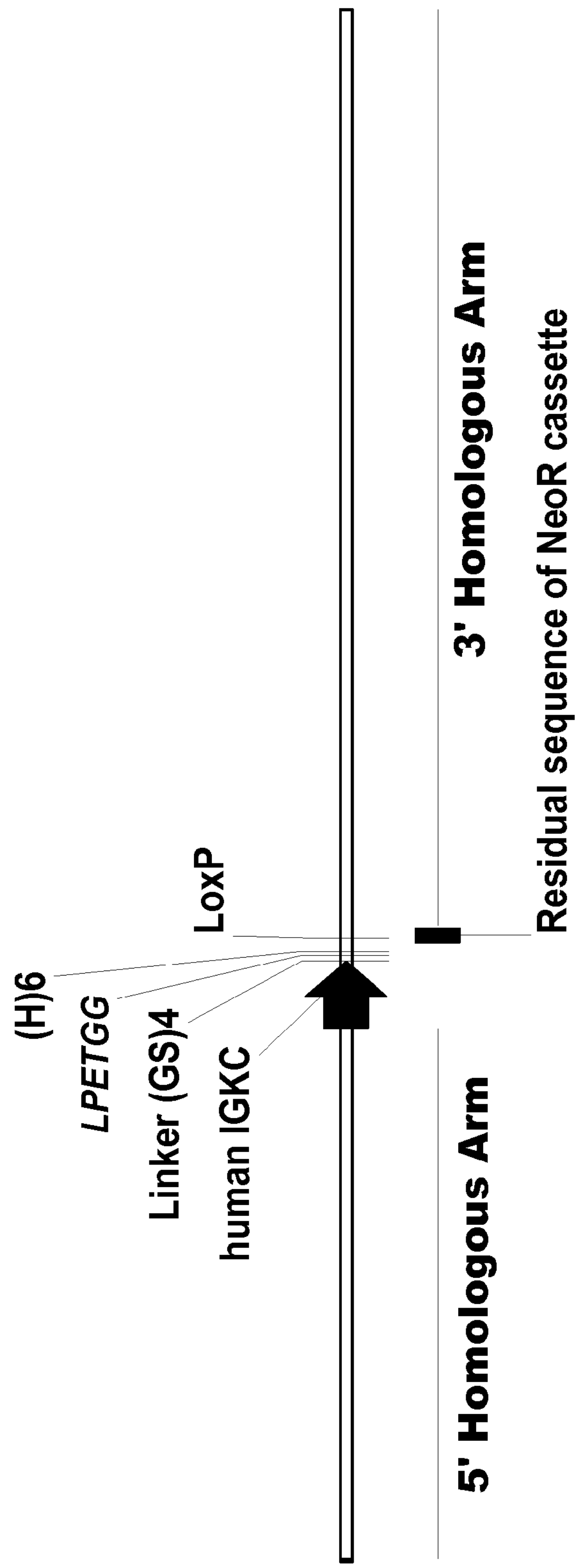
3' Homologous Arm
Residual sequence of NeoR cassette
LoxP
(H)6
LPETGG
Linker (GS)4
human IGKC
5' Homologous Arm

Figure 5

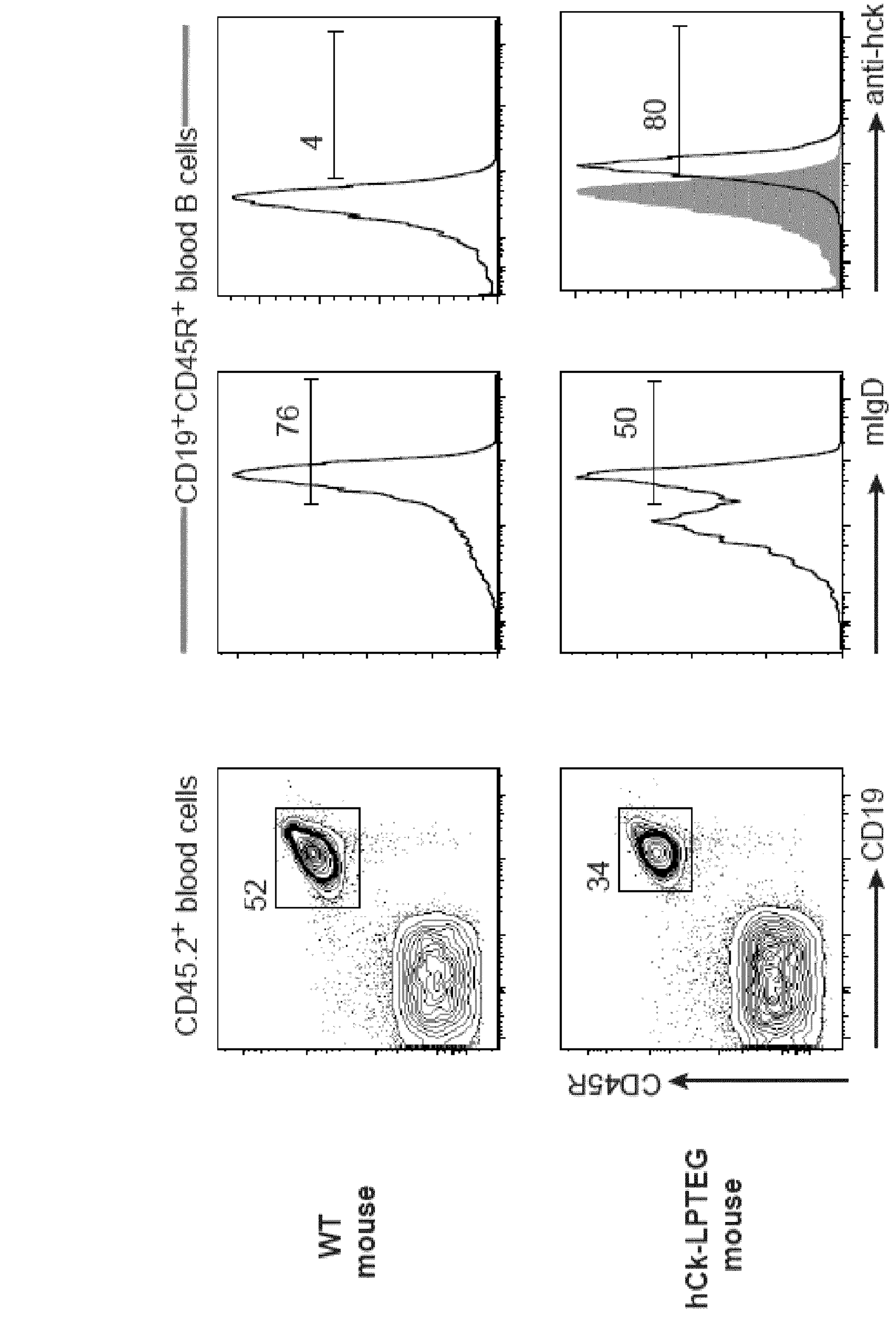
CD45.2+ blood cells
CD19+CD45R+ blood B cells
52
76
4
34
50
80
CD45R
CD19
mIgD
anti-hck
WT mouse
hCk-LPTEG mouse

Figure 6

Europäisches Patentamt
European Patent Office
Office européen des brevets

# EUROPEAN SEARCH REPORT

Application Number
EP 12 30 6424

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 391 714 A2 (WHITEHEAD BIOMEDICAL INST [US]) 7 December 2011 (2011-12-07)<br>* claims 107-135 *<br>----- | 1-3,5-10 | INV.<br>C07K16/00<br>C12N9/48 |
| X | DAVID A LEVARY ET AL: "Protein-Protein Fusion Catalyzed by Sortase A",<br>PLOS ONE, PUBLIC LIBRARY OF SCIENCE, US, [Online]<br>vol. 6, no. 4, 1 April 2011 (2011-04-01), pages e18342.1-e18342.6, XP002686608,<br>ISSN: 1932-6203, DOI: 10.1371/JOURNAL.PONE.0018342<br>Retrieved from the Internet:<br>URL:http://www.plosone.org/article/info%3Adoi%2F10.1371%2Fjournal.pone.0018342><br>[retrieved on 2011-04-06]<br>* the whole document *<br>----- | 1-10 | |
| A | MADEJ M P ET AL: "Engineering of an anti-epidermal growth factor receptor antibody to single chain format and labeling by sortase A-mediated protein ligation",<br>BIOTECHNOLOGY AND BIOENGINEERING, WILEY & SONS, HOBOKEN, NJ, US,<br>vol. 109, no. 6, Sp. Iss. SI, 1 June 2012 (2012-06-01), pages 1461-1470, XP002686223,<br>ISSN: 0006-3592, DOI: 10.1002/BIT.24407<br>[retrieved on 2011-12-26]<br>* the whole document *<br>-----<br>-/-- | 1-10 | TECHNICAL FIELDS SEARCHED (IPC)<br>C07K<br>C12N |

1

The present search report has been drawn up for all claims

| Place of search | Date of completion of the search | Examiner |
|---|---|---|
| Munich | 8 April 2013 | Marinoni J-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 30 6424

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | SWEE LEE KIM ET AL: "Sortase-mediated modification of alpha DEC205 affords optimization of antigen presentation and immunization against a set of viral epitopes", PROCEEDINGS OF THE NATIONAL ACADEMY OF SCIENCES OF THE UNITED STATES OF AMERICA, vol. 110, no. 4, January 2013 (2013-01), pages 1428-1433, XP002694943, ISSN: 0027-8424<br>----- | | |

TECHNICAL FIELDS SEARCHED (IPC)

The present search report has been drawn up for all claims

1

| Place of search | Date of completion of the search | Examiner |
|---|---|---|
| Munich | 8 April 2013 | Marinoni J-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 12 30 6424

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2013

| Patent document cited in search report | Publication date | Patent family member(s) | Publication date |
|---|---|---|---|
| EP 2391714 A2 | 07-12-2011 | EP 2391714 A2 | 07-12-2011 |
| | | US 2011321183 A1 | 29-12-2011 |
| | | WO 2010087994 A2 | 05-08-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5202238 A
- US 5204244 A
- WO 9710354 A
- EP 239400 A
- WO 9109967 A
- US 5225539 A
- US 5530101 A
- US 5585089 A
- EP 592106 A
- EP 519596 A
- US 5565332 A
- EP 125023 A
- WO 9602576 A
- WO 9514785 A
- WO 9622378 A
- US 5882877 A
- US 6013516 A
- US 4861719 A
- US 5278056 A
- WO 9419478 A
- US 5324822 A
- US 5306811 A
- US 4960716 A
- US 4885363 A
- US 5087440 A
- US 5155215 A
- US 5188816 A
- US 5219553 A
- US 5262532 A
- US 5358704 A
- US 20030153043 A, Carr
- US 5677425 A, Bodmer
- US 6165745 A, Ward
- US 6277375 B, Ward.
- US 5869046 A
- US 6121022 A, Presta
- US 5624821 A
- US 5648260 A, Winter
- US 6194551 B, Idusogie
- WO 9429351 A, Bodmer
- WO 0042072 A, Presta
- WO 2010106180 A
- US 5714350 A
- US 6350861 A, Co
- EP 1176195 A, Hang
- WO 03035835 A, Presta
- WO 9954342 A, Umana
- EP 1297172 B1
- EP O154316 A, Nishimura
- EP 0401384 A, Ishikawa
- US 5283433 A
- US 5296703 A, Tsien
- US 6090919 A, Cormack
- US 6461813 B, Lorens
- US 6455263 A, Payan

### Non-patent literature cited in the description


- **MAZMANIAN,S.K. ; LIU,G. ; TON-THAT,H. ; SCHNEEWIND,O.** Staphylococcus aureus sortase, an enzyme that anchors surface proteins to the cell wall. *Science,* 1999, vol. 285 (5428), 760-763
- **ACRES et al.** *Curr Opin Mol Ther,* February 2004, vol. 6, 40-7
- **TAYLOR-PAPADIMITRIOU et al.** *Biochim Biophys Acta,* 08 October 1999, vol. 1455 (2-3), 301-13
- **EMENS et al.** *Cancer Biol Ther.,* July 2003, vol. 2 (4), 161-8
- **OHSHIMA et al.** *Int J Cancer.,* 01 July 2001, vol. 93 (1), 91-6
- **D. MEYER et al.** *Invest. Radiol.,* 1990, vol. 25, 53-55
- **SHIELDS, R. L. et al.** *J. Biol. Chen.,* 2001, vol. 276, 6591-6604
- **SHIELDS, R.L. et al.** *J. Biol. Chem.,* 2002, vol. 277, 26733-26740
- **UMANA et al.** *Nat. Biotech.,* 1999, vol. 17, 176-180
- **E. J. ROBERTSON.** Teratocarcinomas and Embryonic Stem Cells: A Practical Approach. IRL Press, 1987
- **KOHLER ; MILSTEIN.** *Nature,* 1975, vol. 256, 495
- Coding, Monoclonal Antibodies: Principles and Practice: Production and Application of Monoclonal Antibodies in Cell Biology, Biochemistry and Immunology. Academic Press, 1996
- **BRYANT ; PLOEGH.** *Curr Opin Immunol,* 2004, vol. 16, 96-102
- **SHASTRI et al.** *Annu Rev Immunol,* 2002, vol. 20, 463-93

- **BANCHEREAU ; PALUCKA.** *Nat Rev Immunol,* 2005, vol. 5, 296-306
- **GUERMONPREZ et al.** *Annu Rev Immunol,* 2002, vol. 20, 621-67
- **ADEMA et al.** *Curr Opin Immunol,* 2005, vol. 17, 170-4
- **BERZOFSKY et al.** *J Clin Invest,* 2004, vol. 114, 450-62
- **NESTLE et al.** *Curr Opin Immunol,* 2005, vol. 17, 163-9
- Remington's Pharmaceutical Sciences. 1035-10381570-1580
- **TSUKIJI S. ; NAGAMUNE T.** Sortase-Mediated Ligation: A Gift from Gram-Positive Bacteria to Protein Engineering. *ChemBioChem,* 23 March 2009, vol. 10 (5), 787-798
- **POPP MW ; ANTOS JM ; GROTENBREG GM ; SPOONER E ; PLOEGH HL.** Sortagging: a versatile method for protein labeling. *Nat Chem Biol.,* 23 September 2007, vol. 3 (11), 707-8